# EUROPEAN PATENT APPLICATION

(11) **EP 4 108 683 A1**
(43) Date of publication of application: **28.12.2022**
(21) Application number: 21756707.2
(22) Date of filing: 19.02.2021
(51) Int. Cl.: C07K 16/24, A61K 39/395, A61K 39/00

(54) **ANTI-IL-2 ANTIBODY, AND ANTIGEN-BINDING FRAGMENT THEREOF AND MEDICAL USE THEREOF**

(30) Priority: 21.02.2020 CN 202010107662
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: LIN, Yuan, Lianyungang, Jiangsu 222047 (CN); ZHU, Fuxiang, Lianyungang, Jiangsu 222047 (CN); LIAO, Cheng, Lianyungang, Jiangsu 222047 (CN)
(74) Representative: Potter Clarkson
(86) International application number: PCT/CN2021/076806
(87) International publication number: WO 2021/164722

(57) **Abstract**

Provided are an anti-IL-2 antibody, and an antigen-binding fragment thereof and the medical use thereof. Further provided are a complex (including a fusion protein) of the anti-IL-2 antibody, the antigen-binding fragment thereof and IL-2, and the use of the complex as a drug for treating autoimmune diseases and inflammatory diseases.

## Description

The present application claims priority to Chinese Patent Application (Application No. 202010107662.4) filed on Feb. 21, 2020.

### TECHNICAL FIELD

The present disclosure relates to an antibody, e.g., an antibody and an antigen-binding fragment thereof that specifically bind to interleukin-2 (IL-2). The present disclosure relates to a complex (including a fusion protein) of the anti-IL-2 antibody or the antigen-binding fragment thereof and IL-2, and use of the complex as a medicament for treating an immune-related disease (e.g., an autoimmune disease or an inflammatory disease).

### BACKGROUND

Human interleukin-2 (IL-2), also known as T cell growth factor (TCGF), has a gene located on chromosome 4 (4q27) and comprising a sequence of 7 kb in total, and consists of 133 amino acids with a molecular weight of about 15 kD. In 1976 and 1977, Doris Morgan, Francis Ruscetti, Robert Gallo and Steven Gillis, Kendal Smith, et al., separately found that activated T cell culture medium could promote T cell proliferation. Thereafter, the stimulating factor in the culture medium was purified and identified as a single protein, i.e., IL-2.

Early *in vitro* cell experiments showed that T cells could secrete IL-2 and express the IL-2 receptor (IL-2R) on the cell surface after being activated by TCR and CD28. The binding of IL-2 to its receptor can trigger T cell proliferation and enable T cell response. This model makes IL-2 a molecule that plays a central role in T cell immune responses. However, it was found in subsequent *in vivo* experiments that animals developed autoimmunity after IL-2 or its receptor was knocked out. Subsequent experiments showed that IL-2 could activate not only effector cells such as T cells and NK cells, but also regulatory T cells (Tregs), thereby suppressing excess self-directed autoimmunity. IL-2 acts through IL-2R on the cell surface. IL-2R includes three subunits: IL-2Rα (i.e., CD25), IL-2Rβ (i.e., CD122), and IL-2Ry (i.e., CD132). Those three subunits can form three forms of receptors: the high-binding avidity receptor comprising all of the three subunits IL-2Rα/β/γ, the medium-binding avidity receptor comprising IL-2R β/γ, and the low-binding avidity receptor being IL-2Rα. Among them, IL-2Rβ and IL-2Rγ are necessary for IL-2 to activate downstream signaling pathways, and when IL-2 binds to both IL-2Rβ and IL-2Rγ, those two receptor subunits form a heterodimer that phosphorylates STAT5 in cells, and enters cell nucleus to cause corresponding gene transcription and expression; and IL-2Rα is not necessary for signaling, but can enhance the binding of IL-2 to IL-2Rβ and IL-2Rγ. Tregs continuously express the high-binding avidity receptor IL-2Rα/β/γ, while unactivated CD8⁺ T cells, NK cells and the like only express the medium-binding avidity receptor IL-2Rβ/γ. Therefore, low-dose IL-2 preferentially binds to and activates Tregs, while high-dose IL-2 can activate immune effector cells such as CD8⁺ T cells and NK cells.

Since Tregs have immunosuppressive effects and low-dose IL-2 has the property of preferentially activating Tregs, low-dose IL-2 has been reported to be useful for the treatment of a series of immune-related diseases such as autoimmune diseases and inflammatory diseases, for example, systemic lupus erythematosus (SLE), type I diabetes (T1D), chronic graft versus host disease (cGvHD), hematopoietic stem cell transplantation (HSCT), alopecia areata, hepatitis C virus-induced vasculitis, chronic GvHD, asthma, dermatitis, alopecia areata, acute lung injury, chronic beryllium disease, and sepsis. In many patients receiving the low-dose IL-2 treatment, there is an increase in the ratio of Tregs to effector T cells and alleviation in symptoms. However, in one aspect, IL-2 has a short half-life and requires frequent administration; in another aspect, IL-2 has a narrow administration window, and may activate immune effector cells and exacerbate autoimmune diseases if at a slightly higher dose. Those intrinsic properties or shortcomings of IL-2 make it difficult to maintain a sustained low dose of IL-2 in patients, thereby limiting the further use of IL-2.

Anti-IL-2 antibodies can alter the binding of IL-2 to IL-2R. Anti-IL-2 antibodies are provided in WO2017/070561 and WO2015/109212. However, there remain long-standing unmet needs in the art for new therapies for the treatment of IL-2-mediated diseases, disorders and conditions. The present disclosure satisfies those needs.

### SUMMARY

The present disclosure provides an anti-IL-2 antibody and an antigen-binding fragment thereof, a complex comprising the antibody and the antigen-binding fragment thereof, a coding nucleic acid, a vector, a host cell and a pharmaceutical composition, a method thereof for treating or delaying immune-related diseases, and pharmaceutical use thereof.

### Anti-IL-2 Antibody or Antigen-Binding Fragment Thereof

In some embodiments, provided is an IL-2 antibody or an antigen-binding fragment thereof, which has an avidity for IL-2 with a K_{D} value of ≤ 5 nM.

In other embodiments, provided is an IL-2 antibody or an antigen-binding fragment thereof, which has an avidity for IL-2 with a K_{D} value of > 5 nM or ≥ 10 nM, and ≤ 200 nM or ≤ 150 nM or ≤ 100 nM or ≤ 80 nM or ≤ 60 nM. In some specific embodiments, the IL-2 antibody or the antigen-binding fragment thereof has an avidity for IL-2 with a K_{D} value of > 5 nM and ≤ 60 nM, > 5 nM and ≤ 50 nM, ≥ 10 nM and ≤ 50 nM, ≥ 10 nM and ≤ 60 nM, > 5 nM and ≤ 80 nM, ≥ 10 nM and ≤ 80 nM, > 5 nM and ≤ 100 nM, ≥ 10 nM and ≤ 100 nM, > 5 nM and ≤ 150 nM, or ≥ 10 nM and ≤ 150 nM.

In some embodiments, the IL-2 antibody or the antigen-binding fragment thereof of the present disclosure comprises a heavy chain variable region (VH) and a light chain variable region (VL), wherein the VH has an HCDR1, an HCDR2 and an HCDR3, and the VL has an LCDR1, an LCDR2 and an LCDR3.

In some embodiments, the IL-2 antibody or the antigen-binding fragment thereof comprises a VH and a VL, wherein:
the VH has an HCDR1, an HCDR2 and an HCDR3 corresponding to an HCDR1, an HCDR2 and an HCDR3 in SEQ ID NO: 1, respectively, and the VL has an LCDR1, an LCDR2 and an LCDR3 corresponding to an LCDR1, an LCDR2 and an LCDR3 in SEQ ID NO: 2, respectively;
the VH has an HCDR1, an HCDR2 and an HCDR3 corresponding to an HCDR1, an HCDR2 and an HCDR3 in SEQ ID NO: 3, respectively, and the VL has an LCDR1, an LCDR2 and an LCDR3 corresponding to an LCDR1, an LCDR2 and an LCDR3 in SEQ ID NO: 4, respectively;
the VH has an HCDR1, an HCDR2 and an HCDR3 corresponding to an HCDR1, an HCDR2 and an HCDR3 in SEQ ID NO: 5, respectively, and the VL has an LCDR1, an LCDR2 and an LCDR3 corresponding to an LCDR1, an LCDR2 and an LCDR3 in SEQ ID NO: 6, respectively;
the VH has an HCDR1, an HCDR2 and an HCDR3 corresponding to an HCDR1, an HCDR2 and an HCDR3 in SEQ ID NO: 7, respectively, and the VL has an LCDR1, an LCDR2 and an LCDR3 corresponding to an LCDR1, an LCDR2 and an LCDR3 in SEQ ID NO: 8, respectively;
the VH has an HCDR1, an HCDR2 and an HCDR3 corresponding to an HCDR1, an HCDR2 and an HCDR3 in SEQ ID NO: 9, respectively, and the VL has an LCDR1, an LCDR2 and an LCDR3 corresponding to an LCDR1, an LCDR2 and an LCDR3 in SEQ ID NO: 10, respectively; or
the VH has an HCDR1, an HCDR2 and an HCDR3 corresponding to an HCDR1, an HCDR2 and an HCDR3 in SEQ ID NO: 11, respectively, and the VL has an LCDR1, an LCDR2 and an LCDR3 corresponding to an LCDR1, an LCDR2 and an LCDR3 in SEQ ID NO: 12, respectively. The above HCDR1, HCDR2 HCDR3, LCDR1, LCDR2 and LCDR3 are defined according to the Kabat, IMGT, Chothia, AbM or Contact numbering system.

In some embodiments, the anti-IL-2 antibody or the antigen-binding fragment thereof comprises:
an HCDR1 set forth in SEQ ID NO: 13, 19, 25, 31, 37 or 43, and/or
an HCDR2 set forth in SEQ ID NO: 14, 20, 26, 32, 38 or 44, and/or
an HCDR3 set forth in SEQ ID NO: 15, 21, 27, 33, 39 or 45, and/or
an LCDR1 set forth in SEQ ID NO: 16, 22, 28, 34, 40 or 46, and/or
an LCDR2 set forth in SEQ ID NO: 17, 23, 29, 35, 41 or 47, and/or
an LCDR3 set forth in SEQ ID NO: 18, 24, 30, 36, 42 or 48. The Kabat numbering system is used in the embodiments.

In other embodiments, the anti-IL-2 antibody or the antigen-binding fragment thereof comprises:
an HCDR1 set forth in SEQ ID NO: 49, 55, 61, 67, 73 or 79, and/or
an HCDR2 set forth in SEQ ID NO: 50, 56, 62, 68, 74 or 80, and/or
an HCDR3 set forth in SEQ ID NO: 51, 57, 63, 69, 75 or 81, and/or
an LCDR1 set forth in SEQ ID NO: 52, 58, 64, 70, 76 or 82, and/or
an LCDR2 set forth in SEQ ID NO: 53, 59, 65, 71, 77 or 83, and/or
an LCDR3 set forth in SEQ ID NO: 54, 60, 66, 72, 78 or 84. The Chothia numbering system is used in the embodiments.

In other embodiments, the anti-IL-2 antibody or the antigen-binding fragment thereof comprises:
an HCDR1 set forth in SEQ ID NO: 85, 91, 97, 103, 109 or 115, and/or
an HCDR2 set forth in SEQ ID NO: 86, 92, 98, 104, 110 or 116, and/or
an HCDR3 set forth in SEQ ID NO: 87, 93, 99, 105, 111 or 117, and/or
an LCDR1 set forth in SEQ ID NO: 88, 94, 100, 106, 112 or 118, and/or
an LCDR2 set forth in SEQ ID NO: 89, 95, 101, 107, 113 or 119, and/or
an LCDR3 set forth in SEQ ID NO: 90, 96, 102, 108, 114 or 120. The IMGT numbering system is used in the embodiments.

In other embodiments, the anti-IL-2 antibody or the antigen-binding fragment thereof comprises:
an HCDR1 set forth in SEQ ID NO: 121, 127, 133, 139, 145 or 151, and/or
an HCDR2 set forth in SEQ ID NO: 122, 128, 134, 140, 146 or 152, and/or
an HCDR3 set forth in SEQ ID NO: 123, 129, 135, 141, 147 or 153, and/or
an LCDR1 set forth in SEQ ID NO: 124, 130, 136, 142, 148 or 154, and/or
an LCDR2 set forth in SEQ ID NO: 125, 131, 137, 143, 149 or 155, and/or
an LCDR3 set forth in SEQ ID NO: 126, 132, 138, 144, 150 or 156. The AbM numbering system is used in the embodiments.

The above anti-IL-2 antibodies or the antigen-binding fragments thereof are divided into two groups, wherein the first group comprises:
an HCDR1 set forth in SEQ ID NO: 13, 25 or 37, and/or
an HCDR2 set forth in SEQ ID NO: 14, 26 or 38, and/or
an HCDR3 set forth in SEQ ID NO: 15, 27 or 39, and/or
an LCDR1 set forth in SEQ ID NO: 16, 28 or 40, and/or
an LCDR2 set forth in SEQ ID NO: 17, 29 or 41, and/or
an LCDR3 set forth in SEQ ID NO: 18, 30 or 42;
an HCDR1 set forth in SEQ ID NO: 49, 61 or 73, and/or
an HCDR2 set forth in SEQ ID NO: 50, 62 or 74, and/or
an HCDR3 set forth in SEQ ID NO: 51, 63 or 75, and/or
an LCDR1 set forth in SEQ ID NO: 52, 64 or 76, and/or
an LCDR2 set forth in SEQ ID NO: 53, 65 or 77, and/or
an LCDR3 set forth in SEQ ID NO: 54, 66 or 78;
an HCDR1 set forth in SEQ ID NO: 85, 97 or 109, and/or
an HCDR2 set forth in SEQ ID NO: 86, 98 or 110, and/or
an HCDR3 set forth in SEQ ID NO: 87, 99 or 111, and/or
an LCDR1 set forth in SEQ ID NO: 88, 100 or 112, and/or
an LCDR2 set forth in SEQ ID NO: 89, 101 or 113, and/or
an LCDR3 set forth in SEQ ID NO: 90, 102 or 114; or
an HCDR1 set forth in SEQ ID NO: 121, 133 or 145, and/or
an HCDR2 set forth in SEQ ID NO: 122, 134 or 146, and/or
an HCDR3 set forth in SEQ ID NO: 123, 135 or 147, and/or
an LCDR1 set forth in SEQ ID NO: 124, 136 or 148, and/or
an LCDR2 set forth in SEQ ID NO: 125, 137 or 149, and/or
an LCDR3 set forth in SEQ ID NO: 126, 138 or 150.

The second group comprises:
an HCDR1 set forth in SEQ ID NO: 19, 31 or 43, and/or
an HCDR2 set forth in SEQ ID NO: 20, 32 or 44, and/or
an HCDR3 set forth in SEQ ID NO: 21, 33 or 45, and/or
an LCDR1 set forth in SEQ ID NO: 22, 34 or 46, and/or
an LCDR2 set forth in SEQ ID NO: 23, 35 or 47, and/or
an LCDR3 set forth in SEQ ID NO: 24, 36 or 48;
an HCDR1 set forth in SEQ ID NO: 55, 67 or 79, and/or
an HCDR2 set forth in SEQ ID NO: 56, 68 or 80, and/or
an HCDR3 set forth in SEQ ID NO: 57, 69 or 81, and/or
an LCDR1 set forth in SEQ ID NO: 58, 70 or 82, and/or
an LCDR2 set forth in SEQ ID NO: 59, 71 or 83, and/or
an LCDR3 set forth in SEQ ID NO: 60, 72 or 84;
an HCDR1 set forth in SEQ ID NO: 91, 103 or 115, and/or
an HCDR2 set forth in SEQ ID NO: 92, 104 or 116, and/or
an HCDR3 set forth in SEQ ID NO: 93, 105 or 117, and/or
an LCDR1 set forth in SEQ ID NO: 94, 106 or 118, and/or
an LCDR2 set forth in SEQ ID NO: 95, 107 or 119, and/or
an LCDR3 set forth in SEQ ID NO: 96, 108 or 120; or
an HCDR1 set forth in SEQ ID NO: 127, 139 or 151, and/or
an HCDR2 set forth in SEQ ID NO: 128, 140 or 152, and/or
an HCDR3 set forth in SEQ ID NO: 129, 141 or 153, and/or
an LCDR1 set forth in SEQ ID NO: 130, 142 or 154, and/or
an LCDR2 set forth in SEQ ID NO: 131, 143 or 155, and/or
an LCDR3 set forth in SEQ ID NO: 132, 144 or 156.

The anti-IL-2 antibody or the antigen-binding fragment thereof of the first group may also have one or more amino acid mutations in the CDR regions. The number of the mutated amino acids may be 1, 2, 3, 4, 5, 6, 7, 8, 9, 10, 11, 12, 13, 14 or 15. In some embodiments, the IL-2 antibody or the antigen-binding fragment thereof of the first group has an avidity for IL-2 with a K_{D} value of > 5 nM or ≥ 10 nM, and ≤ 200 nM or ≤ 150 nM or ≤ 100 nM or ≤ 80 nM or ≤ 60 nM. In some specific embodiments, the IL-2 antibody or the antigen-binding fragment thereof has an avidity for IL-2 with a K_{D} value of > 5 nM and ≤ 60 nM, > 5 nM and ≤ 50 nM, ≥ 10 nM and ≤ 60 nM, ≥ 10 nM and ≤ 50 nM, > 5 nM and ≤ 80 nM, ≥ 10 nM and ≤ 80 nM, > 5 nM and ≤ 100 nM, ≥ 10 nM and ≤ 100 nM, > 5 nM and ≤ 150 nM, or ≥ 10 nM and ≤ 150 nM.

The anti-IL-2 antibody or the antigen-binding fragment thereof of the second group may also have one or more amino acid mutations in the CDR regions. The number of the mutated amino acids may be 1, 2, 3, 4, 5, 6, 7, 8, 9, 10, 11, 12, 13, 14 or 15. In some embodiments, the anti-IL-2 antibody or the antigen-binding fragment thereof of the second group has an avidity for IL-2 with a K_{D} value of ≤ 5 nM.

In some embodiments, provided is an IL-2 antibody or an antigen-binding fragment thereof, which binds to the same antigen or epitope as the anti-IL-2 antibody or the antigen-binding fragment thereof described above, or competes for binding to the same antigen or epitope with the anti-IL-2 antibody or the antigen-binding fragment thereof described above, or binds to or competes for binding to IL-2 with the anti-IL-2 antibody or the antigen-binding fragment thereof described above.

In some embodiments, the anti-IL-2 antibody or the antigen-binding fragment thereof described above reduces or blocks the binding of IL-2 to IL-2Rβ, e.g., blocks the binding of IL-2 to IL-2Rβ.

In some embodiments, the anti-IL-2 antibody or the antigen-binding fragment thereof described above reduces the binding of IL-2 to IL-2Rα.

In some embodiments, the anti-IL-2 antibody or the antigen-binding fragment thereof described above is a fully human antibody or an antigen-binding fragment thereof, or a humanized antibody or an antigen-binding fragment thereof.

In some embodiments, the anti-IL-2 antibody or the antigen-binding fragment thereof described above is an IgG antibody or an antigen-binding fragment thereof, e.g., an IgG1 or IgG4 antibody or an antigen-binding fragment thereof. In some specific embodiments, the anti-IL-2 antibody or the antigen-binding fragment thereof described above is an IgG1 antibody with reduced effector functions, e.g., an IgG1 antibody with reduced or eliminated ADCC function (e.g., an IgG1 antibody with an LALA mutation in Fc).

In some embodiments, the anti-IL-2 antibody or the antigen-binding fragment thereof described above comprises HCDR1-3 and/or LCDR1-3 selected from the group consisting of the following groups:
HCDR1-3 set forth in SEQ ID NOs: 13-15, respectively, and/or LCDR1-3 set forth in SEQ ID NOs: 16-18, respectively;
HCDR1-3 set forth in SEQ ID NOs: 19-21, respectively, and/or LCDR1-3 set forth in SEQ ID NOs: 22-24, respectively;
HCDR1-3 set forth in SEQ ID NOs: 25-27, respectively, and/or LCDR1-3 set forth in SEQ ID NOs: 28-30, respectively;
HCDR1-3 set forth in SEQ ID NOs: 31-33, respectively, and/or LCDR1-3 set forth in SEQ ID NOs: 34-36, respectively;
HCDR1-3 set forth in SEQ ID NOs: 37-39, respectively, and/or LCDR1-3 set forth in SEQ ID NOs: 40-42, respectively;
HCDR1-3 set forth in SEQ ID NOs: 43-45, respectively, and/or LCDR1-3 set forth in SEQ ID NOs: 46-48, respectively;
HCDR1-3 set forth in SEQ ID NOs: 49-51, respectively, and/or LCDR1-3 set forth in SEQ ID NOs: 52-54, respectively;
HCDR1-3 set forth in SEQ ID NOs: 55-57, respectively, and/or LCDR1-3 set forth in SEQ ID NOs: 58-60, respectively;
HCDR1-3 set forth in SEQ ID NOs: 61-63, respectively, and/or LCDR1-3 set forth in SEQ ID NOs: 64-66, respectively;
HCDR1-3 set forth in SEQ ID NOs: 67-69, respectively, and/or LCDR1-3 set forth in SEQ ID NOs: 70-72, respectively;
HCDR1-3 set forth in SEQ ID NOs: 73-75, respectively, and/or LCDR1-3 set forth in SEQ ID NOs: 76-78, respectively;
HCDR1-3 set forth in SEQ ID NOs: 79-81, respectively, and/or LCDR1-3 set forth in SEQ ID NOs: 82-84, respectively;
HCDR1-3 set forth in SEQ ID NOs: 85-87, respectively, and/or LCDR1-3 set forth in SEQ ID NOs: 88-90, respectively;
HCDR1-3 set forth in SEQ ID NOs: 91-93, respectively, and/or LCDR1-3 set forth in SEQ ID NOs: 94-96, respectively;
HCDR1-3 set forth in SEQ ID NOs: 97-99, respectively, and/or LCDR1-3 set forth in SEQ ID NOs: 100-102, respectively;
HCDR1-3 set forth in SEQ ID NOs: 103-105, respectively, and/or LCDR1-3 set forth in SEQ ID NOs: 106-108, respectively;
HCDR1-3 set forth in SEQ ID NOs: 109-111, respectively, and/or LCDR1-3 set forth in SEQ ID NOs: 112-114, respectively;
HCDR1-3 set forth in SEQ ID NOs: 115-117, respectively, and/or LCDR1-3 set forth in SEQ ID NOs: 118-120, respectively;
HCDR1-3 set forth in SEQ ID NOs: 121-123, respectively, and/or LCDR1-3 set forth in SEQ ID NOs: 124-126, respectively;
HCDR1-3 set forth in SEQ ID NOs: 127-129, respectively, and/or LCDR1-3 set forth in SEQ ID NOs: 130-132, respectively;
HCDR1-3 set forth in SEQ ID NOs: 133-135, respectively, and/or LCDR1-3 set forth in SEQ ID NOs: 136-138, respectively;
HCDR1-3 set forth in SEQ ID NOs: 139-141, respectively, and/or LCDR1-3 set forth in SEQ ID NOs: 142-144, respectively;
HCDR1-3 set forth in SEQ ID NOs: 145-147, respectively, and/or LCDR1-3 set forth in SEQ ID NOs: 148-150, respectively; and
HCDR1-3 set forth in SEQ ID NOs: 151-153, respectively, and/or LCDR1-3 set forth in SEQ ID NOs: 154-156, respectively.

In some embodiments, provided is an anti-IL-2 antibody or an antigen-binding fragment thereof, which comprises a VH and a VL, wherein:
(i) as defined according to the Kabat numbering system,
   the VH comprises HCDR1-3 set forth in SEQ ID NOs: 37-39, respectively, and the VL comprises LCDR1-3 set forth in SEQ ID NOs: 40-42, respectively;
   the VH comprises HCDR1-3 set forth in SEQ ID NOs: 13-15, respectively, and the VL comprises LCDR1-3 set forth in SEQ ID NOs: 16-18, respectively;
   the VH comprises HCDR1-3 set forth in SEQ ID NOs: 25-27, respectively, and the VL comprises LCDR1-3 set forth in SEQ ID NOs: 28-30, respectively;
   the VH comprises HCDR1-3 set forth in SEQ ID NOs: 19-21, respectively, and the VL comprises LCDR1-3 set forth in SEQ ID NOs: 22-24, respectively;
   the VH comprises HCDR1-3 set forth in SEQ ID NOs: 31-33, respectively, and the VL comprises LCDR1-3 set forth in SEQ ID NOs: 34-36, respectively; or
   the VH comprises HCDR1-3 set forth in SEQ ID NOs: 43-45, respectively, and the VL comprises LCDR1-3 set forth in SEQ ID NOs: 46-48, respectively;
(ii) as defined according to the Chothia numbering system,
   the VH comprises HCDR1-3 set forth in SEQ ID NOs: 73-75, respectively, and the VL comprises LCDR1-3 set forth in SEQ ID NOs: 76-78, respectively;
   the VH comprises HCDR1-3 set forth in SEQ ID NOs: 49-51, respectively, and the VL comprises LCDR1-3 set forth in SEQ ID NOs: 52-54, respectively;
   the VH comprises HCDR1-3 set forth in SEQ ID NOs: 61-63, respectively, and the VL comprises LCDR1-3 set forth in SEQ ID NOs: 64-66, respectively;
   the VH comprises HCDR1-3 set forth in SEQ ID NOs: 55-57, respectively, and the VL comprises LCDR1-3 set forth in SEQ ID NOs: 58-60, respectively;
   the VH comprises HCDR1-3 set forth in SEQ ID NOs: 67-69, respectively, and the VL comprises LCDR1-3 set forth in SEQ ID NOs: 70-72, respectively; or
   the VH comprises HCDR1-3 set forth in SEQ ID NOs: 79-81, respectively, and the VL comprises LCDR1-3 set forth in SEQ ID NOs: 82-84, respectively;
(iii) as defined according to the IMGT numbering system,
   the VH comprises HCDR1-3 set forth in SEQ ID NOs: 109-111, respectively, and the VL comprises LCDR1-3 set forth in SEQ ID NOs: 112-114, respectively;
   the VH comprises HCDR1-3 set forth in SEQ ID NOs: 85-87, respectively, and the VL comprises LCDR1-3 set forth in SEQ ID NOs: 88-90, respectively;
   the VH comprises HCDR1-3 set forth in SEQ ID NOs: 97-99, respectively, and the VL comprises LCDR1-3 set forth in SEQ ID NOs: 100-102, respectively;
   the VH comprises HCDR1-3 set forth in SEQ ID NOs: 91-93, respectively, and the VL comprises LCDR1-3 set forth in SEQ ID NOs: 94-96, respectively;
   the VH comprises HCDR1-3 set forth in SEQ ID NOs: 103-105, respectively, and the VL comprises LCDR1-3 set forth in SEQ ID NOs: 106-108, respectively; or
   the VH comprises HCDR1-3 set forth in SEQ ID NOs: 115-117, respectively, and the VL comprises LCDR1-3 set forth in SEQ ID NOs: 118-120, respectively;
(iv) as defined according to the AbM numbering system,
   the VH comprises HCDR1-3 set forth in SEQ ID NOs: 145-147, respectively, and the VL comprises LCDR1-3 set forth in SEQ ID NOs: 148-150, respectively;
   the VH comprises HCDR1-3 set forth in SEQ ID NOs: 121-123, respectively, and the VL comprises LCDR1-3 set forth in SEQ ID NOs: 124-126, respectively;
   the VH comprises HCDR1-3 set forth in SEQ ID NOs: 133-135, respectively, and the VL comprises LCDR1-3 set forth in SEQ ID NOs: 136-138, respectively;
   the VH comprises HCDR1-3 set forth in SEQ ID NOs: 127-129, respectively, and the VL comprises LCDR1-3 set forth in SEQ ID NOs: 130-132, respectively;
   the VH comprises HCDR1-3 set forth in SEQ ID NOs: 139-141, respectively, and the VL comprises LCDR1-3 set forth in SEQ ID NOs: 142-144, respectively; or
   the VH comprises HCDR1-3 set forth in SEQ ID NOs: 151-153, respectively, and the VL comprises LCDR1-3 set forth in SEQ ID NOs: 154-156, respectively.

In some embodiments, the anti-IL-2 antibody or the antigen-binding fragment thereof described above comprises:
a VH set forth in one of SEQ ID NOs: 1, 3, 5, 7, 9 and 11, or a VH having at least 90%, 95%, 98% or 99% identity thereto; and/or
a VH set forth in one of SEQ ID NOs: 2, 4, 6, 8, 10 and 12, or a VH having at least 90%, 95%, 98% or 99% identity thereto.

In some embodiments, the anti-IL-2 antibody or the antigen-binding fragment thereof described above comprises a VH and/or a VL selected from the group consisting of the following groups:
a VH set forth in SEQ ID NO: 1 or a VH having at least 90% identity thereto, and a VL set forth in SEQ ID NO: 2 or a VL having at least 90% identity thereto;
a VH set forth in SEQ ID NO: 3 or a VH having at least 90% identity thereto, and a VL set forth in SEQ ID NO: 4 or a VL having at least 90% identity thereto;
a VH set forth in SEQ ID NO: 5 or a VH having at least 90% identity thereto, and a VL set forth in SEQ ID NO: 6 or a VL having at least 90% identity thereto;
a VH set forth in SEQ ID NO: 7 or a VH having at least 90% identity thereto, and a VL set forth in SEQ ID NO: 8 or a VL having at least 90% identity thereto;
a VH set forth in SEQ ID NO: 9 or a VH having at least 90% identity thereto, and a VL set forth in SEQ ID NO: 10 or a VL having at least 90% identity thereto; and
a VH set forth in SEQ ID NO: 11 or a VH having at least 90% identity thereto, and a VL set forth in SEQ ID NO: 12 or a VL having at least 90% identity thereto.

In some embodiments, the anti-IL-2 antibody or the antigen-binding fragment thereof comprises a heavy chain variable region (VH) linked to the heavy chain constant region of human IgG1, and a light chain variable region (VL) linked to the kappa or lamda chain constant region.

In some embodiments, the anti-IL-2 antibody described above comprises:
an HC set forth in one of SEQ ID NOs: 157, 159, 161, 163, 165 and 167, or an HC having at least 80%, 90%, 95%, 98% or 99% identity thereto; and/or
an LC set forth in one of SEQ ID NOs: 158, 160, 162, 164, 166 and 168, or an LC having at least 80%, 90%, 95%, 98% or 99% identity thereto.

In some embodiments, the anti-IL-2 antibody or the antigen-binding fragment thereof described above comprises an HC and an LC selected from the group consisting of the following groups:
a full-length heavy chain (HC) set forth in SEQ ID NO: 157 or a full-length HC having at least 80%, 90%, 95%, 98% or 99% identity thereto, and a full-length light chain (LC) set forth in SEQ ID NO: 158 or a full-length LC having at least 80%, 90%, 95%, 98% or 99% identity thereto;
an HC set forth in SEQ ID NO: 159 or an HC having at least 80%, 90%, 95%, 98% or 99% identity thereto, and an LC set forth in SEQ ID NO: 160 or an LC having at least 80%, 90%, 95%, 98% or 99% identity thereto;
an HC set forth in SEQ ID NO: 161 or an HC having at least 80%, 90%, 95%, 98% or 99% identity thereto, and an LC set forth in SEQ ID NO: 162 or an LC having at least 80%, 90%, 95%, 98% or 99% identity thereto;
an HC set forth in SEQ ID NO: 163 or an HC having at least 90% identity thereto, and an LC set forth in SEQ ID NO: 164 or an LC having at least 80%, 90%, 95%, 98% or 99% identity thereto;
an HC set forth in SEQ ID NO: 165 or an HC having at least 90% identity thereto, and an LC set forth in SEQ ID NO: 166 or an HC having at least 80%, 90%, 95%, 98% or 99% identity thereto; and
an HC set forth in SEQ ID NO: 167 or an HC having at least 80%, 90%, 95%, 98% or 99% identity thereto, and an LC set forth in SEQ ID NO: 168 or an LC having at least 80%, 90%, 95%, 98% or 99% identity thereto.

In some embodiments, the antigen-binding fragment of the anti-IL-2 antibody is Fab, Fv, sFv, Fab', F(ab')₂, a linear antibody, a single-chain antibody, scFv, sdAb, sdFv, a nanobody, a peptibody, a domain antibody, and a multispecific antibody (bispecific antibody, diabody, triabody, and tetrabody, tandem di-scFv, tandem tri-scFv), for example, specifically, scFv, Fv, Fab or Fab' fragment.

### Complex

The present disclosure provides a complex comprising an IL-2 antibody or an antigen-binding fragment thereof and IL-2.

In some specific embodiments, the IL-2 antibody or the antigen-binding fragment thereof and the IL-2 in the complex are bound by non-covalent force, and the IL-2 antibody or the antigen-binding fragment thereof has an avidity for IL-2 with a K_{D} value of ≤ 5 nM.

In other specific embodiments, the IL-2 antibody or the antigen-binding fragment thereof and the IL-2 in the complex are bound by covalent force (e.g., to form a fusion protein), wherein the IL-2 antibody or the antigen-binding fragment thereof has an avidity for IL-2 with a K_{D} value of > 5 nM or ≥ 10 nM, and ≤ 200 nM or ≤ 150 nM or ≤ 100 nM or ≤ 80 nM or ≤ 60 nM. In some specific embodiments, the avidity has a K_{D} value of > 5 nM and ≤ 60 nM, > 5 nM and ≤ 50 nM, ≥ 10 nM and ≤ 60 nM, ≥ 10 nM and ≤ 50 nM, > 5 nM and ≤ 80 nM, ≥ 10 nM and ≤ 80 nM, > 5 nM and ≤ 100 nM, ≥ 10 nM and ≤ 100 nM, > 5 nM and ≤ 150 nM, or ≥ 10 nM and ≤ 150 nM.

In some embodiments, provided is a fusion protein comprising an anti-IL-2 antibody or an antigen fragment thereof and IL-2, wherein the anti-IL-2 antibody or the antigen fragment thereof has an avidity for the IL-2 with a K_{D} value of > 5 nM or ≥ 10 nM, and ≤ 50 nM or ≤ 60 nM or ≤ 80 nM. In some specific embodiments, the anti-IL-2 antibody or the antigen fragment thereof has an avidity for IL-2 with a K_{D} value of ≥ 10 nM and ≤ 60 nM.

In some embodiments, the anti-IL-2 antibody or the antigen fragment thereof and the IL-2 are bound by covalent or non-covalent force. In some embodiments, the anti-IL-2 antibody or the antigen fragment thereof forms a fusion protein with IL-2. IL-2 is linked to the light chain variable region or the heavy chain variable region of the anti-IL-2 antibody or the antigen fragment thereof, e.g., IL-2 is linked to the N-terminus of the light chain variable region or the heavy chain variable region of the anti-IL-2 antibody or the antigen fragment thereof. In some specific embodiments, IL-2 is linked to the N-terminus of the light chain variable region.

In some embodiments, in the fusion protein described above, IL-2 is linked to the anti-IL-2 antibody or the antigen fragment thereof via a linker. The linker is selected from the group consisting of: amino acid sequences represented by (GₘSₙ)ₓ or (GGNGT)ₓ or (YGNGT)ₓ, wherein m and n are each independently selected from the group consisting of integers from 1 to 8 (e.g., 1, 2, 3, 4, 5, 6, 7 or 8), and x is independently selected from the group consisting of integers from 1 to 20 (e.g., 1, 2, 3, 4, 5, 6, 7, 8, 9, 10, 11, 12, 13, 14, 15, 16, 17, 18, 19 or 20). In some specific embodiments, the linker has an amino acid sequence represented by (G₄S)ₓ, wherein x is independently selected from the group consisting of integers from 1 to 6 (e.g., x is 4 or 5). The linker may also be another linker having similar flexibility and length to the amino acid sequence represented by (GₘSₙ)ₓ or (GGNGT)ₓ or (YGNGT)ₓ described above; the linker may also be selected from the group consisting of AKTTPKLEEGEFSEAR, AKTTPKLEEGEFSEARV, AKTTPKLGG, SAKTTPKLGG, AKTTPKLEEGEFSEARV, SAKTTP, SAKTTPKLGG, RADAAP, RADAAPTVS, RADAAAAGGPGS, RADAAAA(G₄S)₄, SAKTTP, SAKTTPKLGG, SAKTTPKLEEGEFSEARV, ADAAP, ADAAPTVSIFPP, TVAAP, TVAAPSVFIFPP, QPKAAP, QPKAAPSVTLFPP, AKTTPP, AKTTPPSVTPLAP, AKTTAP, AKTTAPSVYPLAP, ASTKGP, ASTKGPSVFPLAP, GENKVEYAPALMALS, GPAKELTPLKEAKVS and GHEAAAVMQVQYPAS.

In some embodiments, the complex described above selectively enhances the activity of or promotes the proliferation of cells highly expressing IL-2Ra, e.g., regulatory T (Treg) cells. In some specific embodiments, the complex is a fusion protein formed by an anti-IL-2 antibody or an antigen fragment thereof and IL-2.

In some embodiments, IL-2 in the fusion protein described above has an amino acid substitution at position 3 (as an example, T3) or an N-terminal deletion. In some specific embodiments, the amino acid substitution at position 3 of IL-2 (as an example, T3A, T3Q or T3E) is Ala, Gln or Glu substitution, and the N-terminal deletion is deletion of first 3 or 7 amino acids at the N-terminus.

In some embodiments, the fusion protein described above comprises an HC and an LC selected from the group consisting of the following groups:
an HC set forth in SEQ ID NO: 169, and an LC set forth in SEQ ID NO: 158;
an HC set forth in SEQ ID NO: 171, and an LC set forth in SEQ ID NO: 160;
an HC set forth in SEQ ID NO: 159, and an LC set forth in SEQ ID NO: 172;
an HC set forth in SEQ ID NO: 173, and an LC set forth in SEQ ID NO: 162;
an HC set forth in SEQ ID NO: 175, and an LC set forth in SEQ ID NO: 164;
an HC set forth in SEQ ID NO: 163, and an LC set forth in SEQ ID NO: 176;
an HC set forth in SEQ ID NO: 177, and an LC set forth in SEQ ID NO: 166;
an HC set forth in SEQ ID NO: 179, and an LC set forth in SEQ ID NO: 168;
an HC set forth in SEQ ID NO: 167, and an LC set forth in SEQ ID NO: 180;
an HC set forth in SEQ ID NO: 161, and an LC set forth in any one of SEQ ID NOs: 174 and 181-186;
an HC set forth in SEQ ID NO: 157, and an LC set forth in SEQ ID NO: 170 or 187;
an HC set forth in SEQ ID NO: 165, and an LC set forth in any one of SEQ ID NOs: 178, 188 and 189; and
sequences having at least 80%, 90%, 95%, 98% or 99% identity to the HC and LC described above.

In some embodiments, the complex of the present disclosure described above selectively enhances the effect of IL-2 on the activity of Tregs (e.g., proliferation-promoting activity and STAT5 phosphorylation-promoting activity), and/or selectively increases the ability of IL-2 to promote the expression of one or more of FOXP3, CD25 and Icos in Treg cells.

In some embodiments, the complex of the present disclosure also has one or more of (i)-(iii):
(i) a higher degree of activation on the activity of Tregs than that of CD8⁺ T cells;
(ii) a higher degree of promotion of STAT5 phosphorylation in Treg cells than that in CD8⁺ T cells; and
(iii) a higher degree of promotion of proliferation of Treg cells than that of CD8⁺ T cells, CD4⁺ T cells, or NK cells.

In some embodiments, the Fc region of the anti-IL-2 antibody or the antigen-binding fragment thereof of the present disclosure comprises one or more amino acid substitutions, wherein the one or more amino acid substitutions reduce the binding of the Fc region to an Fc receptor, e.g., the binding of the Fc region to an Fcγ receptor, and reduce or eliminate effector functions. In some specific embodiments, the anti-IL-2 antibody or the antigen-binding fragment thereof of the present disclosure has reduced or eliminated ADCC function.

In some embodiments, the anti-IL-2 antibody or the antigen-binding fragment thereof of the present disclosure has an engineered Fc region, wherein the engineered Fc region has a binding avidity for an Fc receptor decreased by 50%, 80%, 90% or 95% or more as compared to the native Fc region. In some embodiments, the Fc receptor is an Fcγ receptor. In some embodiments, the Fc receptor is a human Fcγ receptor, e.g., FcγRI, FcγRIIa, FcγRIIB, or FcγRIIIa. In some embodiments, the engineered Fc region also has a reduced binding avidity for a complement (e.g., C1q) as compared to the native Fc region. In some embodiments, the engineered Fc region has no reduced binding avidity for neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming.

In some embodiments, for the IgGi Fc region, amino acid residue substitutions at positions such as positions 238, 265, 269, 270, 297, 327 and 329 can reduce effector functions. In some embodiments, the Fc region is a human IgG₁ Fc region, and the amino acid residues at positions 234 and 235 are A, as numbered according to the EU index. For the IgG₄ Fc region, amino acid residue substitutions at positions such as position 228 can reduce effector functions. Amino acid substitutions that alter effector functions are selected from the group consisting of one or more of the following groups: S298A/E333A/K334A; S239D/I332E/A330L; S239D/I332E/G236A; G236A/S239D/A330L/I332E; F243L/R292P/Y300L/V305I/P396L; K326A/E333A; K326W/E333S; K326M/E333S; C221D/D222C; S267E/H268F/S324T; E345R; S298A/E333A/K334A/N434A; E294 deletion/T307P/N434Y; T256N/A378V/S383N/N434Y; T252L/T253S/T254F; M252Y/S254T/T256E; M428L/N434S; L234A/L235A; S228P/L235E; L234A/L235A/P331S; L234A/L235A/P329G; D265A/E233P; H268Q/V309L/A330S/P331S; V234A/G237A/P238S/H268A/V309L/A300S/P331S; L234A/L235A/G237A/P238S/H268A/V309L/A300S/P331S; S228P/F234A/L235A; D270A/P329A; L234F/L235E; L234F/L235E/P331S; F241A/V264A/D265A; N297G/D265A; and L234Y/G236W/S298A.

The present disclosure provides an anti-IL-2 antibody or an antigen-binding fragment thereof that has a moderate avidity for IL-2, wherein the moderate avidity is an avidity of the antibody for IL-2 with a K_{D} value of ≥ 5 nM or ≥ 10 nM, and ≤ 200 nM or ≤ 150 nM or ≤ 100 nM or ≤ 80 nM or ≤ 60 nM (e.g., ≥ 10 nM and ≤ 50 nM). When the antibody forms a fusion protein with IL-2, the antibody is linked to IL-2 via a linker. In addition, the antibody and IL-2 are bound by a non-covalent bond, but the binding degree (i.e., avidity) thereof by the non-covalent bond is not higher than the avidity of IL-2Rα for IL-2. Therefore, when the fusion protein is contacted with IL-2Rα on the cell surface, the non-covalent bond between the anti-IL-2 antibody and IL-2 is dissociated, and IL-2 binds to IL-2Rα, thereby activating the cell. Tregs continuously express the high-binding avidity receptor IL-2Rα/β/γ, while unactivated CD8⁺ T cells, NK cells and the like only express the medium-binding avidity receptor IL-2Rβ/γ. Therefore, fusion proteins of such antibodies with IL-2 can selectively or preferentially activate Tregs. Such antibodies provided by the present disclosure include, e.g., A2-22, B2-15, D2-60 and antibodies having identical HCDR1-3 and LCDR1-3 thereto.

The present disclosure also provides an anti-IL-2 antibody or an antigen-binding fragment thereof that has a high avidity for IL-2, wherein the high avidity is an avidity of the antibody for IL-2 with a K_{D} value of ≤ 5 nM. The antibody and IL-2 can be bound by a non-covalent bond, and the binding degree (i.e., avidity) thereof by the non-covalent bond is similar to or slightly greater than the avidity of IL-2Rα for IL-2. Therefore, when the complex of the antibody and IL-2 (by a non-covalent bond) is contacted with IL-2Rα on the cell surface, the non-covalent bond between the anti-IL-2 antibody and IL-2 is dissociated or partially dissociated, and IL-2 binds to IL-2Rα, thereby activating the cell. Therefore, complexes of such antibodies and IL-2 (by a non-covalent bond) can selectively or preferentially activate Tregs. The present disclosure does not exclude fusion protein forms of such antibodies and IL-2. Such antibodies provided by the present disclosure include, e.g., C2-53, D3-68 and antibodies having identical HCDR1-3 and LCDR1-3 thereto.

In the present disclosure, the avidity of the anti-IL-2 antibody or the antigen-binding fragment thereof for IL-2 is detected by a conventional method in the art. When the avidity is expressed by a K_{D} value, it is detected by, for example, Biacore (see Example 6 for a specific detection method). When the avidity is expressed by an EC₅₀ value, it is detected by, for example, ELISA (see Example 5 for a specific detection method).

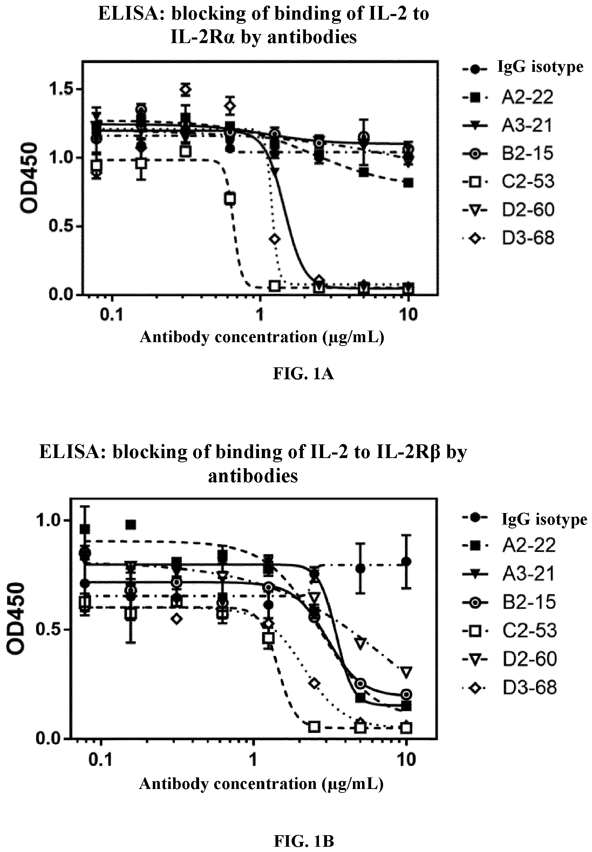

In some embodiments, part of the anti-IL-2 antibodies or the antigen-binding fragments thereof of the present disclosure can reduce the binding of IL-2 to IL-2Rα, as detected by an ELISA assay (see Example 3 for details); or by an Octet assay (see Example 4 for details). In those embodiments, an antibody having HCDR1-3 and LCDR1-3 with sequences set forth in SEQ ID NOs: 9 and 10, respectively (e.g., D2-60), an antibody having HCDR1-3 and LCDR1-3 with sequences set forth in SEQ ID NOs: 5 and 6, respectively (e.g., B2-15), and an antibody having HCDR1-3 and LCDR1-3 with sequences set forth in SEQ ID NOs: 1 and 3, respectively (e.g., A2-22) cannot block the binding of IL-2 to IL-2Rα, but only can reduce it. Herein, the expression "cannot block the binding of IL-2 to IL-2Rα" means that no significant IC₅₀ values can be obtained by fitting the curve at the high antibody concentrations of Example 3 (e.g., 10 µg/mL). The expression "reduce" means that the binding of IL-2 to IL-2Rα can still be affected to a certain extent under the conditions of Example 3 (e.g., FIG. 1A), or there is a blocking value for IL-2Rα of greater than 0.600 (e.g., 0.700, 0.754, or 0.848) at an Octet level under the experimental conditions of Example 4.

In some embodiments, part of the anti-IL-2 antibodies or the antigen-binding fragments thereof of the present disclosure can block the binding of IL-2 to IL-2Rα, as detected by an ELISA assay (see Example 3 for details); or by an Octet assay (see Example 4 for details). In those embodiments, an antibody having HCDR1-3 and LCDR1-3 with sequences set forth in SEQ ID NOs: 3 and 4, respectively (e.g., A3-21), an antibody having HCDR1-3 and LCDR1-3 with sequences set forth in SEQ ID NOs: 7 and 8, respectively (e.g., C2-53), and an antibody having HCDR1-3 and LCDR1-3 with sequences set forth in SEQ ID NOs: 11 and 12, respectively (e.g., D3-68) can block the binding of IL-2 to IL-2Rα. The expression "block the binding of IL-2 to IL-2Rα" means that an IC₅₀ value (e.g., 0.2-5 µg/mL, further, e.g., 0.5-2 µ g/mL) of effectively blocking the binding of IL-2 to IL-2Rα can be obtained under the conditions of Example 3, or there is a blocking value for IL-2Rα of less than 0.600 (e.g., 0.410, 0.428, or 0.473) at an Octet level under the experimental conditions of Example 4.

In some embodiments, part of the anti-IL-2 antibodies or the antigen-binding fragments thereof of the present disclosure all can block the binding of IL-2 to IL-2Rβ, as detected by an ELISA assay (see Example 3 for details); or by an Octet assay (see Example 4 for details). In some embodiments, an antibody having HCDR1-3 and LCDR1-3 with sequences set forth in SEQ ID NOs: 9 and 10, respectively (e.g., D2-60), an antibody having HCDR1-3 and LCDR1-3 with sequences set forth in SEQ ID NOs: 5 and 6 (e.g., B2-15), an antibody having HCDR1-3 and LCDR1-3 with sequences set forth in SEQ ID NOs: 1 and 3 (e.g., A2-22), an antibody having HCDR1-3 and LCDR1-3 with sequences set forth in SEQ ID NOs: 1 and 2 (e.g., A3-21), an antibody having HCDR1-3 and LCDR1-3 with sequences set forth in SEQ ID NOs: 7 and 8 (e.g., C2-53), and an antibody having HCDR1-3 and LCDR1-3 with sequences set forth in SEQ ID NOs: 11 and 12 (e.g., D3-68) all can block the binding of IL-2 to IL-2Rβ. Herein, the expression "block the binding of IL-2 to IL-2Rβ means that an IC₅₀ value (e.g., 0.1-9 µg/mL, further, e.g., 1-9 µg/mL) of effectively blocking the binding of IL-2 to IL-2Rβ is obtained under the conditions of Example 3, or there is a blocking value for IL-2Rβ of less than 0.300 (e.g., less than 0.2, further, e.g., less than 0.1) at an Octet level under the conditions of Example 3.

Both IL-2 and the complex of the present disclosure can activate T cells (e.g., Tregs and CD8⁺ T cells). The expression "selectively enhance the effect of IL-2 on the activity of Tregs" means that if the effect of IL-2 on the activity of Treg cells is set as A, and that on the activity of CD8⁺ T cells is set as B; and the effect of the complex of the present disclosure on the activity of Treg cells is set as A', and that on the activity of CD8⁺ T cells is set as B', the ratio of A'/B' is higher than the ratio of A/B (to any extent). The "activity" is obtained under equivalent assay conditions, including but not limited to EC₅₀ of promoting cell proliferation and EC₅₀ of promoting STAT5 phosphorylation (as in the assay of Example 10) of a test sample (e.g., IL-2 or the complex of the present disclosure).

In the present disclosure, the expression "having a higher degree of activation on the activity of Tregs than that of CD8⁺ T cells" has an equivalent meaning to the expression "having a lower degree of activation on the activity of Tregs than that of CD8⁺ T cells", and means that the anti-IL-2 antibody, or the antigen-binding fragment or complex thereof of the present disclosure has a higher degree of activation on the activity of Tregs than that of CD8' T cells (to any extent).

In the present disclosure, the expression "having a higher degree of promotion of STAT5 phosphorylation in Treg cells than that in CD8⁺ T cells" has an equivalent meaning to the expression "having a lower degree of inhibition of STAT5 phosphorylation in Treg cells than that in CD8⁺ T cells", and means that the anti-IL-2 antibody, or the antigen-binding fragment or complex thereof of the present disclosure has a higher degree of promotion of STAT5 phosphorylation in Treg cells than that in CD8⁺ T cells (to any extent). See Example 7 for the detection method of STAT5 phosphorylation.

In the present disclosure, the expression "having a higher degree of promotion of proliferation of Treg cells than that of CD8⁺ T cells, CD4⁺ T cells, or NK cells" has an equivalent meaning to the expression "having a lower degree of inhibition of proliferation of Treg cells than that of CD8⁺ T cells, CD4⁺ T cells, or NK cells", and means that the anti-IL-2 antibody, or the antigen-binding fragment or complex thereof of the present disclosure has a higher degree of promotion of proliferation of Treg cells than that of CD8⁺ T cells (or CD4⁺ T cells, or NK cells) (to any extent).

The expression "increasing the expression of one or more of FOXP3, CD25, and Icos in Treg cells" encompasses increase in the expression of one or more of FOXP3, CD25, and Icos in Tregs by the complex of the present disclosure to any extent; or a lower degree of inhibition of expression of one or more of FOXP3, CD25 and Icos in Tregs than that in CD8⁺ T cells, CD4⁺ T cells, or NK cells, by the anti-IL-2 antibody, or the antigen-binding fragment or complex thereof of the present disclosure to any extent.

### Polynucleotide

The present disclosure provides an isolated polynucleotide encoding the anti-IL-2 antibody or the antigen-binding fragment thereof described above, or encoding the complex (e.g., fusion protein) described above. The polynucleotide may be DNA or RNA.

The present disclosure provides an expression vector comprising the polynucleotide described above, which may be a eukaryotic expression vector, a prokaryotic expression vector, or a viral vector, e.g., a plasmid, a cosmid, or a phage.

### Host Cell

The present disclosure provides a host cell transformed with the expression vector described above, which may be a eukaryotic cell or a prokaryotic cell.

In some embodiments, the host cell is a bacterium, a yeast or a mammalian cell. In some specific embodiments, the host cell is *Escherichia coli, Pichia pastoris,* a Chinese hamster ovary (CHO) cell, or a human embryonic kidney (HEK) 293 cell.

### Preparation Method

The present disclosure provides a method for preparing an anti-IL-2 antibody or an antigen-binding fragment thereof, or an anti-IL-2 antibody or a complex (e.g., fusion protein) thereof, which comprises:
- expressing the antibody, or the antigen-binding fragment or complex (e.g., fusion protein) thereof in the host cell described above, and
- isolating the antibody, or the antigen-binding fragment or complex (e.g., fusion protein) thereof from the host cell.

Optionally, the preparation method of the present disclosure may further comprise a purification step. For example, purification is performed using an A or G Sepharose FF column containing an adjusted buffer to wash off non-specifically bound components, and then bound antibodies are eluted by a pH gradient method, detected by SDS-PAGE, and collected. Optionally, the antibody solution can be filtered and concentrated by a conventional method. Soluble mixtures and polymers can also be removed by a conventional method, such as a molecular sieve and ion exchange. The resulting product needs to be immediately frozen, e.g., at -70 °C, or lyophilized.

Methods for producing and purifying antibodies and antigen-binding fragments are well known in the prior art and can be found in, for example, *"*Antibodies: A Laboratory Manual", Cold Spring Harbor Press (chapters 5-8 and 15). For example, mice can be immunized with human IL-2 or a fragment thereof, and the resulting antibodies can be renatured and purified, and amino acid sequencing can be performed by a conventional method. Similarly, antigen-binding fragments can be prepared by a conventional method. The antibody or the antigen-binding fragment described in the present invention is genetically engineered to contain one or more additional human FRs in the non-human-derived CDRs. Human FR germline sequences can be obtained from the ImMunoGeneTics (IMGT) database or from the Immunoglobulin Journal, 2001ISBN012441351.

The engineered antibody or antigen-binding fragment of the present disclosure can be prepared and purified by a conventional method. For example, cDNA sequences encoding the heavy and light chains can be cloned and recombined into an expression vector. Recombinant immunoglobulin expression vectors can be stably transfected into CHO cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture medium with the secreted antibody can be purified and collected by a conventional technique. The antibody can be filtered and concentrated by a conventional method. Soluble mixtures and polymers can also be removed by a conventional method, such as a molecular sieve and ion exchange.

### Composition

The present disclosure provides a composition, e.g., a pharmaceutical composition, comprising a therapeutically effective amount of the anti-IL-2 antibody, or the antigen-binding fragment or complex (e.g., fusion protein) thereof described above, and a pharmaceutically acceptable excipient, diluent or carrier. In some specific embodiments, the pharmaceutical composition may contain 0.01 wt% to 99 wt% of the anti-IL-2 antibody or the antigen-binding fragment thereof, or the anti-IL-2 antibody or the complex (e.g., fusion protein) thereof in a unit dose, or the pharmaceutical composition may contain 0.1-2000 mg, and in some specific embodiments, 1-1000 mg, of the anti-IL-2 antibody or the antigen-binding fragment thereof, or the anti-IL-2 antibody or the complex (e.g., fusion protein) thereof in a unit dose.

### Method and Use

The present disclosure provides use of a substance selected from the group consisting of any one of or a combination of the following in the preparation of a medicament or a pharmaceutical composition: the anti-IL-2 antibody, or the antigen-binding fragment or complex (e.g., fusion protein) thereof described above, and a coding polynucleotide.

In some embodiments, the medicament or pharmaceutical composition described above is used for treating or preventing an immune-related disorder (e.g., an autoimmune disease or an inflammatory disease) or delaying progression of immune-related disorders.

In some embodiments, provided is a method for treating or preventing autoimmune diseases or delaying progression of an autoimmune disease, which comprises administering to a subject the anti-IL-2 antibody, or the antigen-binding fragment or complex (e.g., fusion protein) thereof according to the present disclosure, or the pharmaceutical composition according to the present disclosure in an effective amount for treating or delaying the disease.

In some embodiments, the autoimmune disease or inflammatory disease described above is selected from the group consisting of any one of the following: inflammatory dermatosis including psoriasis and dermatitis (e.g., atopic dermatitis); dermatomyositis; systemic scleroderma and sclerosis; conditions associated with inflammatory bowel disease (e.g., Crohn's disease and ulcerative colitis); colitis; gastritis; respiratory distress syndrome (including adult respiratory distress syndrome and ARDS); dermatitis; meningitis; encephalitis; uveitis; glomerulonephritis; allergic conditions such as eczema and asthma and other conditions involving T cell infiltration and chronic inflammatory responses; atherosclerosis; leukocyte adhesion deficiency; rheumatoid arthritis; systemic lupus erythematosus (SLE); diabetes (e.g., type I diabetes); multiple sclerosis; Raynaud's syndrome; autoimmune thyroiditis; allergic encephalomyelitis; sicca syndrome; juvenile onset diabetes; immune responses associated with acute and delayed hypersensitivity reactions mediated by cytokines and T lymphocytes commonly found in tuberculosis, sarcoidosis, polymyositis, granulomatosis and vasculitis; Wegener's disease; pernicious anemia (Addison's disease); diseases involving leukopedesis; inflammatory disorders of central nervous system (CNS); multiple organ injury syndrome; hemolytic anemia (including but not limited to cryoglobulinemia or Coombs positive anemia); myasthenia gravis; antigen-antibody complex mediated diseases; anti-glomerular basement membrane; antiphospholipid syndrome; allergic neuritis; Graves disease; Lang-Ellis myasthenia syndrome; pemphigoid; pemphigus; autoimmune polyendocrine gland disease; vitiligo; Bright's disease; stiff-person syndrome; Behcet's disease; giant cell arteritis; immune complex nephritis; IgA nephropathy; IgM polyneuropathy; immune thrombocytopenic purpura (ITP) or autoimmune thrombocytopenia and autoimmune hemolytic disease; Hashimoto's thyroiditis; autoimmune hepatitis; autoimmune hemophilia; autoimmune lymphoproliferative syndrome (ALPS); experimental autoimmune uveoretinitis; Guillain-Barre syndrome; Goodpasture's syndrome; mixed connective tissue disease; autoimmune infertility; polyarteritis nodosa; alopecia areata; idiopathic myxoedema; graft versus host disease; and muscular dystrophy (Duchenne muscular dystrophy, Becker muscular dystrophy, myotonic dystrophy, limb-girdle muscular dystrophy, facioscapulohumeral muscular dystrophy, congenital muscular dystrophy, oculopharyngeal muscular dystrophy, distal dystrophy and Emery-Dreifuss muscular dystrophy), spontaneous disappearance of pregnancy, atopic diseases or inflammatory bowel disease.

In some embodiments, the autoimmune disease or inflammatory disease is selected from the group consisting of: lupus, graft versus host disease, hepatitis C virus-induced vasculitis, type I diabetes, multiple sclerosis, atopic diseases and inflammatory bowel disease.

In some embodiments, provided is a method for increasing the ratio of Tregs to non-Tregs in a population of T cells, which comprises contacting the population of T cells with an effective amount of the anti-IL-2 antibody, or the antigen-binding fragment or complex (e.g., fusion protein) thereof described above. In one embodiment, the ratio of CD3⁺FoxP3⁺ cells to CD3⁺FoxP3⁻ is increased. In another embodiment, the ratio of CD3⁺FoxP3⁺ cells to CD3⁺FoxP3⁻ is increased by at least 50%.

In some embodiments, provided is a method for increasing the ratio of Tregs to non-Tregs in peripheral blood of a subject, which comprises contacting the population of T cells with an effective amount of the anti-IL-2 antibody, or the antigen-binding fragment or complex (e.g., fusion protein) thereof described above. In one embodiment, the ratio of CD3⁺FoxP3⁺ cells to CD3⁺FoxP3⁻ is increased. In another embodiment, the ratio of CD3^{÷}FoxP3^{÷} cells to CD3⁺FoxP3⁻ is increased by at least 50%.

In some embodiments, provided is a method for any one selected from the group consisting of the following: increasing the ratio of Tregs in a population of T cells to natural killer (NK) cells, or increasing the number of Tregs, or increasing the activity of Tregs, wherein the method comprises administering an effective amount of the anti-IL-2 antibody, or the antigen-binding fragment or complex (e.g., fusion protein) thereof described above. In one embodiment, the ratio of CD3⁺FoxP3⁺ cells to CD3⁻CD19⁻lymphocytes expressing CD56 and/or CD16 is increased. In another embodiment, the ratio of CD3⁺FoxP3⁺ cells to CD3⁻CD19⁻ lymphocytes expressing CD56 and/or CD16 is increased by at least 50%.

In some embodiments, provided is a method for any one selected from the group consisting of the following: increasing the ratio of Tregs to natural killer (NK) cells in peripheral blood of a subject, or increasing the number of Tregs, or increasing the activity of Tregs, wherein the method comprises administering an effective amount of the anti-IL-2 antibody, or the antigen-binding fragment or complex (e.g., fusion protein) thereof described above. In one embodiment, the ratio of CD3⁺FoxP3⁺ cells to CD3⁻CD19⁻ lymphocytes expressing CD56 and/or CD16 is increased. In another embodiment, the ratio of CD3⁺FoxP3⁺ cells to CD3⁻CD19⁻ lymphocytes expressing CD56 and/or CD16 is increased by at least 50%.

In some embodiments, provided is a method for monitoring the response of a subject to the anti-IL-2 antibody, or the antigen-binding fragment or complex (e.g., fusion protein) thereof described above, which comprises detecting a change in the subject selected from the group consisting of any one or a combination of the following: an increase in body temperature, an increase in CRP in peripheral blood of the subject, a decrease in platelets in peripheral blood of the subject, a decrease in neutrophils in peripheral blood of the subject, and a decrease in albumin in peripheral blood of the subject. After the change is detected, the treatment is discontinued or interrupted, the frequency of administration is reduced, or the dose is reduced. In one embodiment, the change comprises a change selected from the group consisting of any one or a combination of the following: an increase in body temperature of at least 0.5 °C, an increase in CRP in peripheral blood of the subject of at least 0.2 mg/mL, a decrease in platelets in peripheral blood of at least 0.8 times, a decrease in neutrophils in peripheral blood of the subject of at least 0.8 times, and a decrease in albumin in peripheral blood of the subject of at least 0.4 times.

In some embodiments, the anti-IL-2 antibody, or the antigen-binding fragment or complex (e.g., fusion protein) thereof described above can specifically bind to IL-2 and reduce the binding of IL-2 to IL-2Rα and/or IL-2Rβ. Those antibodies, antigen-binding fragments or complexes inhibit the proliferation of non-Treg cells (including effector CD8⁺, non-Treg CD4⁺, and NK cells) more intensely than the inhibition of Treg cells; and/or increase the proliferation of Tregs as compared to isotype control antibodies; and/or increase the ratio of Treg cells/non-Treg cells; or maintaining a Treg marker.

### BRIEF DESCRIPTION OF THE DRAWINGS

FIGs. 1A and 1B show the results of ELISA assay on the blocking of the binding of IL-2 to IL-2Rα and IL-2Rβ by anti-IL-2 antibodies, respectively.
FIG. 2 shows the results of ELISA assay on the binding ability of anti-IL-2 antibodies to IL-2.
FIGs. 3A-3H show the experimental results of stimulation of STAT5 phosphorylation in Tregs and CD8⁺ T cells in human peripheral blood by non-covalent complexes of anti-IL-2 antibody and IL-2.
FIGs. 4A-4D show the experimental results of activity of fusion proteins for stimulation of STAT5 phosphorylation in regulatory T cells (Tregs) and CD8⁺ T cells in human peripheral blood.
FIGs. 5A-5F show the experimental results of the effect of fusion proteins on the proliferation of lymphocytes in peripheral blood of Balb/c mice.
FIG. 6 shows the experimental results of the effect of fusion proteins on the proliferation of lymphocytes in the spleen of OVA-immunized mice.
FIG. 7 shows the results of the efficacy of fusion proteins in the delayed hypersensitivity mouse model.
FIGs. 8A-8B show the results of the efficacy of fusion proteins in the arthritis mouse model.
FIGs. 9A-9F show the experimental results of activity of fusion proteins formed by anti-IL-2 antibody and different IL-2 variants for stimulation of STAT5 phosphorylation in regulatory T cells (Tregs) and CD8⁺ T cells in human peripheral blood.
FIG. 10 shows the experimental results of the effect of D2-60-b-T3E on the protein content in the urine of MRL/lpr mice (n = 7-10).
FIGs. 11A and 11B show the experimental results of the effect of D2-60-b-T3E on the content of dsDNA IgG and dsDNA IgM in serum of MRL/lpr mice, respectively (^{∗∗}P < 0.01 vs. model control group).
FIGs. 12A and 12B show the experimental results of the effect of D2-60-b-T3E on renal function of MRL/lpr mice, respectively. ^{∗} P < 0.05, ^{∗∗}P < 0.01, ^{∗∗∗∗}P < 0.0001 vs. model control group, n = 9-10.
FIGs. 13A and 13B show the experimental results of the effect of D2-60-b-T3E on the content of IL-6 and INF-γ in serum of MRL/lpr mice, respectively. ^{∗} P < 0.05, ^{∗∗}P < 0.01, ^{∗∗∗∗}P < 0.0001 vs. model control group, n = 9-10.
FIG. 14 shows the experimental results of the effect of D2-60-b-T3E on the content of IL-10 in serum of MRL/lpr mice. ^{∗} P < 0.05, ^{∗∗}P < 0.01, ^{∗∗∗∗}P < 0.0001 vs. model control group, n = 9-10.
FIG. 15 shows the effect of D2-60-b-T3E on the pathological score of MRL/lpr mice. ^{∗} P < 0.05, ^{∗∗}P < 0.01, ^{∗∗∗∗}P < 0.0001 vs. model control group, n = 9-10.

### DETAILED DESCRIPTION

### Terms

In order to facilitate the understanding of the present disclosure, some technical and scientific terms are specifically defined below. Unless otherwise specifically defined elsewhere herein, all other technical and scientific terms used herein have the meanings generally understood by those of ordinary skill in the art to which the present disclosure belongs.

The three-letter and single-letter codes for amino acids used in the present disclosure are described as in J. Biol. Chem, 243, p3558 (1968).

"Interleukin-2" or "IL-2" is intended to be interpreted broadly and includes any IL-2 related product, including but not limited to, human and non-human IL-2 homologs, fragments or truncations, fusion proteins (e.g., fused to a signal peptide or other active components (e.g., an antibody or antigen-binding fragment thereof) and inactive components), modified forms (e.g., PEGylated, glycosylated, albumin conjugated/fused, Fc conjugated and/or fused, and hydroxyethylated forms), conservatively modified proteins, etc. The term encompasses any natural IL-2 of any vertebrate source, including mammals such as primates (e.g., humans) and rodents (e.g., mice and rats). The term also encompasses an unprocessed IL-2 as well as any form of processed IL-2 derived from cells; naturally occurring IL-2 variants, such as splice variants or allelic variants; and conservatively modified variants of IL-2.

"IL-2Rα", "CD25" or "α subunit of IL-2 receptor" refers to any natural CD25 of any vertebrate source, including mammals such as primates (e.g., humans) and rodents (e.g., mice and rats). The term encompasses an unprocessed "full-length" CD25 as well as any form of processed CD25 derived from cells; and naturally occurring CD25 variants, such as splice variants or allelic variants. In certain embodiments, CD25 is human CD25, with an illustrative sequence set forth in SEQ ID NO: 37.

A "high avidity IL-2 receptor" refers to the heterotrimer form of the IL-2 receptor that consists of a receptor γ subunit (also known as a common cytokine-receptor γ subunit, γc, or CD132), a receptor β subunit (also known as CD122, p70, or IL-2Rβ), and a receptor α subunit (also known as CD25, p55, or IL-2Rα). In contrast, a "moderate avidity IL-2 receptor" refers to an IL-2 receptor that comprises only γ and β subunits but no α subunit (see, e.g., Olejniczak and Kasprzak, MedSci Monit 14, RA179-189 (2008)). "Conservative modifications" is applicable to amino acid and nucleotide sequences. For particular nucleotide sequences, conservative modifications refer to those nucleic acids encoding identical or substantially identical amino acid sequences, or, in the case of nucleotides not encoding amino acid sequences, to substantially identical nucleotide sequences. For amino acid sequences, "conservative modifications" refer to the replacement of amino acids in a protein with other amino acids having similar characteristics (e.g., charge, side chain size, hydrophobicity/hydrophilicity, backbone conformation, and rigidity) such that changes can be made frequently without altering the biological activity of the protein. Those skilled in the art recognize that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)).

"Amino acid mutations" include amino acid substitutions, deletions, insertions, modifications, and any combination thereof, to obtain a final construct that possesses desired properties, such as enhanced stability and increased activity. Amino acid sequence deletions and insertions include amino-terminal and/or carboxyl-terminal deletions and amino acid insertions. Preferred amino acid mutations are amino acid substitutions. To alter the binding properties of, for example, an anti-IL-2 antibody or an antigen-binding fragment thereof, non-conservative amino acid substitutions may be made, i.e., one amino acid is replaced with another amino acid having different structural and/or chemical properties. Preferred amino acid substitutions include the replacement of hydrophobic amino acids with hydrophilic amino acids. Amino acid substitutions include the replacement with non-naturally occurring amino acids or with naturally occurring amino acid derivatives of the 20 standard amino acids (e.g., 4-hydroxyproline, 3-methylhistidine, ornithine, homoserine, and 5-hydroxylysine). Amino acid mutations can be generated using genetic or chemical methods well known in the art, including site-directed mutagenesis, PCR, gene synthesis, chemical modification, and the like.

An "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding portions) so long as they exhibit the desired antigen-binding activity. An antibody may refer to an immunoglobulin, which is of a tetrapeptide chain structure formed by two heavy chains and two light chains linked by interchain disulfide bonds. The heavy chain constant regions of an immunoglobulin differ in their amino acid composition and arrangement, and thus in their antigenic specificity. Accordingly, immunoglobulins can be divided into five classes, or isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the composition of amino acids of the hinge regions and the number and positions of disulfide bonds of the heavy chains; for example, IgG can be divided into IgG1, IgG2, IgG3 and IgG4. Light chains are classified into κ or λ chains according to differences in the constant regions. Each of the five classes of Ig may have a κ chain or λ chain. In the heavy and light chains of antibody, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity determining regions (CDRs). Each of the light chain variable regions (VLs) and the heavy chain variable region (VHs) consists of 3 CDR regions and 4 FR regions arranged from the amino terminus to the carboxyl terminus in the following order: FR1, CDR1, FR2, CDR2, FR3, CDR3, and FR4. The 3 CDR regions of the light chain refer to LCDR1, LCDR2, and LCDR 3; and the 3 CDR regions of the heavy chain refer to HCDR1, HCDR2 and HCDR3.

For determination or definition of CDRs, the deterministic depiction of CDRs can be accomplished by resolving the structure of the antibody and/or resolving the structure of the antibody-ligand complex, and identifying residues involved in the antigen binding site. This can be accomplished by any of a variety of techniques known to those skilled in the art, such as X-ray crystallography. A variety of analysis methods can be used to identify CDRs, including but not limited to Kabat numbering system, Chothia numbering system, AbM numbering system, IMGT numbering system, contact definition, and conformational definition.

The Kabat numbering system is a standard for numbering residues in antibodies and is generally used to identify CDR regions (see, e.g., Johnson & Wu, 2000, Nucleic Acids Res., 28: 214-8).

The Chothia numbering system is similar to the Kabat numbering system, except that it takes into account the location of certain structural loop regions (see, e.g., Chothia et al., 1986, J. Mol. Biol., 196: 901-17; Chothia et al., 1989, Nature, 342: 877-83).

The AbM numbering system adopts a computer program integration suite for modeling antibody structures manufactured by Oxford Molecular Group (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd.). The AbM numbering system adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3: 194-198).

The contact definition is based on the analysis of the available complex crystal structures (see, e.g., MacCallum et al., 1996, J. Mol. Biol., 5: 732-45).

In the conformational definition, the position of the CDRs can be identified as residues that contribute enthalpy to the antigen binding (see, e.g., Makabe et al., 2008, Journal of Biological Chemistry, 283: 1156-1166).

Other CDR boundary definitions may not strictly follow one of the above methods, but still overlap with at least a portion of the Kabat CDRs, although they may be shortened or lengthened based on predictions or experimental results that a particular residue or a particular group of residues do not significantly affect the antigen binding. As used herein, a CDR may refer to a CDR defined by any method known in the art, including combinations of methods. In the methods used herein, CDRs defined according to any of those methods may be used. For any given embodiment comprising more than one CDR, the CDRs may be defined according to any of Kabat, Chothia, extended, AbM, IMGT, contact, and/or conformational definitions.

A "monoclonal antibody" or "mAb" refers to an antibody obtained from a population of substantially homogeneous antibodies, that is to say, the antibodies comprised in the population are identical except for naturally occurring mutations that may be present in minor amounts. Monoclonal antibodies are highly specific, and target a single antigen site. Furthermore, unlike polyclonal antibodies, each monoclonal antibody targets a single determinant on the antigen. The modifier "monoclonal" indicates the characteristic of the antibody as obtained from a population of substantially homogeneous antibodies, and is not to be construed as requiring production of the antibody by any particular method. For example, monoclonal antibodies used according to the present disclosure can be prepared by the hybridoma methods firstly described in Kohler and Milstein, 1975, Nature 256: 495, or can be prepared by the recombinant DNA method described in U.S. Patent No. 4,816,567. For example, monoclonal antibodies can also be isolated from the generated phage library using the technique described in McCafferty et al., 1990, Nature 348: 552-554.

A "human antibody" includes antibodies having variable and constant regions derived from human germline immunoglobulin sequences. The human antibody of the present disclosure may include amino acid residues not encoded by human germline immunoglobulin sequences (e.g., mutations introduced by random or site-specific mutagenesis *in vitro* or by somatic mutations *in vivo).* However, a "human antibody" does not include antibodies in which CDR sequences derived from the germline of another mammalian species (such as mice) have been grafted into a human framework sequence (i.e., "humanized antibodies").

A "human antibody" or "recombinant human antibody" includes human antibodies prepared, expressed, created, or isolated by recombinant methods, involving techniques and methods well known in the art, such as:
(1) antibodies isolated from transgenic human immunoglobulin genes, transchromosomal animals (e.g., mice), or hybridomas prepared therefrom;
(2) antibodies isolated from host cells that have been transformed to express the antibodies, such as transfectomas;
(3) antibodies isolated from a recombinant combinatorial human antibody library; and
(4) antibodies prepared, expressed, created or isolated by a method such as splicing human immunoglobulin gene sequences to other DNA sequences.

Such recombinant human antibodies comprise variable and constant regions that utilize specific human germline immunoglobulin sequences encoded by germline genes, and also include subsequent rearrangements and mutations which occur, for example, during antibody maturation.

An "antigen (Ag)" refers to a molecule used to immunize an immunologically active vertebrate to produce an antibody (Ab) that recognizes Ag; or a molecule or mimic used for screening in an expression library, e.g., especially a phage, yeast, or ribosome display library. Ag is defined more broadly herein, and is generally intended to include target molecules recognized by Abs. Therefore, Ag includes molecules, or portions or mimics thereof, used in immunization processes for producing Abs or in library screening for selecting Abs. Therefore, for the antibody of the present disclosure that binds to IL-2, full-length IL-2 from mammalian species (e.g., human, monkey, mouse, and rat IL-2), including monomers and polymers thereof (e.g., dimers, trimers, etc.), as well as truncated variants and other variants of IL-2, are all referred to as antigens.

An "epitope" refers to a site on an antigen to which an immunoglobulin (or antibody) specifically binds. An epitope may be formed from contiguous amino acids, or non-contiguous amino acids juxtaposed by tertiary folding of the protein. An epitope formed from contiguous amino acids are generally retained after exposure to a denaturing solvent, while an epitope formed by tertiary folding are generally lost after a denaturing solvent treatment. An epitope generally comprise, for example, 3 to 15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described herein, such as X-ray crystallography and two-dimensional nuclear magnetic resonance. An "antigen-binding fragment" include a single-chain antibody (i.e., full-length heavy and light chains); Fab, a modified Fab, Fab', a modified Fab', F(ab')₂, Fv, Fab-Fv, Fab-dsFv, a single domain antibody (e.g., VH or VL or VHH), scFv, a bivalent or trivalent or tetravalent antibody, Bis-scFv, a diabody, a tribody, a triabody, a tetrabody and an epitope-binding fragment of any of the above (see, e.g., Holliger and Hudson, 2005, Nature Biotech. 23 (9): 1126-1136; Adair and Lawson, 2005, Drug Design Reviews-Online 2 (3), 209-217). Methods for producing and preparing such antibody fragments are well known in the art (see, e.g., Verma et al., 1998, Journal of Immunological Methods, 216, 165-181). Fab-Fv was first disclosed in WO2009/040562, and its disulfide-stabilized form Fab-dsFv was first disclosed in WO2010/035012. The antigen-binding fragment of the present disclosure also include Fab and Fab' fragments described in WO2005/003169, WO2005/003170 and WO2005/003171. Multivalent antibodies may comprise multiple specificities (e.g., bispecificites) or may be monospecific (see, e.g., WO92/22583 and WO05/113605), and an example of the latter is Tri-Fab (or TFM) described in WO92/22583.

"Binding to IL-2" refers to the ability to interact with IL-2 or an epitope thereof, wherein the IL-2 or the epitope thereof may be derived from human.

An "antigen-binding site" refers to a continuous or discontinuous three-dimensional spatial site on an antigen that is recognized by an antibody or an antigen-binding fragment of the present disclosure.

A "regulatory T cell" or "Treg" refers to a specialized CD4⁺ T cell type that can inhibit the response of other T cells. Tregs are characterized by expression of the α subunit of IL-2 receptor (CD25) and the transcription factor forkhead box P3 (FOXP3), and play a key role in the induction and maintenance of peripheral self-tolerance to antigens, including those expressed by tumors. Treg requires IL-2 for its function and development as well as for the induction of its inhibitory characteristics.

"Effector function" refers to those biological activities attributable to the Fc region of an antibody (either the natural sequence Fc region or the amino acid sequence variant Fc region) and which vary with the antibody isotype. Examples of effector functions of an antibody include: C1q binding and complement-dependent cytotoxicity (CDC); Fc receptor binding; antibody-dependent cell-mediated cytotoxicity (ADCC); phagocytosis; down-regulation of cell surface receptors (e.g., B cell receptors); and B cell activation. "ADCC" refers to the direct killing of antibody-coated target cells by Fc receptor-expressing cells through recognition of the Fc segment of the antibody. The ADCC effector function of the antibody may be reduced or eliminated by modification of the Fc segment of the IgG. The modification refers to mutations in the heavy chain constant region of the antibody, such as mutations selected from the group consisting of N297A, L234A, L235A and P329G of IgG1, IgG2/4 chimera, and F234A/L235A of IgG4. "CDC" refers to the activation of the cytotoxic form of the complement cascade by binding the complement component C1q to the antibody Fc. Methods for detecting ADCC and CDC activity of an antibody are known in the art. For example, CDC can be evaluated by determining the binding activity between an antibody to be tested and an Fc receptor (e.g., C1q).

An "Fc receptor" or "FcR" refers to a receptor that binds to the Fc region of an antibody. A preferred FcR is a human FcR. In addition, a preferred FcR is an FcR (γ receptor) binding to an IgG antibody and includes receptors of FcγRI, FcγRII, and FcγRIII subclasses, including allelic variants and alternatively spliced forms of those receptors. An FcγRII receptor includes FcγRIIA ("activated receptor") and FcγRIIB ("inhibitory receptor"), which have similar amino acid sequences that differ primarily in their cytoplasmic domains. The activated receptor FcγRIIA comprises an immunoreceptor tyrosine-based activation motif (ITAM) in its cytoplasmic domain. The inhibitory receptor FcγRIIB comprises an immunoreceptor tyrosine-based inhibitory motif (ITIM) in its cytoplasmic domain (see review M. Daeron, Annu. Rev. Immunol., 15: 203-234 (1997)). FcRs are reviewed in Ravetch and Kinet, Annu. Rev. Immunol., 9: 457-92 (1991); Capel et al., Immunomethods, 4: 25-34 (1994); and de Haas et al., J. Lab. Clin. Med., 126: 330-41 (1995). The term "FcR" herein encompasses other FcRs, including those to be identified in the future. The term also includes the neonatal receptor FcRn responsible for transferring maternal IgG to the fetus (Guyer et al., J. Immunol., 117: 587 (1976) and Kim et al., J. Immunol. 24: 249 (1994)).

A "polynucleotide" or "nucleic acid" refers to a nucleotide strand of any length, including DNA and RNA. The nucleotides may be deoxyribonucleotides, ribonucleotides, modified nucleotides or bases, and/or analogs thereof, or any substrate that can be incorporated into a strand by a DNA or RNA polymerase.

"Specific binding" or "selective binding" refers to binding of an antibody to an epitope on a predetermined antigen. Typically, an antibody binds to a predetermined antigen or epitope thereof with an equilibrium dissociation constant (K_{D}) of about less than 10⁻⁷ M or even less and with an avidity that is at least twice as high as its avidity for binding to a non-specific antigen other than the predetermined antigen (or epitope thereof) or a closely related antigen (e.g., BSA, etc.), when determined by surface plasmon resonance (SPR) techniques in an instrument using recombinant human IL-2 or an epitope thereof as the analyte and an antibody as the ligand. The term "antigen-recognizing antibody" is used interchangeably herein with the term "specifically bound antibody".

"Avidity" is used herein as a measure of the strength of a non-covalent interaction between two molecules (e.g., an antibody or an antigen-binding fragment thereof and an antigen). The term "avidity" is used to describe monovalent interactions (intrinsic activity). The binding avidity between two molecules can be quantified by determining the dissociation constant (K_{D}). K_{D} can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods and can be calculated by methods such as those described by Caceci et al (1984, Byte 9: 340-362) even for complex mixtures. For example, K_{D} can be determined by using a dual filtration nitrocellulose filter binding assay such as that disclosed by Wong & Lohman (1993, Proc. Natl. Acad. Sci. USA 90: 5428-5432). Other standard assays for evaluating the binding ability of an antibody to a target antigen are known in the art and include, for example, ELISA, western blot, RIA and flow cytometry, as well as other assays exemplified elsewhere herein. The binding kinetics and binding avidity of the antibody can also be evaluated by standard assays known in the art, such as surface plasmon resonance (SPR), for example by using the Biacore^{™} system or KinExA. The binding avidities associated with different molecular interactions, e.g., the binding avidities of different antibodies to a given antigen, can be compared by comparing the K_{D} values of antibodies/antigen complexes. Similarly, the specificity of an interaction can be evaluated by determining and comparing the K_{D} value for the interaction of interest (e.g., a specific interaction between an antibody and an antigen) to the K_{D} value for the non-interaction of interest (e.g., a control antibody known not to bind to IL-2). In some embodiments, an IL-2 antibody or an antigen-binding fragment of the present disclosure is capable of binding to its target with an avidity at least 2-fold, 10-fold, 50-fold, 100-fold, 200-fold, 500-fold, 1,000-fold, or 10,000-fold greater than its avidity for binding to another non-IL-2 molecule, without limitation herein.

A "complex" of an anti-IL-2 antibody or an antigen-binding fragment thereof and IL-2 refers to a complex comprising at least one anti-IL-2 antibody or antigen-binding fragment thereof of the present disclosure, which specifically binds to IL-2 or cells expressing IL-2. The complex comprises an anti-IL-2 antibody or an antigen-binding fragment thereof and an IL-2 molecule that are bound by covalent force, non-covalent force, or any other force. In some embodiments, the anti-IL-2 antibody or the antigen-binding fragment thereof forms a fusion protein with IL-2. In other embodiments, the anti-IL-2 antibody or the antigen-binding fragment thereof binds to IL-2 by non-covalent force. In some embodiments, the anti-IL-2 antibody or the antigen-binding fragment thereof and the IL-2 may still be maintained as a complex even after administration of the complex. It should be understood that, among other variables, the anti-IL-2 antibody or the antigen-binding fragment thereof and the IL-2 will form complexes that differ in binding ability based on the different K_{D} values of their binding interactions.

"Inhibition" and "blocking" are used interchangeably and encompass both partial and complete inhibition/blocking. Inhibition and blocking are also intended to include any measurable reduction in binding avidity and cell (e.g., T cell) proliferation-promoting activity of IL-2 in contact with an anti-IL-2 antibody, as compared to IL-2 not in contact with an anti-IL-2 antibody.

An "antibody that competes for binding with" a reference antibody refers to an antibody that blocks binding of the reference antibody to an antigen by 50% or more, or an antibody whose binding to an antigen is blocked by 50% or more by the reference antibody, in a competition assay. The antigen-binding activity of the anti-IL-2 antibody of the present disclosure can be tested by known methods such as ELISA and western blot. For example, antibodies that compete for binding to IL-2 can be identified by a competition assay. In certain embodiments, the competitive antibody binds to the same epitope (e.g., a linear or conformational epitope) as the antigen-binding molecule or the anti-IL-2 antibody. For example, the method described in International Patent Publication No. WO03/48731. Therefore, an antibody and an antigen-binding fragment thereof that competes with the antibody molecule of the present disclosure for binding to the same epitope on IL-2 can be obtained using conventional techniques known to those skilled in the art.

"Giving", "administering" and "treating", when applied to animals, humans, experimental subjects, cells, tissues, organs or biological fluid, refer to contact of an exogenous drug, a therapeutic agent, a diagnostic agent or a composition with the animals, humans, subjects, cells, tissues, organs or biological fluid. "Giving", "administering," and "treating" can refer to, for example, therapeutic, pharmacokinetic, diagnostic, research, and experimental methods. The treatment of the cells comprises contacting the reagent with the cells and contacting the reagent with fluid, wherein the fluid are in contact with the cells. "Giving", "administering" and "treating" refers to treating, e.g., a cell, by a reagent, diagnosis, a binding composition, or by another cell *in vitro* and *ex vivo.* "Treating", when applied to human, veterinary or research subjects, refers to therapeutic treatment, preventive or prophylactic measures, and research and diagnostic applications.

"Treating" or "treatment" refers to administering a therapeutic agent, such as a composition comprising any of the IL-2 antibodies or the antigen-binding fragments thereof of the present disclosure or complexes thereof with IL-2, either internally or externally to a subject who has had, is suspected of having, or is predisposed to having one or more immune-related diseases or symptoms thereof on which the therapeutic agent is known to have a therapeutic effect. Typically, the therapeutic agent is administered in an amount effective to alleviate one or more symptoms of the disease in the subject or population being treated, whether by inducing regression of such symptoms or inhibiting the development of such symptoms to any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of the disease has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating symptoms of a disease of interest in a certain subject, they shall alleviate the symptoms of the disease of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

An "effective amount" comprises an amount sufficient to ameliorate or prevent a symptom or condition of a medical condition. An effective amount also refers to an amount sufficient to allow or facilitate diagnosis. The effective amount for a particular subject or veterinary subject may vary depending on the factors such as the condition to be treated, the general health of the subject, the method and route and dosage of administration, and the severity of side effects. An effective amount can be the maximum dose or dosage regimen to avoid significant side effects or toxic effects.

"Homology" or "identity" refers to sequence similarity between two polynucleotide sequences or between two polypeptides. When positions in both compared sequences are occupied by the same base or amino acid monomer subunit, e.g., if each position of two DNA molecules is occupied by adenine, the molecules are homologous at that position. The homology percentage between two sequences is a function of the number of matched or homologous positions shared by the two sequences divided by the number of positions compared ×100%. For example, if 6 out of 10 positions are matched or homologous when two sequences are optimally aligned, the two sequences are 60% homologous. Generally, the comparison is made when the two sequences are aligned to give the greatest homology percentage.

A "host cell" includes various cells or cell cultures and may be or has been the receptor of a vector for incorporation of the polynucleotide insert. The host cell includes progenies of a single host cell, and the progenies may not necessarily be identical (in morphology or genomic DNA complement) to the original parent cell due to natural, accidental, or intentional mutations. The host cell includes cells transfected and/or transformed *in vivo* with polynucleotides of the present disclosure. "Cell", "cell line" and "cell culture" are used interchangeably, and all such designations include their progenies. It should also be understood that all progenies may not be exactly identical in DNA content due to intentional or unintentional mutations. Mutant progenies with the same function or biological activity as those screened from the original transformed cells are included.

A "vector" refers to a construct capable of delivering and, in some embodiments, expressing one or more genes or sequences of interest in a host cell. Examples of vectors include, but are not limited to, viral vectors, naked DNA or RNA expression vectors, plasmid, cosmid or phage vectors, DNA or RNA expression vectors bound to cationic condensing agents, DNA or RNA expression vectors encapsulated in liposomes, and certain eukaryotic cells such as producer cells.

The term "optional" or "optionally" means that the event or circumstance subsequently described may, but not necessarily, occur, and that the description includes instances where the event or circumstance occurs or does not occur. For example, "optionally comprising 1-3 antibody heavy chain variable regions" means that an antibody heavy chain variable region of a particular sequence may, but not necessarily, be present.

The term "pharmaceutical composition" refers to a mixture containing one or more of the antibodies or the antigen-binding fragments described herein or a physiologically/pharmaceutically acceptable salt or pro-drug thereof, and other chemical components such as physiologically/pharmaceutically acceptable carriers and excipients. The purpose of the pharmaceutical composition is to promote the administration to an organism, which facilitates the absorption of the active ingredient, thereby exerting biological activities.

A "pharmaceutically acceptable carrier" or "pharmaceutically acceptable excipient" includes any material that, when combined with an active ingredient, allows the ingredient to retain biological activity and not react with the immune system of a subject. Examples include, but are not limited to, any standard pharmaceutical carrier, such as a phosphate buffered saline solution, water, an emulsion such as an oil/water emulsion, and various types of wetting agents. In some embodiments, the diluent for aerosol or parenteral administration is phosphate buffered saline (PBS) or normal (0.9%) saline. Compositions containing such carriers are formulated by well-known conventional methods (see, e.g., Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

Herein, when "about" is applied to a value or parameter, it includes (and describes) embodiments involving the value or parameter itself. For example, a description referring to "about X" includes a description of "X". A numerical range includes the numbers defining the range. Although the numerical ranges and parameters setting forth the broad scope of the present disclosure are approximations, the numerical values set forth in the specific examples are reported as precisely as possible. However, any numerical values inherently contain certain errors necessarily resulting from the standard deviation found in their respective tests or measurements. Moreover, all ranges disclosed herein should be understood to encompass any and all subranges subsumed therein. For example, a stated range of "1 to 10" should be considered to include any and all subranges between (and inclusive of) the minimum value of 1 and the maximum value of 10; i.e., all subranges beginning with a minimum value of 1 or more, e.g., 1 to 6.1, and ending with a maximum value of 10 or less, e.g., 5.5 to 10.

### Examples

The present disclosure is further described below with reference to examples, which, however, are not intended to limit the scope of the present disclosure.

Experimental procedures without specific conditions indicated in the examples or test examples are generally conducted according to conventional conditions, or according to conditions recommended by the manufacturer of the starting materials or commercial products, see Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated are commercially available conventional reagents.

### Example 1. Screening of Anti-IL-2 Antibodies

### A) Screening of human natural phage Fab library

The preserved natural library phage suspension was diluted and blocked with BSA, and incubated with magnetic bead Dynabeads (M-280, Invitrogen). The phages were collected after negative screening and incubation. The beads were bound and washed according to the Kingfisher magnetic bead screening system method (Thermo Scientific), and incubated with 5% BSA. Dynabeads were coated and blocked by biotin-labeled IL-2 with a human Fc-tag (Sanyou Biopharmaceutical, GenBank Accession No: P60568-1), incubated with a phage suspension collected after negative screening, and bound and washed according to the Kingfisher magnetic bead screening system method. The phages were eluted with pancreatin. After the elution, the phage solution was mixed well with *E. coli* SS320 cells (Sanyou Biopharmaceutical) in the logarithmic growth phase, and incubated at 37 °C for 30 min. *E. coli* SS320 cells were applied to a 2YT-Car⁺-Tet⁺ plate and cultured overnight in an incubator at 37 °C. The phage input, output and the like were calculated, and the input phages were prepared by scraping plates and screened for 3 rounds.

### B) Primary screening for monoclonal phages expressing anti-IL-2 Fab capable of binding to IL-2 by ELISA

2000 clones from the 3rd round of screening were picked and cultured overnight in a 96-well deep-well plate. The supernatant was collected by centrifugation. Anti-human IgG (STAR161, Bio-rad) was diluted to 2 µg/mL with PBS and added at 30 µL/well to coat an ELISA plate, and the plate was incubated overnight at 4 °C. The plate was washed with PBST (PBS, pH 7.4 + 0.1% Tween 20) 3 times. The plate was blocked with 1% BSA for 1 h at room temperature and washed with PBST 3 times. 30 µL of the supernatant obtained by centrifugation was added, and the plate was incubated at room temperature for 2 h and washed with PBST 3 times. Biotin-labeled IL-2 with a human Fc-tag was diluted to 2 µg/mL with PBS and added to the plate at 30 µL/well. The plate was incubated at room temperature for 1 h and washed with PBST 3 times. 30 µL of secondary antibody NeutrAvidin-HRP diluted in a 1:8000 ratio was added. The plate was incubated at room temperature for 1 h and washed with PBST 9 times. 30 µL of TMB was added for color developing for 5-10 min at room temperature, then 30 µL of 2M HCl or H₂SO₄ was added to terminate the reaction. The plate was read for data by a microplate reader at OD 450. Finally, 364 clones with specific sequences were obtained. C) Primary screening for anti-IL-2 Fab capable of blocking the binding of IL-2 to IL-2Rβ by ELISA

The above 364 clones were inoculated into 50 mL of 2YT-Car⁺-Tet⁺ medium in a 1:1000 ratio, and cultured overnight at 37 °C. The bacterial solution was transferred to a 50 mL centrifuge tube, and centrifuged at room temperature. The supernatant was discarded, and 0.5 mL of protein lysis buffer (10 mM Tris-HCl, pH 9.0, 1 mM EDTA, 5 mM MgCl₂, 25 U/mL Bezonase nuclease (Merck)) pre-cooled to 4 °C was added thereto. The mixture was left to stand on ice and incubated for 1 h, and centrifuged at 4 °C. The supernatant was collected for later use.

IL-2Rβ-Fc (CJ82, Novoprotein) (GenBank Accession No: NP_000869.1) was diluted to 8 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The obtained Fab lysate was incubated with biotin-labeled IL-2 with a human Fc-tag (2.5 µg/mL) at room temperature for 1 h, and added to the plate at 30 µL/well. The plate was incubated at room temperature for 1 h and washed with PBST 3 times. 30 µL of secondary antibody NeutrAvidin-HRP diluted in a 1:8000 ratio was added. The plate was incubated at room temperature for 1 h and washed with PBST 9 times. 30 µL of TMB was added for color developing for 5-10 min at room temperature, then 30 µL of 2M HCl or H₂SO₄ was added to terminate the reaction. The plate was read for data by a microplate reader at OD 450.

6 Fabs with strong capability of blocking the binding of IL-2 to IL-2Rβ were obtained. For 6 fully human antibodies, the sequences of the heavy chain and light chain variable regions are shown in Table 1, and the sequences of the complementarity determining regions (CDRs) according to different numbering systems are shown in Table 2.

**Table 1. Sequences of heavy chain (HCVR) and light chain (LCVR) variable regions of fully human anti-IL-2 antibodies**

| Antibody No. | Amino acid sequences of heavy chain variable region (VH) and light chain variable region (VL) | | Sequence No. |
|---|---|---|---|
| A2-22 | VH | QVQLVQSGAEVKKPGASVKVSCKASGYTFTSYYMHWVRQ APGQGLEWMGIINPSGGSTSYAQKFQGRVTMTRDTSTSTVY MELSSLRSEDTAVYYCAREDWMEYEYYYGMDVWGQGTLV TVSS | SEQ ID No: 1 |
| | VL | EIVLTQSPGTLSLSPGERATLSCRASQSVSNNYLAWYQQKPG QAPRLLIYGASSRATGIPDRFSGSGSGTDFTLTISRLEPEDFAV YSCQQYGSSPLTFGQGTRLEIK | SEQ ID No: 2 |
| A3-21 | VH | EVQLLESGGGLAQPGGSLRLSCAGSGFTFSSYAMSWVRQAP | SEQ ID No: 3 |
| | | GKGLEWVSGITGTGYRTYYADSVKGRFTVSRDNSRNTVYL QMNSLRAEDTAVYYCVRESGHYYGWFDSWGQGTLVTVSS | |
| | VL | QSALTQPPSVSGAPGQRVTISCTGSSSNIGAGYNVHWYQQV PGTVPKLLIDGKGNRPSGVPDRFSGSKSGTSASLAITGLQAE DEADYYCQSYDYMLSGSVVFGRGTKLTVL | SEQ ID No: 4 |
| B2-15 | VH | EVQLLESGAEVKKPGASVKVSCKASGYTFTSYYMHWVRQ APGQGLEWMGIINPSGGSTSYAQKFQGRVTMTRDTSTSTVY MELSSLRSEDTAVYYCAREGNSSGYYNAFDIWGQGTMVTV SS | SEQ ID No: 5 |
| | VL | EIVLTQSPGTLSLSPGERATLSCRASQSVSSSYLAWYQQKPG QAPRLLIYGASNRATGIPDRFSGSGSGADFTLTISRLEPEDFA VYYCQQYGSSPLTFGGGTKVEIK | SEQ ID No: 6 |
| C2-53 | VH | EVQLVESGGGLVQPGRSLILSCAASGFTFDDYAMHWVRQAP GKGLEWVSGISWNSGSIGYADSVKGRFTISRDNAKNSLYLQ MNSLRAEDTALYYCAKDQLRWGTYYYYGMDVWGQGTTV TVSS | SEQ ID No: 7 |
| | VL | DIQMTQSPSTLSASVGDRVTITCRASQSVSIWVAWYQQKPG KAPKLLIYKASSLERGVPTRFSGSGSGTEFTLTISSLQPEDFAT YHCQQYDTGATFGQGTKVEMK | SEQ ID No: 8 |
| D2-60 | VH | QVQLVQSGAEVKKPGASVKVSCKASGYTFTSYGISWVRQA PGQGLEWMGWISAYNGNTNYAQKLQGRVTMTTDTSTSTAY MELRSLRSDDTAVYYCAREYSSWYSGGMDVWGQGTTVTV SS | SEQ ID No: 9 |
| | VL | DIQMTQSPSSLSASVGDRVTITCRASQGISNYLAWYQQKPG KVPKLLIYAASTLQSGVPSRFSGSGSGTDFTLTISSLQPEDVA TYYCQKYDSAPFTFGQGTRLEIK | SEQ ID No: 10 |
| D3-68 | VH | QVQLVQSGPEVKKPGASVKVSCKASGYTFTSYGISWVRRA PGQGLEWMGWISAYNGNTNYAQKLQGRVTMTTDTSTSTAY MELRSLRSDDTAVYYCARDLQSPTYYYGSGVIDYWGQGTL VTVSS | SEQ ID No: 11 |
| | VL | DIQLTQSPSTLSASVGDRVTISCRASRAVSPWLAWYQHRPGQ APKLLIYAASSLQSGVPSRFSGSGSGTDFTLTISSLQPEDFATY YCQQYRSYPFTFGPGTTVDIR | SEQ ID No: 12 |

**Table 2. Sequences of heavy chain and light chain complementarity determining regions (CDRs) of fully human anti-IL-2 antibodies**

| Antibody No. | CDR | Kabat numbering system | Chothia numbering system | IMGT numbering system | AbM numbering system |
|---|---|---|---|---|---|
| A2-22 | HCDR1 | SYYMH (SEQ ID No: 13) | GYTFTSY (SEQ ID No: 49) | GYTFTSYY (SEQ ID No: 85) | GYTFTSYYMH (SEQ ID No: 121) |
| | HCDR2 | IINPSGGSTSYAQ KFQG (SEQ ID No: 14) | NPSGGS (SEQ ID No: 50) | INPSGGST (SEQ ID No: 86) | IINPSGGSTS (SEQ ID No: 122) |
| | HCDR3 | EDWMEYEYYYG MDV (SEQ ID No: 15) | EDWMEYEYYYG MDV (SEQ ID No: 51) | AREDWMEYEYY YGMDV (SEQ ID No: 87) | EDWMEYEYYYG MDV (SEQ ID No: 123) |
| | LCDR1 | RASQSVSNNYLA (SEQ ID No: 16) | RASQSVSNNYLA (SEQ ID No: 52) | QSVSNNY (SEQ ID No: 88) | RASQSVSNNYLA (SEQ ID No: 124) |
| | LCDR2 | GASSRAT (SEQ ID No: 17) | GASSRAT (SEQ ID No: 53) | GA (SEQ ID No: 89) | GASSRAT (SEQ ID No: 125) |
| | LCDR3 | QQYGSSPLT (SEQ ID No: 18) | QQYGSSPLT (SEQ ID No: 54) | QQYGSSPLT (SEQ ID No: 90) | QQYGSSPLT (SEQ ID No: 126) |
| A3-21 | HCDR1 | SYAMS (SEQ ID No: 19) | GFTFSSY (SEQ ID No: 55) | GFTFSSYA (SEQ ID No: 91) | GFTFSSYAMS (SEQ ID No: 127) |
| | HCDR2 | GITGTGYRTYYA DSVKG (SEQ ID No: 20) | TGTGYR (SEQ ID No: 56) | ITGTGYRT (SEQ ID No: 92) | GITGTGYRTY (SEQ ID No: 128) |
| | HCDR3 | ESGHYYGWFDS (SEQ ID No: 21) | ESGHYYGWFDS (SEQ ID No: 57) | VRESGHYYGWF DS (SEQ ID No: 93) | ESGHYYGWFDS (SEQ ID No: 129) |
| | LCDR1 | TGSSSNIGAGYN VH (SEQ ID No: 22) | TGSSSNIGAGYN VH (SEQ ID No: 58) | SSNIGAGYN (SEQ ID No: 94) | TGSSSNIGAGYN VH (SEQ ID No: 130) |
| | LCDR2 | GKGNRPS (SEQ ID No: 23 | GKGNRPS (SEQ ID No: 59 | GK (SEQ ID No: 95 | GKGNRPS (SEQ ID No: 131) |
| | LCDR3 | QSYDYMLSGSV V (SEQ ID No: 24) | QSYDYMLSGSV V (SEQ ID No: 60) | QSYDYMLSGSV V (SEQ ID No: 96) | QSYDYMLSGSV V (SEQ ID No: 132) |
| B2-15 | HCDR1 | SYYMH (SEQ ID No: 25) | GYTFTSY (SEQ ID No: 61) | GYTFTSYY (SEQ ID No: 97) | GYTFTSYYMH (SEQ ID No: 133) |
| | HCDR2 | IINPSGGSTSYAQ KFQG (SEQ ID No: 26) | NPSGGS (SEQ ID No: 62) | INPSGGST (SEQ ID No: 98) | IINPSGGSTS (SEQ ID No: 134) |
| | HCDR3 | EGNSSGYYNAFD I (SEQ ID No: 27) | EGNSSGYYNAFD I (SEQ ID No: 63) | AREGNSSGYYN AFDI (SEQ ID No: 99) | EGNSSGYYNAFD I (SEQ ID No: 135) |
| | LCDR1 | RASQSVSSSYLA (SEQ ID No: 28) | RASQSVSSSYLA (SEQ ID No: 64) | QSVSSSY (SEQ ID No: 100) | RASQSVSSSYLA (SEQ ID No: 136) |
| | LCDR2 | GASNRAT (SEQ ID No: 29) | GASNRAT (SEQ ID No: 65) | GA (SEQ ID No: 101) | GASNRAT (SEQ ID No: 137) |
| | LCDR3 | QQYGSSPLT (SEQ ID No: 30) | QQYGSSPLT (SEQ ID No: 66) | QQYGSSPLT (SEQ ID No: 102) | QQYGSSPLT (SEQ ID No: 138) |
| C2-53 | HCDR1 | DYAMH (SEQ ID No: 31) | GFTFDDY (SEQ ID No: 67) | GFTFDDYA (SEQ ID No: 103) | GFTFDDYAMH (SEQ ID No: 139) |
| | HCDR2 | GISWNSGSIGYA DSVKG (SEQ ID No: 32) | SWNSGS (SEQ ID No: 68) | ISWNSGSI (SEQ ID No: 104) | GISWNSGSIG (SEQ ID No: 140) |
| | HCDR3 | DQLRWGTYYYY GMDV (SEQ ID No: 33) | DQLRWGTYYYY GMDV (SEQ ID No: 69) | AKDQLRWGTYY YYGMDV (SEQ ID No: 105) | DQLRWGTYYYY GMDV (SEQ ID No: 141) |
| | LCDR1 | RASQSVSIWVA (SEQ ID No: 34) | RASQSVSIWVA (SEQ ID No: 70) | QSVSIW (SEQ ID No: 106) | RASQSVSIWVA (SEQ ID No: 142) |
| | LCDR2 | KASSLER (SEQ ID No: 35) | KASSLER (SEQ ID No: 71) | KA (SEQ ID No: 107) | KASSLER (SEQ ID No: 143) |
| | LCDR3 | QQYDTGAT (SEQ ID No: 36) | QQYDTGAT (SEQ ID No: 72) | QQYDTGAT (SEQ ID No: 108) | QQYDTGAT (SEQ ID No: 144) |
| D2-60 | HCDR1 | SYGIS (SEQ ID No: 37) | GYTFTSY (SEQ ID No: 73) | GYTFTSYG (SEQ ID No: 109) | GYTFTSYGIS (SEQ ID No: 145) |
| | HCDR2 | WISAYNGNTNYA QKLQG (SEQ ID No: 38) | SAYNGN (SEQ ID No: 74) | ISAYNGNT (SEQ ID No: 110) | WISAYNGNTN (SEQ ID No: 146) |
| | HCDR3 | EYSSWYSGGMD V (SEQ ID No: 39) | EYSSWYSGGMD V (SEQ ID No: 75) | AREYSSWYSGG MDV (SEQ ID No: 111) | EYSSWYSGGMD V (SEQ ID No: 147) |
| | LCDR1 | RASQGISNYLA (SEQ ID No: 40) | RASQGISNYLA (SEQ ID No: 76) | QGISNY (SEQ ID No: 112) | RASQGISNYLA (SEQ ID No: 148) |
| | LCDR2 | AASTLQS (SEQ ID No: 41) | AASTLQS (SEQ ID No: 77) | AA (SEQ ID No: 113) | AASTLQS (SEQ ID No: 149) |
| | LCDR3 | QKYDSAPFT (SEQ ID No: 42) | QKYDSAPFT (SEQ ID No: 78) | QKYDSAPFT (SEQ ID No: 114) | QKYDSAPFT (SEQ ID No: 150) |
| D3-68 | HCDR1 | SYGIS (SEQ ID No: 43) | GYTFTSY (SEQ ID No: 79) | GYTFTSYG (SEQ ID No: 115) | GYTFTSYGIS (SEQ ID No: 151) |
| | HCDR2 | WISAYNGNTNYA QKLQG (SEQ ID No: 44) | SAYNGN (SEQ ID No: 80) | ISAYNGNT (SEQ ID No: 116) | WISAYNGNTN (SEQ ID No: 152) |
| | HCDR3 | DLQSPTYYYGSG VIDY (SEQ ID No: 45) | DLQSPTYYYGSG VIDY (SEQ ID No: 81) | ARDLQSPTYYYG SGVIDY (SEQ ID No: 117) | DLQSPTYYYGSG VIDY (SEQ ID No: 153) |
| | LCDR1 | RASRAVSPWLA (SEQ ID No: 46) | RASRAVSPWLA (SEQ ID No: 82) | RAVSPW (SEQ ID No: 118) | RASRAVSPWLA (SEQ ID No: 153) |
| | LCDR2 | AASSLQS (SEQ ID No: 47) | AASSLQS (SEQ ID No: 83) | AA (SEQ ID No: 119) | AASSLQS (SEQ ID No: 155) |
| | LCDR3 | QQYRSYPFT (SEQ ID No: 48) | QQYRSYPFT (SEQ ID No: 84) | QQYRSYPFT (SEQ ID No: 120) | QQYRSYPFT (SEQ ID No: 156) |

### Example 2. Preparation of Full-Length Fully Human Anti-IL-2 Antibodies

The heavy chain and light chain variable regions of the 6 antibodies of Example 1 were linked to the heavy chain constant region of human IgG1 and the kappa or lamda chain constant region, respectively, to construct full-length fully human anti-IL-2 antibodies. The heavy chain constant region carries L234A and L235A mutations (LALA mutation) to remove possible ADCC function of the antibody. The full-length sequences of the 6 antibodies are shown in Table 3.

**Table 3. Full-length heavy and light chain sequences of fully human anti-IL-2 antibodies**

| Antibody No. | Full-length heavy chain (HC) and light chain (LC) amino acid sequences | | Sequence No. |
|---|---|---|---|
| A2-22 | HC | QVQLVQSGAEVKKPGASVKVSCKASGYTFTSYYMH WVRQAPGQGLEWMGIINPSGGSTSYAQKFQGRVTMT RDTSTSTVYMELSSLRSEDTAVYYCAREDWMEYEYY YGMDVWGQGTLVTVSSASTKGPSVFPLAPSSKSTSG GTAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSRDE LTKNQVSLTCLVKGFYPSDIAVEWESNGQPENNYKTT PPVLDSDGSFFLYSKLTVDKSRWQQGNVFSCSVMHE ALHNHYTQKSLSLSPGK | SEQ ID No: 157 |
| | LC | EIVLTQSPGTLSLSPGERATLSCRASQSVSNNYLAWYQ QKPGQAPRLLIYGASSRATGIPDRFSGSGSGTDFTLTIS RLEPEDFAVYSCQQYGSSPLTFGQGTRLEIKRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC | SEQ ID No: 158 |
| A3-21 | HC | EVQLLESGGGLAQPGGSLRLSCAGSGFTFSSYAMSW VRQAPGKGLEWVSGITGTGYRTYYADSVKGRFTVSR DNSRNTVYLQMNSLRAEDTAVYYCVRESGHYYGWF DSWGQGTLVTVSSASTKGPSVFPLAPSSKSTSGGTAA LGCLVKDYFPEPVTVSWNSGALTSGVHTFPAVLQSSG LYSLSSVVTVPSSSLGTQTYICNVNHKPSNTKVDKKV | SEQ ID No: 159 |
| | | EPKSCDKTHTCPPCPAPEAAGGPSVFLFPPKPKDTLMI SRTPEVTCVVVDVSHEDPEVKFNWYVDGVEVHNAK TKPREEQYNSTYRVVSVLTVLHQDWLNGKEYKCKVS NKALPAPIEKTISKAKGQPREPQVYTLPPSRDELTKNQ VSLTCLVKGFYPSDIAVEWESNGQPENNYKTTPPVLD SDGSFFLYSKLTVDKSRWQQGNVFSCSVMHEALHNH YTQKSLSLSPGK | |
| | LC | QSALTQPPSVSGAPGQRVTISCTGSSSNIGAGYNVHW YQQVPGTVPKLLIDGKGNRPSGVPDRFSGSKSGTSAS LAITGLQAEDEADYYCQSYDYMLSGSVVFGRGTKLT VLGQPKANPTVTLFPPSSEELQANKATLVCLISDFYPG AVTVAWKADGSPVKAGVETTKPSKQSNNKYAASSYL SLTPEQWKSHRSYSCQVTHEGSTVEKTVAPTECS | SEQ ID No: 160 |
| B2-15 | HC | EVQLLESGAEVKKPGASVKVSCKASGYTFTSYYMH WVRQAPGQGLEWMGIINPSGGSTSYAQKFQGRVTMT RDTSTSTVYMELSSLRSEDTAVYYCAREGNSSGYYN AFDIWGQGTMVTVSSASTKGPSVFPLAPSSKSTSGGT AALGCLVKDYFPEPVTVSWNSGALTSGVHTFPAVLQS SGLYSLSSVVTVPSSSLGTQTYICNVNHKPSNTKVDK KVEPKSCDKTHTCPPCPAPEAAGGPSVFLFPPKPKDT LMISRTPEVTCVVVDVSHEDPEVKFNWYVDGVEVHN AKTKPREEQYNSTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGK | SEQ ID No: 161 |
| | LC | EIVLTQSPGTLSLSPGERATLSCRASQSVSSSYLAWYQ QKPGQAPRLLIYGASNRATGIPDRFSGSGSGADFTLTI SRLEPEDFAVYYCQQYGSSPLTFGGGTKVEIKRTVAAP SVFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGEC | SEQ ID No: 162 |
| C2-53 | HC | EVQLVESGGGLVQPGRSLILSCAASGFTFDDYAMHW VRQAPGKGLEWVSGISWNSGSIGYADSVKGRFTISRD NAKNSLYLQMNSLRAEDTALYYCAKDQLRWGTYYY YGMDVWGQGTTVTVSSASTKGPSVFPLAPSSKSTSG GTAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSRDE LTKNQVSLTCLVKGFYPSDIAVEWESNGQPENNYKTT PPVLDSDGSFFLYSKLTVDKSRWQQGNVFSCSVMHE ALHNHYTQKSLSLSPGK | SEQ ID No: 163 |
| | LC | DIQMTQSPSTLSASVGDRVTITCRASQSVSIWVAWYQ QKPGKAPKLLIYKASSLERGVPTRFSGSGSGTEFTLTI SSLQPEDFATYHCQQYDTGATFGQGTKVEMKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC | SEQ ID No: 164 |
| D2-60 | HC | QVQLVQSGAEVKKPGASVKVSCKASGYTFTSYGISW VRQAPGQGLEWMGWISAYNGNTNYAQKLQGRVTM TTDTSTSTAYMELRSLRSDDTAVYYCAREYSSWYSGG MDVWGQGTTVTVSSASTKGPSVFPLAPSSKSTSGGT AALGCLVKDYFPEPVTVSWNSGALTSGVHTFPAVLQS SGLYSLSSVVTVPSSSLGTQTYICNVNHKPSNTKVDK KVEPKSCDKTHTCPPCPAPEAAGGPSVFLFPPKPKDT LMISRTPEVTCVVVDVSHEDPEVKFNWYVDGVEVHN | SEQ ID No: 165 |
| | | AKTKPREEQYNSTYRVVSVLTVLHQDWLNGKEYKC KVSNKALPAPIEKTISKAKGQPREPQVYTLPPSRDELT KNQVSLTCLVKGFYPSDIAVEWESNGQPENNYKTTPP VLDSDGSFFLYSKLTVDKSRWQQGNVFSCSVMHEAL HNHYTQKSLSLSPGK | |
| | LC | DIQMTQSPSSLSASVGDRVTITCRASQGISNYLAWYQ QKPGKVPKLLIYAASTLQSGVPSRFSGSGSGTDFTLTI SSLQPEDVATYYCQKYDSAPFTFGQGTRLEIKRTVAA PSVFIFPPSDEQLKSGTASVVCLLNNFYPREAKVQWK VDNALQSGNSQESVTEQDSKDSTYSLSSTLTLSKADY EKHKVYACEVTHQGLSSPVTKSFNRGEC | SEQ ID No: 166 |
| D3-68 | HC | QVQLVQSGPEVKKPGASVKVSCKASGYTFTSYGISW VRRAPGQGLEWMGWISAYNGNTNYAQKLQGRVTMT TDTSTSTAYMELRSLRSDDTAVYYCARDLQSPTYYYG SGVIDYWGQGTLVTVSSASTKGPSVFPLAPSSKSTSG GTAALGCLVKDYFPEPVTVSWNSGALTSGVHTFPAVL QSSGLYSLSSVVTVPSSSLGTQTYICNVNHKPSNTKV DKKVEPKSCDKTHTCPPCPAPEAAGGPSVFLFPPKPK DTLMISRTPEVTCVVVDVSHEDPEVKFNWYVDGVEV HNAKTKPREEQYNSTYRVVSVLTVLHQDWLNGKEY KCKVSNKALPAPIEKTISKAKGQPREPQVYTLPPSRDE LTKNQVSLTCLVKGFYPSDIAVEWESNGQPENNYKTT PPVLDSDGSFFLYSKLTVDKSRWQQGNVFSCSVMHE ALHNHYTQKSLSLSPGK | SEQ ID No: 167 |
| | LC | DIQLTQSPSTLSASVGDRVTISCRASRAVSPWLAWYQ HRPGQAPKLLIYAASSLQSGVPSRFSGSGSGTDFTLTIS SLQPEDFATYYCQQYRSYPFTFGPGTTVDIRRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC | SEQ ID No: 168 |

| | | | |
|---|---|---|---|
| (Note: the underlined portion of HC is the heavy chain constant region of human IgG1, and the underlined portion of LC is the kappa chain constant region) | | | |

The above sequences were synthesized, digested with BamHI and XhoI, and inserted into a pcDNA3.1 expression vector (Life Technologies Cat. No. V790-20) through the BamHI/XhoI enzymatic digestion site. The expression vector and transfection reagent PEI (Polysciences, Inc. Cat. No. 23966) were transfected into HEK293 cells (Life Technologies Cat. No. 11625019) in a 1:2 ratio, and the cells were placed in a CO₂ incubator and incubated for 4-5 days. The expressed antibodies were isolated by centrifugation, purified by a conventional method, and identified to obtain the full-length fully human antibodies of the present disclosure.

### Example 3. ELISA Assay on Anti-IL-2 Antibodies Capable of Blocking or Reducing the Binding of IL-2 to IL-2Rα and Blocking the Binding of IL-2 to IL-2Rβ

### A) ELISA assay on the blocking or reducing of the binding of IL-2 to IL-2Rα by antibodies

IL-2Rα-Fc (CJ78, Novoprotein) (Accession # NP_000408) was diluted to 0.5 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The anti-IL-2 antibody was diluted in a gradient to 10 µg/mL, 5 µg/mL, 2.5 µg/mL, 1.25 µg/mL, 0.63 µg/mL, 0.31 µg/mL, 0.16 µg/mL, and 0.08 µg/mL with PBS, pH 7.4, incubated with biotin-labeled IL-2 with a human Fc-tag (final concentration: 1 µg/mL) at room temperature for 15 min, added to the plate at 30 µL/well. The plate was incubated at room temperature for 1 h, and washed with PBST 3 times. 30 µL of secondary antibody NeutrAvidin-HRP diluted in a 1:6000 ratio was added, and the plate was incubated at room temperature for 1 h and washed with PBST 9 times. 30 µL of TMB was added for color developing for 5-10 min at room temperature, then 30 µL of 2 M HCl or H₂SO₄ was added to terminate the reaction. The plate was read for data by a microplate reader at OD 450.

The results are shown in FIG. 1A. The results showed that C2-53, D3-68 and A3-21 could completely block the binding of IL-2 to IL-2Rα, and the remaining clones might have only a weak ability to block (i.e., reduce) the binding of IL-2 to IL-2Rα at a high concentration of 10 µg/mL. IC₅₀ values for the antibodies are shown in Table 4.

**Table 4. IC₅₀ values for anti-IL-2 antibodies blocking the binding of IL-2 to IL-2Rα**

| Antibody No. | IC₅₀ (µg/mL) |
|---|---|
| A2-22 | N.A. |
| A3-21 | 1.456 |
| B2-15 | N.A. |
| C2-53 | 0.6591 |
| D2-60 | N.A. |
| D3-68 | 1.196 |

| | |
|---|---|
| (Note: N.A.: due to the weak blocking effect, no meaningful IC₅₀ values could be obtained by fitting the curve.) | |

### B) ELISA assay on the blocking of the binding of IL-2 to IL-2Rβ by antibodies

IL-2Rβ-Fc (CJ82, Novoprotein) was diluted to 8 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The anti-IL-2 antibody was diluted in a gradient to 10 µg/mL, 5 µg/mL, 2.5 µg/mL, 1.25 µg/mL, 0.63 µg/mL, 0.31 µg/mL, 0.16 µg/mL, and 0.08 µg/mL with PBS, pH 7.4, incubated with biotin-labeled IL-2 with a human Fc-tag (final concentration: 5 µg/mL) at room temperature for 15 min, added to the plate at 30 µL/well. The plate was incubated at room temperature for 1 h, and washed with PBST 3 times. 30 µL of secondary antibody NeutrAvidin-HRP diluted in a 1:6000 ratio was added, and the plate was incubated at room temperature for 1 h and washed with PBST 9 times. 30 µL of TMB was added for color developing for 5-10 min at room temperature, then 30 µL of 2 M HCl or H₂SO₄ was added to terminate the reaction. The plate was read for data by a microplate reader at OD 450.

The results are shown in FIG. 1B. The results showed that all antibodies could completely block the binding of IL-2 to IL-2Rβ. IC₅₀ values for the antibodies are shown in Table 5.

**Table 5. IC₅₀ values for anti-IL-2 antibodies blocking the binding of IL-2 to IL-2Rβ**

| Antibody No. | IC₅₀ (µg/mL) |
|---|---|
| A2-22 | 2.728 |
| A3-21 | 3.475 |
| B2-15 | 3.045 |
| C2-53 | 1.441 |
| D2-60 | 8.272 |
| D3-68 | 2.134 |

### Example 4. Octet Assay on Anti-IL-2 Antibodies Blocking or Reducing the Binding of IL-2 to IL-2Rα and Blocking the Binding of IL-2 to IL-2Rβ

### A) Octet assay on the blocking or reducing of the binding of IL-2 to IL-2Rα by antibodies

A Streptavidin biosensor (Fortebio, # 18-5020) was immersed in 200 µL of KB buffer (PBS, pH 7.4, 0.05% tween-20, 0.1% BSA) for 60 s for the wetting treatment. Then, biotinylated IL-2Rα-Fc (CJ78, Novoprotein) was diluted to 10 µg/mL with the KB buffer, and the sensor was immersed in 200 µL of the solution for 150 s. The sensor was immersed in the KB buffer for 60 s to elute excess IL-2Rα. IL-2-His (CX66, Novoprotein) was mixed with an anti-IL-2 antibody and diluted with KB buffer to final concentrations of 100 nM and 500 nM, and incubated at room temperature for 30 min. The sensor was immersed in the mixture for 300 s for association. The stronger the ability of the anti-IL-2 antibody to block or reduce the binding of IL-2 to IL-2Rα is, the lower the reading is. The values at 230 s after the beginning of the binding were read. The results are shown in Table 6.

**Table 6. The blocking or reducing of the binding of IL-2 to IL-2Rα by anti-IL-2 antibodies in Octet assay**

| Antibody No. | Values for blocking IL-2Rα at an Octet level |
|---|---|
| C2-53 | 0.410 |
| A3-21 | 0.428 |
| D3-68 | 0.473 |
| B2-15 | 0.700 |
| D2-60 | 0.754 |
| A2-22 | 0.848 |

### B) Octet assay on the blocking of the binding of IL-2 to IL-2Rβ by antibodies

A Streptavidin biosensor (Fortebio, # 18-5020) was immersed in 200 µL of KB buffer (PBS, pH 7.4, 0.05% tween-20, 0.1% BSA) for 60 s for the wetting treatment. Then, biotinylated IL-2-Fc (Sanyou Biopharmaceutical) was diluted to 10 µg/mL with the KB buffer, and the sensor was immersed in 200 µL of the solution for 300 s. The sensor was immersed in the KB buffer for 60 s to elute excess IL-2. The anti-IL-2 antibody was diluted to a final concentration of 500 nM with the KB buffer, and the sensor was immersed in the antibody solution for association for 300 s. The sensor was immersed in the KB buffer for 60 s to elute excess anti-IL-2 antibody. The IL-2Rβ-Fc (CJ82, Novoprotein) was diluted to a final concentration of 8000 nM with the KB buffer, and the sensor was immersed in the antibody solution for association for 300 s. The stronger the ability of the anti-IL-2 antibody to block the binding of IL-2 to IL-2Rβ is, the lower the reading is. The values at 230 s after the beginning of the binding of IL-2Rβ-Fc were read. The results are shown in Table 7.

**Table 7. The blocking of the binding of IL-2 to IL-2Rβ by anti-IL-2 antibodies in Octet assay**

| Antibody No. | Values for blocking IL-2Rβ at an Octet level |
|---|---|
| A3-21 | 0.003 |
| C2-53 | 0.017 |
| D3-68 | 0.027 |
| A2-22 | 0.042 |
| B2-15 | 0.056 |
| D2-60 | 0.086 |

### Example 5. ELISA Assay on Binding of Anti-IL-2 Antibodies to IL-2

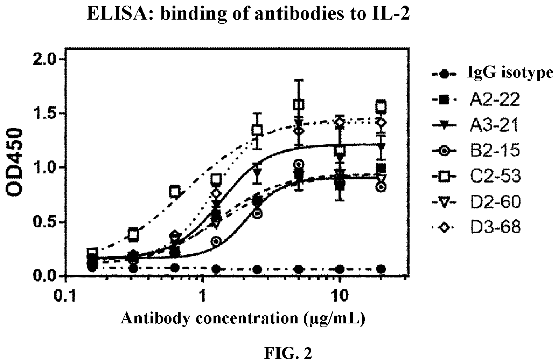

Anti-human IgG (STAR161, Bio-rad) was diluted to 2 µg/mL with PBS and added at 30 µL/well to coat an ELISA plate, and the plate was incubated overnight at 4 °C. The plate was washed with PBST 3 times. The plate was blocked with 1% BSA for 1 h at room temperature and washed with PBST 3 times. The anti-IL-2 antibody was diluted in a gradient to 20 µg/mL, 10 µg/mL, 5 µg/mL, 2.5 µg/mL, 1.25 µg/mL, 0.625 µg/mL, 0.3125 µg/mL, and 0.15625 µg/mL with PBS. 30 µL of the antibody solution was added to an ELISA plate, and the plate was incubated at room temperature for 1 h and washed with PBST 3 times. Biotin-labeled IL-2 with a human Fc-tag (Sanyou Biopharmaceutical) was diluted to 2 µg/mL with PBS and added to the plate at 30 µL/well. The plate was incubated at room temperature for 1 h and washed with PBST 3 times. 30 µL of secondary antibody NeutrAvidin-HRP diluted in a 1:6000 ratio was added. The plate was incubated at room temperature for 1 h and washed with PBST 9 times. 30 µL of TMB was added for color developing for 5-10 min at room temperature, then 30 µL of 2M HCl or H₂SO₄ was added to terminate the reaction. The plate was read for data by a microplate reader at OD 450. The results are shown in FIG. 2 and Table 8. The results showed that all antibodies could bind to IL-2 in this assay, among which C2-53 had a stronger binding ability.

**Table 8. EC₅₀ values for anti-IL-2 antibodies binding to IL-2**

| Antibody No. | EC₅₀ (µg/mL) |
|---|---|
| A2-22 | 1.289 |
| A3-21 | 1.350 |
| B2-15 | 2.117 |
| C2-53 | 0.7301 |
| D2-60 | 1.428 |
| D3-68 | 1.205 |

### Example 6. Biacore Assay on Avidity and Kinetics of Anti-IL-2 Antibodies for IL-2

Antibodies were captured with a Protein A sensor chip (GE, Cat # 29127556) of a Biacore instrument (Biacore T200, GE), in which anti-IL-2 antibodies were diluted to 1 µg/mL with 1× HBS-EP, and flowed at a flow rate of 10 µL/min for 30 s. Then, IL-2 (C013, Novoprotein) solutions at a series of concentration gradients flowed over the chip surface at a flow rate of 30 µL/min for association for 120 s. The solutions flowed at a flow rate of 30 µL/min for dissociation for 300 s. The reaction signals were detected in real time to obtain association and dissociation curves. After the dissociation was completed for each assay cycle, the chip was washed and regenerated with 10 mM Gly-HCl, pH 2.0. The data obtained from the assay were fitted with a 1:1 binding model to obtain the avidity values of the anti-IL-2 antibodies for IL-2. The results are shown in Table 9. The results showed that all antibodies could bind to IL-2 with avidity values spanning from 0.267 nM for C2-53 to 39.8 nM for A2-22.

**Table 9. Avidity of anti-IL-2 antibodies for IL-2**

| Antibody No. | Kon (1/Ms) | Koff (1/s) | KD (M) |
|---|---|---|---|
| C2-53 | 1.64E+07 | 4.37E-03 | 2.67E-10 |
| D3-68 | 1.16E+07 | 5.46E-02 | 4.72E-09 |
| D2-60 | 5.09E+05 | 6.51E-03 | 1.28E-08 |
| B2-15 | 1.46E+06 | 4.48E-02 | 3.06E-08 |
| A2-22 | 1.33E+06 | 5.27E-02 | 3.98E-08 |

### Example 7. Determination of Activity of Non-Covalent Complexes of Anti-IL-2 Antibody and IL-2 for STAT5 Phosphorylation in Human Peripheral Blood PBMC

The ability of the antibodies to regulate IL-2 activity was detected based on the levels of STAT5 phosphorylation in various cell populations (including Tregs, CD4⁺ T cells, and CD8⁺ T cells) in human peripheral blood treated with different concentrations of non-covalent complexes of anti-IL-2 antibodies and IL-2.

Basic medium: RPMI 1640 + 10% fetal bovine serum.

Antibody mixture: CD3 APC-Cy7 (BD 557832), CD4 BB515 (BD 564419), CD8 BB700 (BD 566452), CD25 BV421 (BD 564033), and pSTAT5 AF647 (BD 562076).

Human PBMC STAT5 phosphorylation assay: freshly isolated human PBMC cells were adjusted to a density of 6.5×10⁶ cells/mL with a basic medium, and 80 µL of the mixture were added into a 96-well plate. The anti-IL-2 antibody at different concentrations was premixed with IL-2 at different concentrations for 30 min at room temperature, and 20 µL of the mixture was added to 90 µL of PBMC and stimulated at 37 °C for 20 min. Immediately thereafter, the cells were immobilized with a pre-warmed BD Cytofix buffer (BD, Cat No. 554655) at 37 °C for 15 min and then immobilized on ice for another 15 min. The mixture was centrifuged at 400 g for 7 min at 4 °C. The supernatant was removed, and the plate was washed with 150 µL of PBS once. 150 µL of BD Phosflow Perm Buffer III (BD, Cat No. 558050) pre-cooled at -20 °C was added to perform membrane rupture overnight at -20 °C. The mixture was centrifuged at 500 g for 7 min at 4 °C. The supernatant was removed, and the plate was washed with 150 µL of PBS, pH 7.4 twice. 100 µL of FcR blocking reagent diluted in a 1:200 ratio was added, and the plate was incubated at 4 °C for 20 min. The mixture was centrifuged at 500 g for 7 min at 4 °C. The supernatant was removed, 50 µL of the antibody mixture was added, and the cells were stained at 4 °C for 1 h. The mixture was centrifuged at 500 g for 7 min at 4 °C. The supernatant was removed, and the plate was washed with 200 µL of PBS, pH 7.4 once. The cells were resuspended in 150 µL of PBS at pH 7.4 and detected by flow cytometry (BD FACSCelesta). CD8⁺ T cells were defined as CD3⁺CD4⁻CD8⁺ cells, and Tregs were defined as CD3⁺CD4⁺CD8⁺CD25⁺ cells.

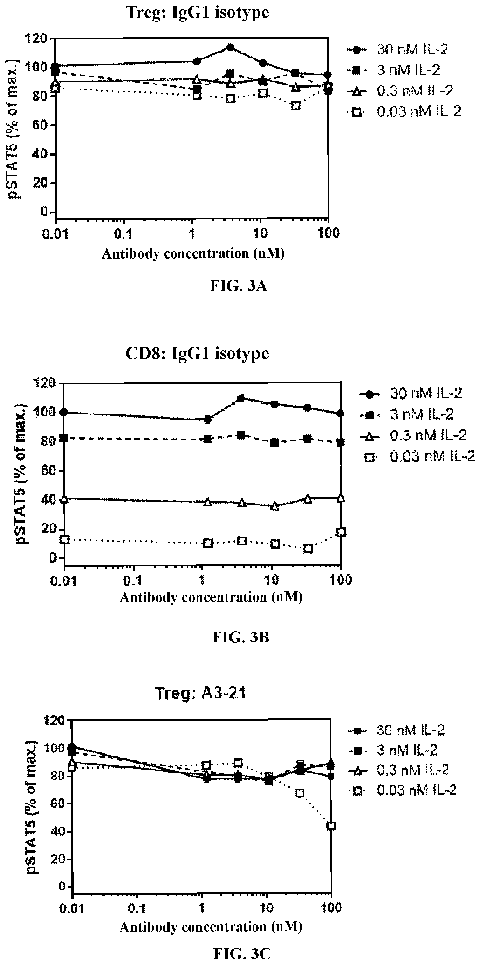

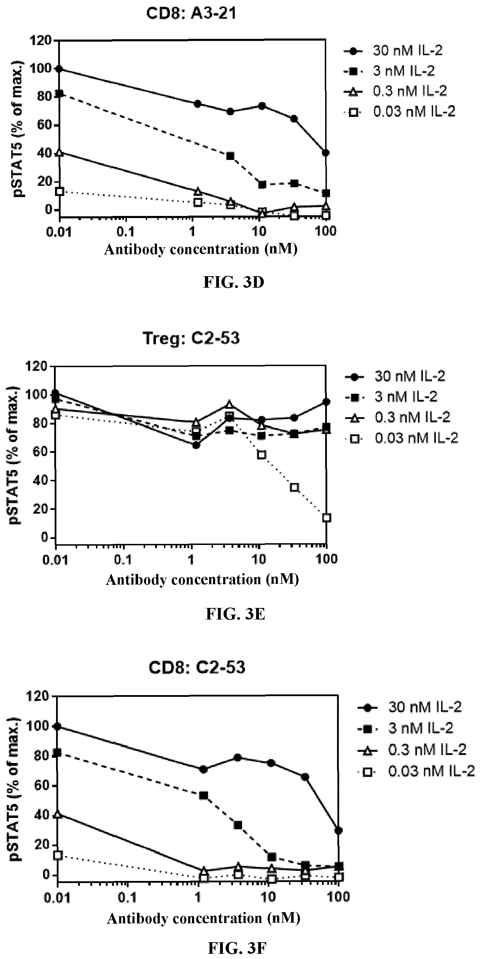

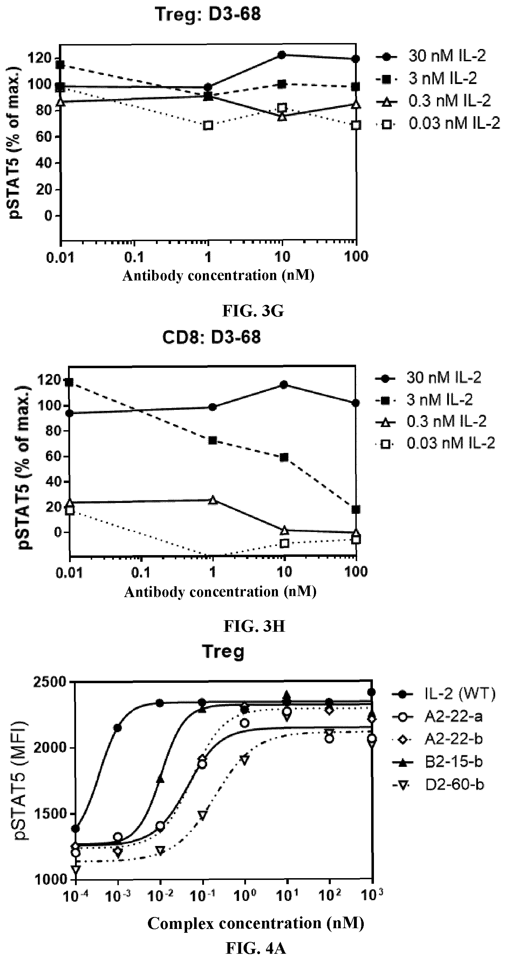

The fluorescence (MFI) values of pSTAT5 were counted for both cell populations, and the percentage of the value at each concentration to the maximum pSTAT5 MFI value in the fully activated state was calculated and fitted using a computer program or four-parameter regression calculation method. The results are shown in FIGs. 3A-3H.

The results showed that the IgG1 isotypes did not have any effect on the activation of pSTAT5 in Tregs and CD8⁺ T cells by IL-2 as antibody concentration increased. In contrast, A3-21 and C2-53 did not substantially affect the activity of IL-2 for Tregs, but significantly reduced the activity of IL-2 for CD8⁺ T cells over a wide concentration range. Only at high concentrations, such as 100 nM (A3-21) and 33.3 nM (C2-53), there was a certain decreased in the activity of IL-2 for Tregs, but under that condition, IL-2 completely had no activity for CD8⁺ T cells. In other words, A3-21 and C2-53 could inhibit or to a greater extent reduce, the activity of IL-2 for immune effector cells such as CD8⁺ T cells while not affecting or to a lesser extent reducing, the activity of IL-2 for Tregs. Similarly, D3-68 may also reduce the activity of IL-2 for CD8⁺ T cells to a greater extent than that for Tregs, although this ability may not be as significant as that of A3-21 and C2-53.

### Example 8. Preparation of Fusion Proteins

Non-covalent complexes of anti-IL-2 antibody and IL-2 may be easily dissociated *in vivo* to produce free IL-2, resulting in toxicity. We obtained 12 fusion proteins of anti-IL-2 antibody and IL-2 by conjugating human IL-2 to the N-terminus of the heavy or light chain of anti-IL-2 antibody via a (G₄S)₅ linker arm (i.e., GGGGSGGGGSGGGGSGGGGSGGGGS).

For antibody numbering, if IL-2 is at the N-terminus of the heavy chain of the antibody, the fusion protein is suffixed a; and if IL-2 is at the N-terminus of the light chain of the antibody, the fusion protein is suffixed b.

For fused IL-2, it carries a T3A mutation (i.e., mutation of the amino acid at position 3 from Thr to Ala) to remove possible glycosylation. The mature human IL-2 protein does not contain amino acid M at position 1, so the numbering starts at amino acid A at position 2. Amino acid sequences of the 12 fusion proteins are as follows:

**Table 10. Full-length sequences of fusion proteins**

| Antibody No. | Full-length heavy chain (HC) and full-length light chain (HL) amino acid sequences | | Sequence No. |
|---|---|---|---|
| A2-22-a | HC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSQVQLVQSGAEVKKPGASVKVSCKASG YTFTSYYMHWVRQAPGQGLEWMGIINPSGGSTSYAQ KFQGRVTMTRDTSTSTVYMELSSLRSEDTAVYYCAR EDWMEYEYYYGMDVWGQGTLVTVSSASTKGPSVFP LAPSSKSTSGGTAALGCLVKDYFPEPVTVSWNSGALT SGVHTFPAVLQSSGLYSLSSVVTVPSSSLGTQTYICNV NHKPSNTKVDKKVEPKSCDKTHTCPPCPAPEAAGGP SVFLFPPKPKDTLMISRTPEVTCVVVDVSHEDPEVKF NWYVDGVEVHNAKTKPREEQYNSTYRVVSVLTVLH QDWLNGKEYKCKVSNKALPAPIEKTISKAKGQPREP QVYTLPPSRDELTKNQVSLTCLVKGFYPSDIAVEWES NGQPENNYKTTPPVLDSDGSFFLYSKLTVDKSRWQQ GNVFSCSVMHEALHNHYTQKSLSLSPGK | SEQ ID No: 169 |
| | LC | SEQ ID No: 158 | SEQ ID No: 158 |
| A2-22-b | HC | SEQ ID No: 157 | SEQ ID No: 157 |
| | LC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEIVLTQSPGTLSLSPGERATLSCRASQSV SNNYLAWYQQKPGQAPRLLIYGASSRATGIPDRFSGS GSGTDFTLTISRLEPEDFAVYSCQQYGSSPLTFGQGTR LEIKRTVAAPSVFIFPPSDEQLKSGTASVVCLLNNFYPR EAKVQWKVDNALQSGNSQESVTEQDSKDSTYSLSST LTLSKADYEKHKVYACEVTHQGLSSPVTKSFNRGEC | SEQ ID No: 170 |
| A3-21-a | HC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEVQLLESGGGLAQPGGSLRLSCAGSGF TFSSYAMSWVRQAPGKGLEWVSGITGTGYRTYYADS VKGRFTVSRDNSRNTVYLQMNSLRAEDTAVYYCVRE SGHYYGWFDSWGQGTLVTVSSASTKGPSVFPLAPSS KSTSGGTAALGCLVKDYFPEPVTVSWNSGALTSGVH TFPAVLQSSGLYSLSSVVTVPSSSLGTQTYICNVNHKP SNTKVDKKVEPKSCDKTHTCPPCPAPEAAGGPSVFLF PPKPKDTLMISRTPEVTCVVVDVSHEDPEVKFNWYV DGVEVHNAKTKPREEQYNSTYRVVSVLTVLHQDWL NGKEYKCKVSNKALPAPIEKTISKAKGQPREPQVYTL PPSRDELTKNQVSLTCLVKGFYPSDIAVEWESNGQPE NNYKTTPPVLDSDGSFFLYSKLTVDKSRWQQGNVFS CSVMHEALHNHYTQKSLSLSPGK | SEQ ID No: 171 |
| | LC | SEQ ID No: 160 | SEQ ID No: 160 |
| A3-21-b | HC | SEQ ID No: 159 | SEQ ID No: 159 |
| | LC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSQSALTQPPSVSGAPGQRVTISCTGSSSNI GAGYNVHWYQQVPGTVPKLLIDGKGNRPSGVPDRFS GSKSGTSASLAITGLQAEDEADYYCQSYDYMLSGSV VFGRGTKLTVLGQPKANPTVTLFPPSSEELQANKATL | SEQ ID No: 172 |
| | | VCLISDFYPGAVTVAWKADGSPVKAGVETTKPSKQS NNKYAASSYLSLTPEQWKSHRSYSCQVTHEGSTVEK TVAPTECS | |
| B2-15-a | HC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEVQLLESGAEVKKPGASVKVSCKASGY TFTSYYMHWVRQAPGQGLEWMGIINPSGGSTSYAQK FQGRVTMTRDTSTSTVYMELSSLRSEDTAVYYCARE GNSSGYYNAFDIWGQGTMVTVSSASTKGPSVFPLAP SSKSTSGGTAALGCLVKDYFPEPVTVSWNSGALTSGV HTFPAVLQSSGLYSLSSVVTVPSSSLGTQTYICNVNHK PSNTKVDKKVEPKSCDKTHTCPPCPAPEAAGGPSVFL FPPKPKDTLMISRTPEVTCVVVDVSHEDPEVKFNWY VDGVEVHNAKTKPREEQYNSTYRVVSVLTVLHQDW LNGKEYKCKVSNKALPAPIEKTISKAKGQPREPQVYT LPPSRDELTKNQVSLTCLVKGFYPSDIAVEWESNGQPE NNYKTTPPVLDSDGSFFLYSKLTVDKSRWQQGNVFS CSVMHEALHNHYTQKSLSLSPGK | SEQ ID No: 173 |
| | LC | SEQ ID No: 162 | SEQ ID No: 162 |
| B2-15-b | HC | SEQ ID No: 161 | SEQ ID No: 161 |
| | LC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEIVLTQSPGTLSLSPGERATLSCRASQSV SSSYLAWYQQKPGQAPRLLIYGASNRATGIPDRFSGS GSGADFTLTISRLEPEDFAVYYCQQYGSSPLTFGGGTK VEIKRTVAAPSVFIFPPSDEQLKSGTASVVCLLNNFYP REAKVQWKVDNALQSGNSQESVTEQDSKDSTYSLSS TLTLSKADYEKHKVYACEVTHQGLSSPVTKSFNRGE C | SEQ ID No: 174 |
| C2-53-a | HC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEVQLVESGGGLVQPGRSLILSCAASGFT FDDYAMHWVRQAPGKGLEWVSGISWNSGSIGYADS VKGRFTISRDNAKNSLYLQMNSLRAEDTALYYCAKD QLRWGTYYYYGMDVWGQGTTVTVSSASTKGPSVFP LAPSSKSTSGGTAALGCLVKDYFPEPVTVSWNSGALT SGVHTFPAVLQSSGLYSLSSVVTVPSSSLGTQTYICNV NHKPSNTKVDKKVEPKSCDKTHTCPPCPAPEAAGGP SVFLFPPKPKDTLMISRTPEVTCVVVDVSHEDPEVKF NWYVDGVEVHNAKTKPREEQYNSTYRVVSVLTVLH QDWLNGKEYKCKVSNKALPAPIEKTISKAKGQPREP QVYTLPPSRDELTKNQVSLTCLVKGFYPSDIAVEWES NGQPENNYKTTPPVLDSDGSFFLYSKLTVDKSRWQQ GNVFSCSVMHEALHNHYTQKSLSLSPGK | SEQ ID No: 175 |
| | LC | SEQ ID No: 164 | SEQ ID No: 164 |
| C2-53-b | HC | SEQ ID No: 163 | SEQ ID No: 163 |
| | LC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSDIQMTQSPSTLSASVGDRVTITCRASQS VSIWVAWYQQKPGKAPKLLIYKASSLERGVPTRFSGS | SEQ ID No: 176 |
| | | GSGTEFTLTISSLQPEDFATYHCQQYDTGATFGQGTKV EMKRTVAAPSVFIFPPSDEQLKSGTASVVCLLNNFYPR EAKVQWKVDNALQSGNSQESVTEQDSKDSTYSLSST LTLSKADYEKHKVYACEVTHQGLSSPVTKSFNRGEC | |
| D2-60-a | HC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSQVQLVQSGAEVKKPGASVKVSCKASG YTFTSYGISWVRQAPGQGLEWMGWISAYNGNTNYA QKLQGRVTMTTDTSTSTAYMELRSLRSDDTAVYYCA REYSSWYSGGMDVWGQGTTVTVSSASTKGPSVFPLA PSSKSTSGGTAALGCLVKDYFPEPVTVSWNSGALTSG VHTFPAVLQSSGLYSLSSVVTVPSSSLGTQTYICNVNH KPSNTKVDKKVEPKSCDKTHTCPPCPAPEAAGGPSVF LFPPKPKDTLMISRTPEVTCVVVDVSHEDPEVKFNWY VDGVEVHNAKTKPREEQYNSTYRVVSVLTVLHQDW LNGKEYKCKVSNKALPAPIEKTISKAKGQPREPQVYT LPPSRDELTKNQVSLTCLVKGFYPSDIAVEWESNGQPE NNYKTTPPVLDSDGSFFLYSKLTVDKSRWQQGNVFS CSVMHEALHNHYTQKSLSLSPGK | SEQ ID No: 177 |
| | LC | SEQ ID No: 166 | SEQ ID No: 166 |
| D2-60-b | HC | SEQ ID No: 165 | SEQ ID No: 165 |
| | LC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSDIQMTQSPSSLSASVGDRVTITCRASQG ISNYLAWYQQKPGKVPKLLIYAASTLQSGVPSRFSGS GSGTDFTLTISSLQPEDVATYYCQKYDSAPFTFGQGTR LEIKRTVAAPSVFIFPPSDEQLKSGTASVVCLLNNFYPR EAKVQWKVDNALQSGNSQESVTEQDSKDSTYSLSST LTLSKADYEKHKVYACEVTHQGLSSPVTKSFNRGEC | SEQ ID No: 178 |
| D3-68-a | HC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSQVQLVQSGPEVKKPGASVKVSCKASG YTFTSYGISWVRRAPGQGLEWMGWISAYNGNTNYA QKLQGRVTMTTDTSTSTAYMELRSLRSDDTAVYYCA RDLQSPTYYYGSGVIDYWGQGTLVTVSSASTKGPSV FPLAPSSKSTSGGTAALGCLVKDYFPEPVTVSWNSGA LTSGVHTFPAVLQSSGLYSLSSVVTVPSSSLGTQTYICN VNHKPSNTKVDKKVEPKSCDKTHTCPPCPAPEAAGG PSVFLFPPKPKDTLMISRTPEVTCVVVDVSHEDPEVKF NWYVDGVEVHNAKTKPREEQYNSTYRVVSVLTVLH QDWLNGKEYKCKVSNKALPAPIEKTISKAKGQPREP QVYTLPPSRDELTKNQVSLTCLVKGFYPSDIAVEWES NGQPENNYKTTPPVLDSDGSFFLYSKLTVDKSRWQQ GNVFSCSVMHEALHNHYTQKSLSLSPGK | SEQ ID No: 179 |
| | LC | SEQ ID No: 168 | SEQ ID No: 168 |
| D3-68-b | HC | SEQ ID No: 167 | SEQ ID No: 167 |
| | LC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSDIQLTQSPSTLSASVGDRVTISCRASRAV SPWLAWYQHRPGQAPKLLIYAASSLQSGVPSRFSGSG | SEQ ID No: 180 |
| | | SGTDFTLTISSLQPEDFATYYCQQYRSYPFTFGPGTTV DIRRTVAAPSVFIFPPSDEQLKSGTASVVCLLNNFYPR EAKVQWKVDNALQSGNSQESVTEQDSKDSTYSLSST LTLSKADYEKHKVYACEVTHQGLSSPVTKSFNRGEC | |

The above 12 fusion proteins were expressed and purified according to the method of Example 2. The expression level and purity in SEC-HPLC are shown in Table 11 below.

**Table 11. Expression level and purity of fusion proteins**

| Antibody No. | Expression level (mg/L) | Purity (%) |
|---|---|---|
| A2-22-a | 39 | 90.780 |
| A2-22-b | 104 | 96.308 |
| A3-21-a | 82 | 89.358 |
| A3-21-b | 2 | 82.095 |
| B2-15-a | 89 | 53.821 |
| B2-15-b | 70 | 99.839 |
| C2-53-a | N.A. | 54.940 |
| C2-53-b | 6 | 26.598 |
| D2-60-a | 2.4 | 40.248 |
| D2-60-b | 129 | 97.540 |
| D3-68-a | 0.3 | N.A. |
| D3-68-b | 106 | 100 |

The results showed that for different anti-IL-2 antibodies, IL-2 conjugated at the N-terminus of the heavy or light chain greatly affected the expression level and purity of the fusion protein. The low expression level or purity suggests that IL-2 and antibody may not form normal intramolecular binding but intermolecular binding in the fusion protein, thereby causing aggregation of the protein. The fusion proteins with higher expression level (at least 20 mg/L) and purity (at least 90%) were picked for subsequent identification.

### Example 9. Octet Assay on Binding of Fusion Proteins to IL-2Rα and IL-2Rβ

A Protein A biosensor (Fortebio, # 18-5010) was immersed in 200 µL of KB buffer (PBS, pH 7.4, 0.02% tween-20, 0.1% BSA) for 60 s for the wetting treatment. Then, the fusion protein was diluted to 10 µg/mL with the KB buffer, and the sensor was immersed in 200 µL of the solution until the reading was 1.2 nm. The sensor was immersed in the KB buffer for 100 s to elute excess antibody-IL-2 fusion protein. IL-2Rα (ILA-H52H9, Acrobiosystem) was diluted in a 2-fold gradient to 100-3.125 nM with the KB buffer. The sensor was immersed in the solution for 60 s for association. The sensor was immersed in the KB buffer for 60 s for dissociation. The data were fitted in a dynamic 1:1 binding mode. The avidity of fusion proteins for IL-2Rα is shown in Table 12. The results showed that IL-2 conjugated with anti-IL-2 antibodies had reduced binding to IL-2Rα as compared to IL-2, among which A3-21-a and D3-68-b did not bind to IL-2Rα at all.

**Table 12. Avidity of fusion proteins for IL-2Rα**

| Test sample | Kon (1/Ms) | Koff (1/s) | KD (M) |
|---|---|---|---|
| IL-2 | 1.40E+06 | 2.51E-02 | 1.80E-08 |
| A2-22-a | 9.22E+05 | 3.75E-02 | 4.06E-08 |
| A2-22-b | 1.63E+06 | 4.13E-02 | 2.53E-08 |
| A3-21-a | N.A. | N.A. | No binding |
| B2-15-b | 1.23E+06 | 3.30E-02 | 2.68E-08 |
| D2-60-b | 1.24E+06 | 5.61E-02 | 4.51E-08 |
| D3-68-b | N.A. | N.A. | No binding |

| | | | |
|---|---|---|---|
| (Note: N.A.: no numerical values can be obtained by fitting due to no binding. In an assay on avidity of IL-2 for IL-2Rα, IL-2Rα was used as a stationary phase, and IL-2 was used as a mobile phase). | | | |

Similar to the above methods, the data were fitted in a steady-state mode by Octet. The avidity of antibody-IL-2 fusion proteins for IL-2Rβ (CD2-H5221, Acrobiosystem) determined is shown in Table 13. The antibody fusion protein was used as a stationary phase, and the IL-2Rβ was used as a mobile phase. The results showed that most of IL-2 conjugated with anti-IL-2 antibodies did not bind to IL-2Rβ at all as compared to IL-2, and individual antibody-IL2 fusion proteins, such as D3-68-b, were weakened in binding to IL-2Rβ, but still had a certain degree of binding. In an assay on avidity of IL-2 for IL-2Rβ, IL-2Rβ was used as a stationary phase, and IL-2 was used as a mobile phase.

**Table 13. Avidity of fusion proteins of anti-IL-2 antibody and IL-2 for IL-2Rβ**

| Test sample | KD (nM) |
|---|---|
| IL-2 | 4.6E-07 |
| A2-22-a | No binding |
| A2-22-b | No binding |
| A3-21-a | No binding |
| B2-15-b | No binding |
| D2-60-b | No binding |
| D3-68-b | 1.1E-06 |

### Example 10. Determination of Activity of Fusion Proteins for STAT5 Phosphorylation in Human Peripheral Blood (PBMC)

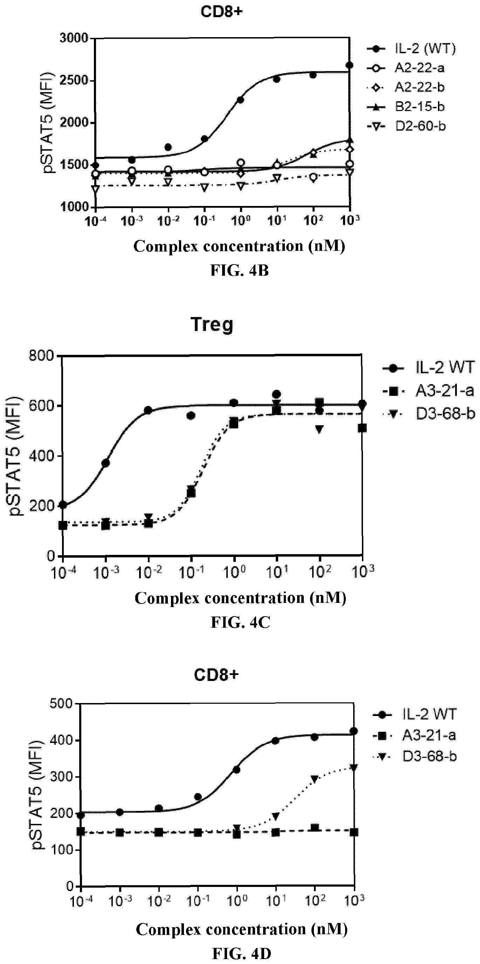

The activity of fusion proteins for STAT5 phosphorylation in Tregs and CD8⁺ T cells in human peripheral blood (PBMC) was determined according to the method in Example 7. As shown in FIGs. 4A-4D, the experimental results showed that all fusion proteins had reduced activity for Tregs and CD8⁺ T cells as compared to IL-2. However, for A2-22-a, A2-22-b, B2-15-b, D2-60-b and A3-21-b, the activity of those fusion proteins for CD8⁺ T cells was reduced more than that for Tregs, suggesting that IL-2 in those fusion proteins may be more inclined to activate Tregs.

However, for D3-68-b, its activity for Tregs was reduced more than that for CD8⁺ T cells (shown in FIGs. 4C and 4D). EC₅₀ values for fusion proteins activating pSTAT5 in Tregs and CD8⁺ T cells are shown in Tables 14 and 15.

**Table 14. EC₅₀ values for fusion proteins activating pSTAT5 in Treg cells**

| Test sample | EC₅₀ (nM) |
|---|---|
| IL-2 | 0.0003714 |
| A2-22-a | 0.04439 |
| A2-22-b | 0.05677 |
| B2-15-b | 0.01072 |
| D2-60-b | 0.1940 |
| D3-68-b | 0.1731 |
| A3-21-a | 0.1923 |

**Table 15. EC₅₀ values for fusion proteins activating pSTAT5 in CD8⁺ T cells**

| Test sample | EC₅₀ (nM) |
|---|---|
| IL-2 | 0.4343 |
| A2-22-a | N.A. |
| A2-22-b | N.A. |
| B2-15-b | N.A. |
| D2-60-b | N.A. |
| D3-68-b | 31.06 |
| A3-21-a | N.A. |

| | |
|---|---|
| (Note: N.A.: values at the highest test concentration were still low, and accurate EC₅₀ values could not be obtained by fitting). | |

### Example 11. Determination of Effect of Fusion Proteins on Immune Cells in Peripheral Blood of Balb/c Mice

Female BALB/c mice (purchased from Charles River Laboratories, Beijing) aged 6-8 weeks and weighing 18-20 g were subjected to adaptive feeding for 5 days before the experiment. All BALB/C mice were housed in an IVC constant temperature and pressure system in an SPF-grade animal room at a temperature of 20-26 °C with humidity at 40%-70%, in a 12/12 hour light/dark cycle. Not more than 6 BALB/c mice were housed in each cage. Mice were grouped according to body weight, and administration was performed after the grouping. The type of drug administered, the dose of administration and the route of administration are shown in Table 16. The day of model grouping was day 0.

**Table 16. Administration regimen for mice**

| **Groups** | **N** | **Treatment** | **Dose of administration (mg/kg)** | **Route of administration** | **Mode of administration** |
|---|---|---|---|---|---|
| 1 | 3 | A2-22-b | 0.7 | Subcutaneously | Single administration |
| 2 | 3 | B2-15-b | 0.7 | Subcutaneously | Single administration |
| 3 | 3 | D2-60-b | 0.7 | Subcutaneously | Single administration |

Freshly collected anticoagulated blood at each time point was lysed with red blood cell lysis buffer and washed with PBS once. A mixed staining solution was prepared with PBS containing 1% FBS. The mixed staining solution comprises CD3 APC-Cy7 (Biolegend 100329), CD8 PE (Biolegend 100708), CD4 PE-Cy7 (eBioscience 25-0042-82) and CD25 PerCP-Cy5.5 (BD 561112). 100 µL of mixed staining solution was added to each sample, and the mixture was incubated at 4 °C for 30 min. The mixture was washed with PBS containing 1% FBS twice. Fixation and membrane rupture were performed using True-Nuclear^{™} Transcription Factor Buffer Set (Biolegend 424401) for 60 min, and 100 µL of anti-mouse Foxp3 antibody (Biolegend 126405) and anti-mouse Ki67 antibody (eBioscience, 25-5698-82) were incubated at room temperature for 60 min. The mixture was washed with PBS (pH 7.4) twice, finally resuspended in 500 µL of PBS washing buffer at pH 7.4, and analyzed using machine. CD8⁺ T cells were defined as CD3⁺CD4⁻CD8⁺ cells, and Tregs were defined as CD3⁺CD4⁺CD25⁺Foxp3⁺ cells.

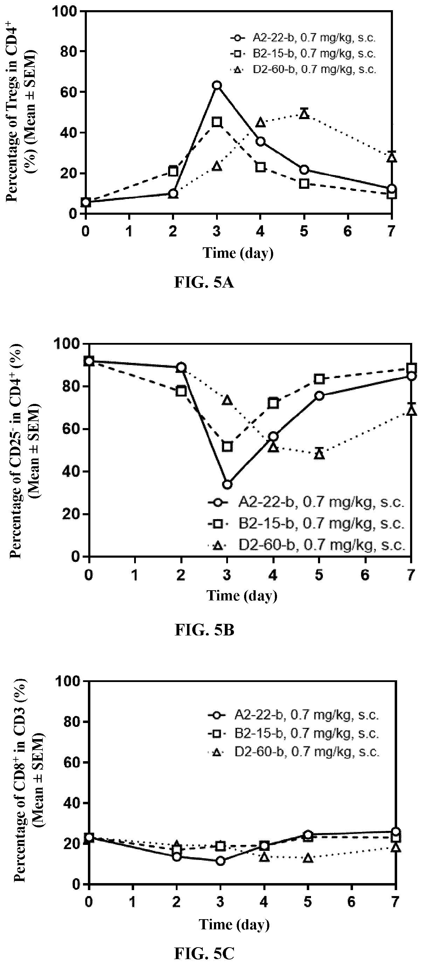

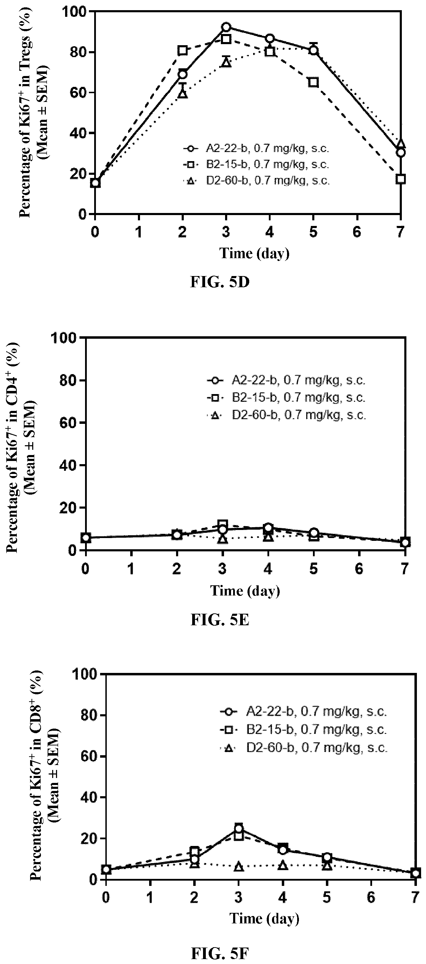

The experimental results are shown in FIGs. 5A-5F. For A2-22-b, B2-15-b, and D2-60-b, in whole blood of mice, the percentage of Tregs to CD4⁺ T cells was increased from Day 2, and the percentage of Ki67⁺ in Tregs was also increased significantly from Day 2 as compared to Day 0, indicating that A2-22-b, B2-15-b, and D2-60-b can strongly stimulate the proliferation of Tregs. In contrast, the percentage of Ki67⁺ to CD4⁺CD25⁻ T cells and CD8⁺ T cells showed little or no change, and the percentage of CD8⁺ T cells to CD3⁺ T cells also showed little change, indicating that A2-22-b, B2-15-b and D2-60-b hardly activate CD8⁺ T cells.

### Example 12. Determination of Effect of Fusion Proteins on Immune Cells in Spleen of Balb/c Mice Immunized with Chicken Ovalbumin OVA

Chicken ovalbumin (OVA, Sigma A5503) was dissolved in PBS to obtain a solution at 0.5 mg/mL. Freund's complete adjuvant (CFA, containing 1 mg/mL inactivated *Mycobacterium tuberculosis,* Sigma, F5881). Male C57B16/J mice (purchased from Shanghai Laboratory Animal Center) aged 6-8 weeks and weighing 18-20 g. Mice were grouped according to body weight. On day 1 of the experiment, 8 mL of OVA solution was taken, mixed well with 8 mL of CFA, and emulsified to form a water-in-oil solution. Each animal was immunized by intraperitoneal injection of 200 µL of the solution. The mice in all groups were each injected subcutaneously (sc) with 10 mL/kg test fusion protein or PBS control on Days 3 and 8. The type of drug administered, the dose of administration and the route of administration are shown in Table 17.

**Table 17. Immunization regimen for mice**

| Group No. | Numb er | Immunizati on | Test sample | Route of administration | Dose of administration (mpk) | Time of administration |
|---|---|---|---|---|---|---|
| 1 | 4 | OVA(50µg) | PBS | Subcutaneously | NA | Days 3 and 8 |
| 2 | 4 | OVA(50µg) | A2-22-b | Subcutaneously | 0.7 | Days 3 and 8 |
| 3 | 4 | OVA(50µg) | B2-15-b | Subcutaneously | 0.7 | Days 3 and 8 |
| 4 | 4 | OVA(50µg) | D2-60-b | Subcutaneously | 0.7 | Days 3 and 8 |

On Day 10, mice were euthanized, and spleens were taken and processed for detection of relevant immune cells by flow cytometry. The procedures were as follows: after being immersed in 75% ethanol for 5 min, the spleen of the mouse was taken out under the aseptic condition, rinsed 1-2 times with PBS, and then cut into pieces. The filter mesh was placed in a 50 mL centrifuge tube, and spleen tissue pieces were transferred to the mesh and ground while fresh PBS was added continuously. The suspension after grinding was centrifuged at 1800 rpm for 3 min, and the supernatant was discarded. 10 mL of red blood cell lysis buffer was added, the mixture was left to stand on ice for 5 min before adding PBS to terminate the lysis. The mixture was centrifuged at 1800 rpm for 3 min twice. After staining according to the BioLegend flow cytometry antibody staining process, cells were resuspended in PBS twice, sieved, and detected by flow cytometry.

The detected cells and their markers were as follows:
splenic germinal center B cells (GCBs) (Fas^{÷}GL-7^{÷}B220^{÷});
follicular T helper cells (Tfhs) (PD1-high, CXCR5-high, FOXP3⁻CD4⁺);
follicle regulatory T cells (Tfrs) (PD1-high, CXCR5-high, FOXP3⁺CD4⁺);
regulatory T cells (Tregs) (FOXP3⁺CD25⁺CD4⁺, CXCR5-low);

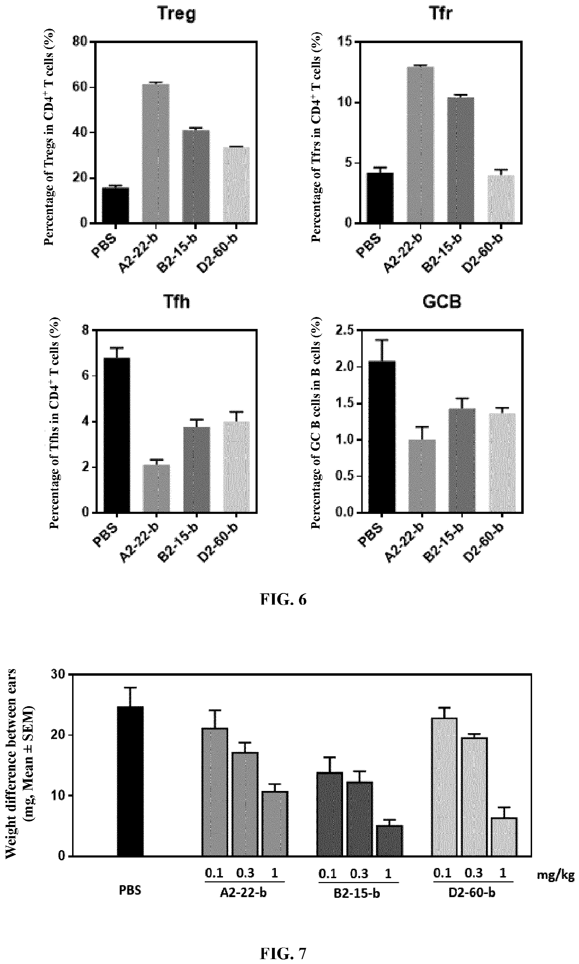

The experimental results are shown in FIG. 6. A2-22-b, B2-15-b and D2-60-b all could stimulate the proliferation of Tregs and Tfrs in the spleen and inhibit the number of Tfhs and GCBs in the spleen, indicating that A2-22-b, B2-15-b and D2-60-b have the function of inhibiting the immune system by activating Tregs.

### Example 13. Experiment on Efficacy of Fusion Proteins in Delayed Hypersensitivity Mouse Model

The experimental procedures were as follows: 2,4-dinitrofluorobenzene (DNFB, Sigma, 42085-50G) was dissolved with a mixture of acetone and olive oil (4:1) to obtain a 1% (1 g/100 mL) solution for later use, which was then diluted to a 0.5% (0.5 g/100 mL) solution for later use. Male ICR mice (purchased from Shanghai Laboratory Animal Center) aged 6 weeks and weighing 18-20 g. Mice were grouped according to body weight. On Day 0 of the experiment, the mice were immunized by applying 50 µL of 1% DNFB solution to the abdomen; on Day 5 of the experiment, the mice was challenged by applying 10 µL of 0.5% DNFB solution (20 µL in total) to the inside and outside of the right ear; and on Day 6 of the experiment, the left and right ear tissues were separately removed by using an 8 mm puncher and weighed, and the weight difference between left and right ear tissues was calculated. The test drug was subcutaneously administered once every 5 days from 2 days before the start of the experiment (Day -2).

The type of drug administered, the dose of administration and the route of administration are shown in Table 18.

**Table 18. Immunization regimen for mice**

| Group No. | Number | Test sample | Dose of administration (mpk) | Route of administration | Time of administration |
|---|---|---|---|---|---|
| 1 | 5 | PBS | NA | Subcutaneously | Days -2 and 3 |
| 2 | 5 | A2-22-b | 1 | Subcutaneously | Days -2 and 3 |
| 3 | 5 | A2-22-b | 0.3 | Subcutaneously | Days -2 and 3 |
| 4 | 5 | A2-22-b | 0.1 | Subcutaneously | Days -2 and 3 |
| 5 | 5 | B2-15-b | 1 | Subcutaneously | Days -2 and 3 |
| 6 | 5 | B2-15-b | 0.3 | Subcutaneously | Days -2 and 3 |
| 7 | 5 | B2-15-b | 0.1 | Subcutaneously | Days -2 and 3 |
| 8 | 5 | D2-60-b | 1 | Subcutaneously | Days -2 and 3 |
| 9 | 5 | D2-60-b | 0.3 | Subcutaneously | Days -2 and 3 |
| 10 | 5 | D2-60-b | 0.1 | Subcutaneously | Days -2 and 3 |

The experimental results are shown in FIG. 7. A2-22-b, B2-15-b and D2-60-b all could reduce the degree of ear swelling and show a dose-response relationship, indicating that A2-22-b, B2-15-b and D2-60-b have the function of inhibiting the immune system.

### Example 14. Experiment on Efficacy of Fusion Proteins in Arthritis Mouse Model

Male DBA/1 inbred mice were purchased from Shanghai SLAC laboratory Animal Co., Ltd. The arthritis model was induced by immunization with bovine type II collagen (Biolead, 20022). On Day 0 of the experiment, 2.5 mL of bovine type II collagen/glacial acetic acid (2 mg/mL) was taken and mixed well with 2.5 mL of Freund's complete adjuvant to form a water-in-oil emulsion. 0.1 mL of the emulsion was injected intradermally into the tail root of each mouse under light ether anesthesia, followed by one booster injection 3 weeks later. The molded animals were randomly divided into following 6 groups: a normal control group, a model control group, an IL-2 (C013, novoprotein) group, an A2-22-b group, and a B2-15-b group, with 8 animals in each group. Meanwhile, normal control animals were included in the normal control group. The groups are shown in Table 19.

**Table 19. Immunization regimen for mice**

| Group No. | Groups | Number of animals (mice) | Dose of administration (mg/kg) | Frequency of administration | Route of administration | Volume for administration (mL/kg) |
|---|---|---|---|---|---|---|
| 1 | Normal control group | 8 | - | Q5D × 14 days | i.p. | - |
| 2 | Model control group | 8 | - | Q5D × 14 days | i.p. | - |
| 3 | IL-2 group | 8 | 100000IU | Q2D × 14 days | s.c. | 5 |
| 4 | A2-22-b group | 8 | 0.7 | Q5D × 14 days | i.p. | 5 |
| 5 | B2-15-b group | 8 | 0.7 | Q5D × 14 days | i.p. | 5 |

| | | | | | | |
|---|---|---|---|---|---|---|
| (Note: Q5D, once every five days; Q2D, once every two days; i.p., intraperitoneal administration; s.c., subcutaneous administration.) | | | | | | |

Evaluation of joint inflammation: mice were weighed and scored semi-quantitatively (clinical scores) for the severity of joint inflammation every 3 days starting from secondary immunization:
0 points: no redness and swelling;
1 point: inflammation or redness and swelling at one place on the foot;
2 points: mild inflammation on the foot or redness and swelling at two or more places on the whole foot;
3 points: moderate redness and swelling on the whole foot; and
4 points: severe redness and swelling, even joint stiffness, deformity and mobility disorder on the whole foot.

The maximum score is 4 points per foot and 16 points per mouse. The observation of the severity of joint inflammation continued until the end of the experiment.

Measurement of concentration of anti-bovine type II collagen antibody in serum: at the end of the experiment, blood was collected, and serum was separated and stored at -80 °C. The level of the anti-bovine type II collagen antibody in serum was measured using an ELISA detection kit (Chondrex, 20322T). The specific steps were carried out according to the instructions.

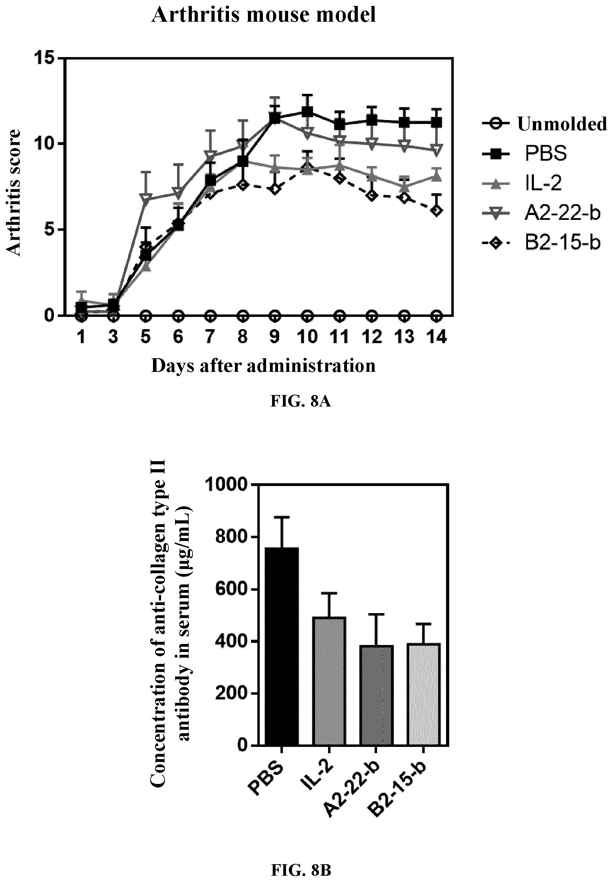

The experimental results are shown in FIGs. 8A and 8B. The weight of the mice in the model control group was significantly reduced and the arthritis score was significantly increased (p < 0.001) as compared to that in the normal control group; and B2-15-b could significantly inhibit the weight loss of arthritis model mice and significantly reduce the arthritis score (p < 0.05) as compared to the model control group. The concentration of the anti-bovine type II collagen IgG2a antibody in serum of mice in the model control group was significantly increased (p < 0.001) compared with that in the normal control group; and the administration of B2-15-b administration could significantly reduce the secretion of the anti-bovine type II collagen IgG2a antibody in serum of arthritis mice (p < 0.01) as compared to the model control group.

### Example 15: Effect of Different Lengths of Linker Arms on Expression Level and Purity of Fusion Protein

The (G₄S)₅ linker arm between IL-2 and the light chain of anti-IL-2 antibody B2-15 in B2-15-b was shortened to 4 repeats of G₄S while the heavy chain remained unchanged, so as to obtain a variant B2-15-b-(G₄S)₄ of B2-15-b. The light chain sequence of the variant is shown in Table 20.

**Table 20. Full-length light chain sequence of the antibody in the fusion protein**

| Fusion protein No. | Full-length light chain amino acid sequence | | Sequence No. |
|---|---|---|---|
| B2-15-b-(G₄S)₄ | LC | APASSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSEIVLTQSPGTLSLSPGERATLSCRASQSVSSSYLA WYQQKPGQAPRLLIYGASNRATGIPDRFSGSGSGADF TLTISRLEPEDFAVYYCQQYGSSPLTFGGGTKVEIKRT | SEQ ID No: 181 |
| | | | |

After the variant of B2-15-b described above was expressed and purified according to the method of Example 2, the expression level could still reach 47 mg/L, and the purity was 99% by SEC-HPLC, indicating that different lengths of linker arms all can achieve the expression of the fusion protein.

### Example 16. Determination of Activity of Fusion Proteins of Anti-IL-2 Antibody and Different IL-2 Variants for STAT5 Phosphorylation in Human Peripheral Blood (PBMC)

IL-2 was mutated to introduce T3E or T3Q, or to remove the first 3 amino acids at the N-terminus of IL-2 (B2-15-b-Del3), or to remove the first 7 amino acids at the N-terminus of IL-2 (B2-15-b-Del7). Alternatively, wild-type IL-2 was directly conjugated with anti-IL-2 antibodies. The following fusion proteins were produced (the heavy chain sequence of each antibody was kept constant), shown in Table 21.

**Table 21. Full-length light chain sequences of antibodies in fusion proteins**

| Fusion protein No. | Full-length light chain amino acid sequence | | Sequence No. |
|---|---|---|---|
| B2-15-b-T3E | LC | APESSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEIVLTQSPGTLSLSPGERATLSCRASQSV SSSYLAWYQQKPGQAPRLLIYGASNRATGIPDRFSGS GSGADFTLTISRLEPEDFAVYYCQQYGSSPLTFGGGTK VEIKRTVAAPSVFIFPPSDEQLKSGTASVVCLLNNFYP REAKVQWKVDNALQSGNSQESVTEQDSKDSTYSLSS TLTLSKADYEKHKVYACEVTHQGLSSPVTKSFNRGE C | SEQ ID No: 182 |
| B2-15-b-WT (wild-type IL-2) | LC | APTSSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSEIVLTQSPGTLSLSPGERATLSCRASQSV SSSYLAWYQQKPGQAPRLLIYGASNRATGIPDRFSGS GSGADFTLTISRLEPEDFAVYYCQQYGSSPLTFGGGTK VEIKRTVAAPSVFIFPPSDEQLKSGTASVVCLLNNFYP REAKVQWKVDNALQSGNSQESVTEQDSKDSTYSLSS TLTLSKADYEKHKVYACEVTHQGLSSPVTKSFNRGE C | SEQ ID No: 183 |
| B2-15-b-T3Q | LC | APQSSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGGSEIVLTQSPGTLSLSPGERATLSCRASQSV SSSYLAWYQQKPGQAPRLLIYGASNRATGIPDRFSGS GSGADFTLTISRLEPEDFAVYYCQQYGSSPLTFGGGTK VEIKRTVAAPSVFIFPPSDEQLKSGTASVVCLLNNFYP REAKVQWKVDNALQSGNSQESVTEQDSKDSTYSLSS TLTLSKADYEKHKVYACEVTHQGLSSPVTKSFNRGE | SEQ ID No: 184 |
| | | C | |
| B2-15-b-Del3 | LC | SSSTKKTQLQLEHLLLDLQMILNGINNYKNPKLTRML TFKFYMPKKATELKHLQCLEEELKPLEEVLNLAQSKN FHLRPRDLISNINVIVLELKGSETTFMCEYADETATIVE FLNRWITFAQSIISTLTGGGGSGGGGSGGGGSGGGGS GGGGSEIVLTQSPGTLSLSPGERATLSCRASQSVSSSY LAWYQQKPGQAPRLLIYGASNRATGIPDRFSGSGSGA DFTLTISRLEPEDFAVYYCQQYGSSPLTFGGGTKVEIK RTVAAPSVFIFPPSDEQLKSGTASVVCLLNNFYPREAK VQWKVDNALQSGNSQESVTEQDSKDSTYSLSSTLTL SKADYEKHKVYACEVTHQGLSSPVTKSFNRGEC | SEQ ID No: 185 |
| B2-15-b-Del7 | LC | KKTQLQLEHLLLDLQMILNGINNYKNPKLTRMLTFKF YMPKKATELKHLQCLEEELKPLEEVLNLAQSKNFHL RPRDLISNINVIVLELKGSETTFMCEYADETATIVEFLN RWITFAQSIISTLTGGGGSGGGGSGGGGSGGGGSGGG GSEIVLTQSPGTLSLSPGERATLSCRASQSVSSSYLAW YQQKPGQAPRLLIYGASNRATGIPDRFSGSGSGADFT LTISRLEPEDFAVYYCQQYGSSPLTFGGGTKVEIKRTV AAPSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC | SEQ ID No: 186 |
| A2-22-b-Del3 | LC | SSSTKKTQLQLEHLLLDLQMILNGINNYKNPKLTRML TFKFYMPKKATELKHLQCLEEELKPLEEVLNLAQSKN FHLRPRDLISNINVIVLELKGSETTFMCEYADETATIVE FLNRWITFAQSIISTLTGGGGSGGGGSGGGGSGGGGS GGGGSEIVLTQSPGTLSLSPGERATLSCRASQSVSNNY LAWYQQKPGQAPRLLIYGASSRATGIPDRFSGSGSGT DFTLTISRLEPEDFAVYSCQQYGSSPLTFGQGTRLEIKR TVAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | SEQ ID No: 187 |
| D2-60-b-Del3 | LC | SSSTKKTQLQLEHLLLDLQMILNGINNYKNPKLTRML TFKFYMPKKATELKHLQCLEEELKPLEEVLNLAQSKN FHLRPRDLISNINVIVLELKGSETTFMCEYADETATIVE FLNRWITFAQSIISTLTGGGGSGGGGSGGGGSGGGGS GGGGSDIQMTQSPSSLSASVGDRVTITCRASQGISNYL AWYQQKPGKVPKLLIYAASTLQSGVPSRFSGSGSGTD FTLTISSLQPEDVATYYCQKYDSAPFTFGQGTRLEIKR TVAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | SEQ ID No: 188 |
| D2-60-b-T3E | LC | APESSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADET ATIVEFLNRWITFAQSIISTLTGGGGSGGGGSGGGGSG GGGSGGGGSDIQMTQSPSSLSASVGDRVTITCRASQG ISNYLAWYQQKPGKVPKLLIYAASTLQSGVPSRFSGS GSGTDFTLTISSLQPEDVATYYCQKYDSAPFTFGQGTR LEIKRTVAAPSVFIFPPSDEQLKSGTASVVCLLNNFYPR EAKVQWKVDNALQSGNSQESVTEQDSKDSTYSLSST LTLSKADYEKHKVYACEVTHQGLSSPVTKSFNRGEC | SEQ ID NO: 189 |

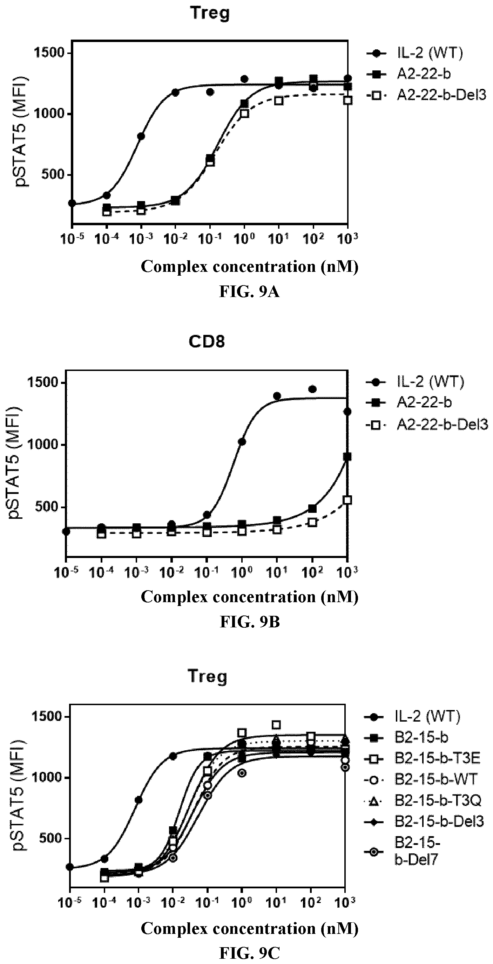

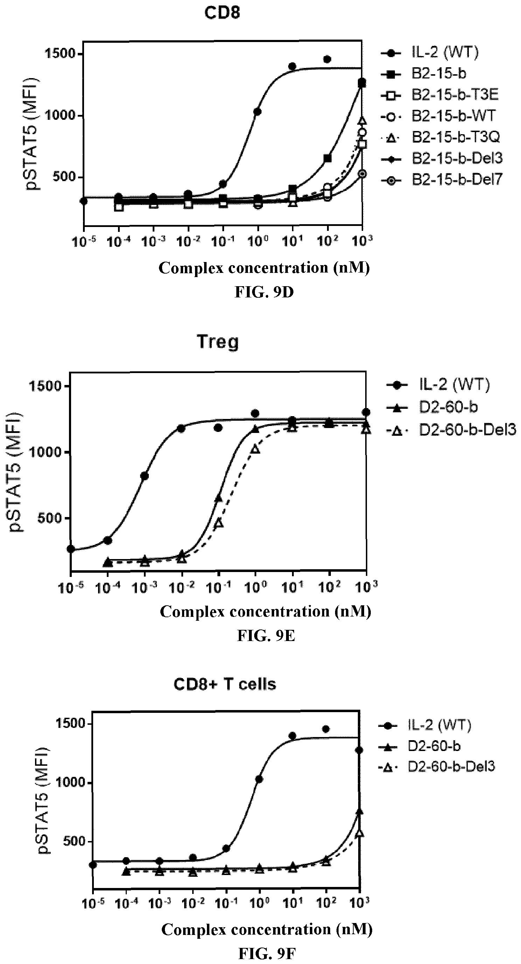

The activity of the fusion proteins of anti-IL-2 antibody and IL-2 variants described above for STAT5 phosphorylation in Tregs and CD8⁺ T cells in human peripheral blood (PBMC) was determined according to the method in Example 7. As shown in FIGs. 9A-9F and Table 22, the experimental results showed that those IL-2 variants had little effect on the activity of the antibody fusion proteins for Tregs and CD8⁺ T cells. Those fusion proteins were still more inclined to activate Tregs instead of CD8⁺ T cells.

**Table 22. EC₅₀ values for fusion proteins of anti-IL-2 antibody and different IL-2 variants activating pSTAT5 in Treg cells**

| Fusion protein No. | EC₅₀ (nM) |
|---|---|
| A2-22-b | 0.168 |
| A2-22-b-Del3 | 0.144 |
| B2-15-b | 0.016 |
| B2-15-b-T3E | 0.031 |
| B2-15-b-WT | 0.041 |
| B2-15-b-T3Q | 0.029 |
| B2-15-b-Del3 | 0.038 |
| B2-15-b-Del7 | 0.055 |
| D2-60-b | 0.114 |
| D2-60-b-Del3 | 0.229 |
| D2-60-b-T3E | 0.168 |

### Example 17. Experiment on Efficacy of Fusion Proteins in MRL/lpr Spontaneous Lupus Erythematosus Mouse Model

Female BALB/c mice and female MRL/lpr mice were purchased from Shanghai SLAC Laboratory Animal Co., Ltd. Female BALB/c mice were included in a normal control group, and female MRL/lpr mice were randomly divided into the following 3 groups according to proteinuria level and body weight: an MRL/lpr control group (model control group), a 1 mg/kg administration group and a 3 mg/kg administration group. The mice in the two administration groups, when aged at 8 weeks, were each subcutaneously injected with D2-60-b-T3E at doses of 1 mg/kg and 3 mg/kg, respectively, once every 3 days for consecutive 8 weeks.

**Table 23. Experimental design and grouping**

| Mouse | Groups | Dose of administration (mg/kg) | Frequency of administration | Route of administration | Number of animals (mice) |
|---|---|---|---|---|---|
| Normal Balb/c | Normal control group | - | Once every 3 days | sc. | 10 |
| | Model control group | - | Once every 3 days | sc. | 10 |
| MRL/lpr | D2-60-b-T3E administration group | 1 | Once every 3 days | sc. | 10 |
| | | 3 | Once every 3 days | sc. | 10 |

During the treatment period, the urine protein content of mice in each group was determined every two weeks; at the end of 8 consecutive weeks of treatment, the following tests were performed: (1) determination of the content of anti-double-stranded DNA IgM and IgG antibodies in the serum of mice in each group; (2) determination of the content of serum creatinine (CRE) and blood urea nitrogen (BUN) in the serum of mice in each group; (3) determination of the content of IL-6, IL-10 and IFN-γ in the serum of mice in each group; and (4) pathological analysis and scoring of renal tissue of mice in each group.

### 1) Determination of the urine protein content of mice

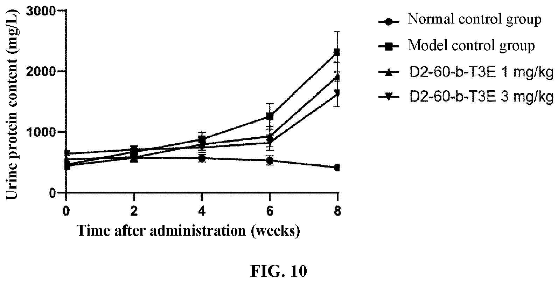

In the experiment, the urine of mice was collected by the tail-lift reflex method, and the urine protein concentration of mice was determined using a urine protein detection kit (CBB method, NanJing JianCheng Bioengineering Institute, # C035-2-1). During the pathogenesis of the lupus erythematosus in MRL/lpr mice, kidney damage occurred due to the deposition of dsDNA antibodies, which in turn led to decreased glomerular filtration function and reabsorption capacity and increased urine protein concentration. As lupus nephritis in MRL/lpr mice progressed, the urine protein level of mice would gradually increase. The determination of the urine protein level of the mice performed every two weeks showed that initially there was no significant difference in the urine protein level among the groups, but as the experiment progressed, the urine protein level of the mice in the MRL/lpr control group (model control group) was gradually increased and significantly higher than that in the normal group, indicating that the MRL/lpr mice suffer from severe spontaneous renal injury due to the deposition of autoantibodies. The average urine protein content of mice in the 1 mg/kg and 3 mg/kg D2-60-b-T3E administration groups was lower than that in the model control group and showed a dose-dependent relationship, but there was no statistical difference between the administration groups and the model control group due to the large individual differences in the data. The data are shown in FIG. 10.

### 2) Determination of the content of anti-double-stranded DNA IgM and IgG antibodies in serum

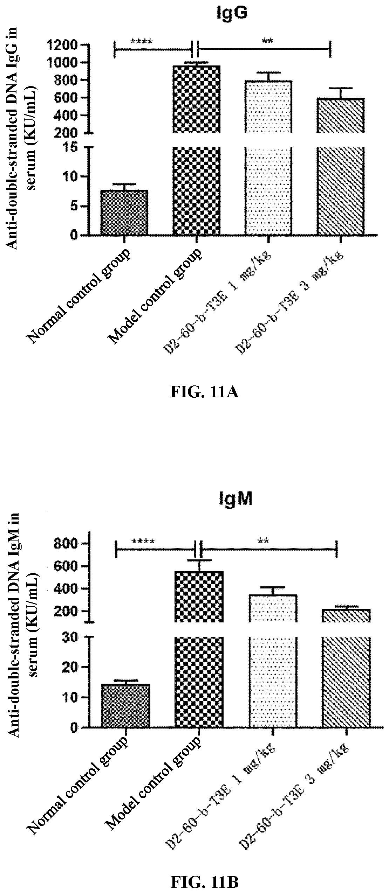

The content of anti-double-stranded DNA (anti-dsDNA) IgG and IgM antibodies in serum was determined by the ELISA method. Refer to the instructions of ELISA kit (Alpha Diagnostic, # 5120 and # 5130) for specific procedures. The average content of anti-dsDNA IgG and IgM antibodies in the serum of mice in the 3 mg/kg D2-60-b-T3E administration group was significantly lower than that in the model control group (P < 0.01), and there was no statistically significant difference between the 1 mg/kg group and the model control group although the content of IgG and IgM in the 1 mg/kg group tended to be lower than that in the model control group. The experimental results are shown in FIGs. 11A and 11B.

### 3) Effect of D2-60-b-T3E on renal function of MRL/lpr mice

The content of serum creatinine and blood urea nitrogen in the serum of mice at the end of the experiment was detected using a creatinine (CRE) determination kit (NanJing JianCheng Bioengineering Institute, # C011-2-1) and a blood urea nitrogen (BUN) determination kit (NanJing JianCheng Bioengineering Institute, # C013-2-1).

Blood urea nitrogen and creatinine are metabolic products of human proteins and muscles, respectively, and the kidney is the final excretory organ. After the renal function is damaged, blood urea nitrogen and creatinine in the serum cannot be effectively excreted, and the concentration of blood urea nitrogen (BUN) and serum creatinine (CRE) is gradually increased due to retention. Therefore, the levels of BUN and CRE in serum are the major indicators of renal function clinically. The content of serum creatinine (CRE) and blood urea nitrogen (BUN) in the serum of mice in the MRL/lpr control group (model control group) was significantly increased as compared to that of normal mice (P < 0.0001), suggesting that the mice in the MRL/lpr model group have functional renal injury.

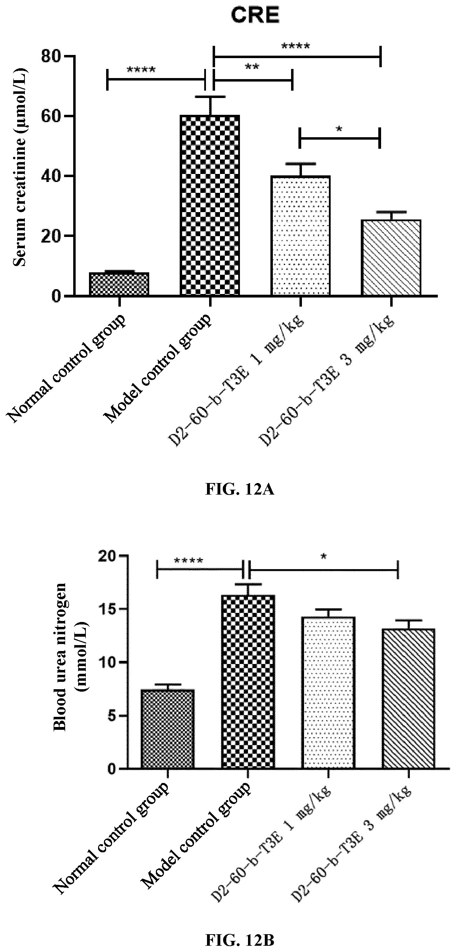

The content of creatinine in the serum of mice in the 1 mg/kg and 3 mg/kg administration groups was significantly lower than that in the model control group (P < 0.01 and P < 0.0001), indicating that the renal function of MRL/lpr mice can be improved to a certain extent. In addition, the content of creatinine in the serum of mice in the 3 mg/kg administration group was significantly lower than that in the 1 mg/kg administration group. The content of blood urea nitrogen in the serum of mice in the 3 mg/kg administration group was also significantly lower than that in the model control group (P < 0.05), indicating that the D2-60-b-T3E at 3 mg/kg can reverse the spontaneous renal injury of MRL/lpr mice. The results are shown in FIGs. 12A and 12B.

### 4) Effect of D2-60-b-T3E on the content of IL-6, IL-10 and INF-γ in serum of MRL/lpr mice

The content of the cytokines IFN-γ, IL-6 and IL-10 in the serum was determined by the ELISA method.

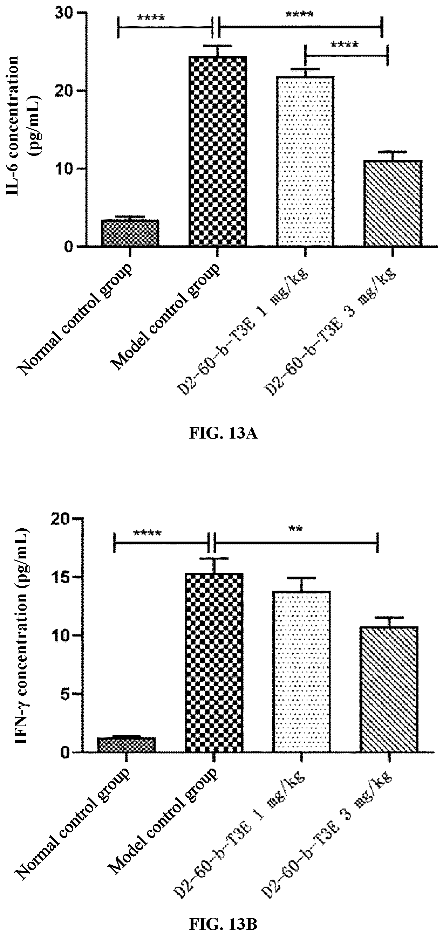

The content of IL-6 and IFN-γ in the serum of mice in the MRL/lpr control group (model group) was significantly increased as compared to that of normal mice (P < 0.0001). The content of IL-6 in the serum of mice in the 3 mg/kg administration group was significantly lower than that in the model control group and the 1 mg/kg administration group (P < 0.0001), and showed a dose-dependent relationship. The content of IFN-γ in the serum of mice in the 3 mg/kg administration group was significantly lower than that in the model control group (P < 0.01). The results are shown in FIGs. 13A and 13B.

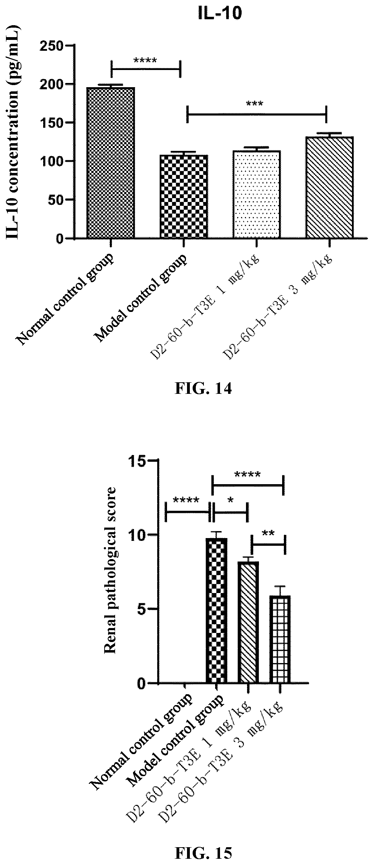

The content of IL-10 in the serum of mice in the MRL/lpr control group (model control group) was significantly decreased as compared to that of normal mice (P < 0.0001). The content of IL-10 in the serum of mice in the 3 mg/kg D2-60-b-T3E administration group was significantly higher than that in a model control group (P < 0.001). The results are shown in FIG. 14.

### 5) Effect of D2-60-b-T3E on renal lesions of MRL/lpr mice

At the end of the experiment, the left kidney of the mouse was fixed in 4% paraformaldehyde, embedded in paraffin, sectioned and then stained by H&E. The section was observed under a microscope and subjected to pathological scoring to grade and score pathological injuries of glomerulus, renal interstitium, blood vessel and the like. The scoring criteria are shown in Table 24.

**Table 24. Renal pathological scoring criteria**

| Scoring | Glomerulonephritis | Interstitial nephritis | Inflammation of blood vessels |
|---|---|---|---|
| 1+ | Focal, mild or early hyperplasia | 1-3 foci (5-10 cells) monocytes | Monocytes around the renal pelvis/blood vessels |
| 2+ | Multifocal hyperplasia with increased stromal and inflammatory cells | Mild monocyte infiltration around the single tubule; isolated atrophic tubules | Monocyte infiltration around large arteries; monocyte foci of 10-20 cells around interlobular arteries |
| 3+ | Diffuse hyperplasia | More extensive infiltration with large tubular atrophic lesions | More extensive monocyte lesions around small arterial branches |
| 4+ | Extensive sclerosis/crescent shape: proteinuria | Extensive monocyte infiltration between tubules; extensive tubular atrophy necrosis | Monocyte infiltration into the surrounding parenchyma/involvement of most of blood vessels/vasculitis |

The mice in the MRL/lpr control group (model control group) showed severe pathological injuries of glomerulus, renal interstitium and blood vessel in kidney, with glomerular sclerosis and partial inward depression, massive cell infiltration in the perivascular and interstitial regions, and severe glomerular swelling and hyperplasia. The renal pathological scores of the mice in the MRL/lpr control group (model control group) were significantly increased as compared to those of normal mice (P < 0.001), suggesting that the mice in the model control group have significant renal lesions.

The pathological injuries of glomerulus, renal interstitium and blood vessel of the mice in the administration groups were relieved to a certain extent. The renal pathological scores of the mice in the 1 mg/kg and 3 mg/kg administration groups were significantly lower than those of the model control group (P < 0.05 and P < 0.0001), indicating that D2-60-b-T3E can significantly reduce spontaneous renal lesions in MRL/lpr mice. The results are shown in FIG. 15.

## Claims

1. An anti-IL-2 antibody or an antigen-binding fragment thereof, comprising a heavy chain variable region VH and a light chain variable region VL, wherein:
the VH comprises HCDR1, HCDR2 and HCDR3 in SEQ ID NO: 9, and the VL comprises LCDR1, LCDR2 and LCDR3 in SEQ ID NO: 10;
the VH comprises HCDR1, HCDR2 and HCDR3 in SEQ ID NO: 1, and the VL comprises LCDR1, LCDR2 and LCDR3 in SEQ ID NO: 2;
the VH comprises HCDR1, HCDR2 and HCDR3 in SEQ ID NO: 5, and the VL comprises LCDR1, LCDR2 and LCDR3 in SEQ ID NO: 6;
the VH comprises HCDR1, HCDR2 and HCDR3 in SEQ ID NO: 3, and the VL comprises LCDR1, LCDR2 and LCDR3 in SEQ ID NO: 4;
the VH comprises HCDR1, HCDR2 and HCDR3 in SEQ ID NO: 7, and the VL comprises LCDR1, LCDR2 and LCDR3 in SEQ ID NO: 8; or
the VH comprises HCDR1, HCDR2 and HCDR3 in SEQ ID NO: 11, and the VL comprises LCDR1, LCDR2 and LCDR3 in SEQ ID NO: 12;
the above CDRs are defined according to the Kabat, IMGT, Chothia, AbM or Contact numbering system;
preferably, as defined according to the Kabat numbering system,
the VH comprises HCDR1-3 set forth in SEQ ID NOs: 37-39, respectively, and the VL comprises LCDR1-3 set forth in SEQ ID NOs: 40-42, respectively;
the VH comprises HCDR1-3 set forth in SEQ ID NOs: 13-15, respectively, and the VL comprises LCDR1-3 set forth in SEQ ID NOs: 16-18, respectively;
the VH comprises HCDR1-3 set forth in SEQ ID NOs: 25-27, respectively, and the VL comprises LCDR1-3 set forth in SEQ ID NOs: 28-30, respectively;
the VH comprises HCDR1-3 set forth in SEQ ID NOs: 19-21, respectively, and the VL comprises LCDR1-3 set forth in SEQ ID NOs: 22-24, respectively;
the VH comprises HCDR1-3 set forth in SEQ ID NOs: 31-33, respectively, and the VL comprises LCDR1-3 set forth in SEQ ID NOs: 34-36, respectively; or
the VH comprises HCDR1-3 set forth in SEQ ID NOs: 43-45, respectively, and the VL comprises LCDR1-3 set forth in SEQ ID NOs: 46-48, respectively;
preferably, as defined according to the Chothia numbering system,
the VH comprises HCDR1-3 set forth in SEQ ID NOs: 73-75, respectively, and the VL comprises LCDR1-3 set forth in SEQ ID NOs: 76-78, respectively;
the VH comprises HCDR1-3 set forth in SEQ ID NOs: 49-51, respectively, and the VL comprises LCDR1-3 set forth in SEQ ID NOs: 52-54, respectively;
the VH comprises HCDR1-3 set forth in SEQ ID NOs: 61-63, respectively, and the VL comprises LCDR1-3 set forth in SEQ ID NOs: 64-66, respectively;
the VH comprises HCDR1-3 set forth in SEQ ID NOs: 55-57, respectively, and the VL comprises LCDR1-3 set forth in SEQ ID NOs: 58-60, respectively;
the VH comprises HCDR1-3 set forth in SEQ ID NOs: 67-69, respectively, and the VL comprises LCDR1-3 set forth in SEQ ID NOs: 70-72, respectively; or
the VH comprises HCDR1-3 set forth in SEQ ID NOs: 79-81, respectively, and the VL comprises LCDR1-3 set forth in SEQ ID NOs: 82-84, respectively;
preferably, as defined according to the IMGT numbering system,
the VH comprises HCDR1-3 set forth in SEQ ID NOs: 109-111, respectively, and the VL comprises LCDR1-3 set forth in SEQ ID NOs: 112-114, respectively;
the VH comprises HCDR1-3 set forth in SEQ ID NOs: 85-87, respectively, and the VL comprises LCDR1-3 set forth in SEQ ID NOs: 88-90, respectively;
the VH comprises HCDR1-3 set forth in SEQ ID NOs: 97-99, respectively, and the VL comprises LCDR1-3 set forth in SEQ ID NOs: 100-102, respectively;
the VH comprises HCDR1-3 set forth in SEQ ID NOs: 91-93, respectively, and the VL comprises LCDR1-3 set forth in SEQ ID NOs: 94-96, respectively;
the VH comprises HCDR1-3 set forth in SEQ ID NOs: 103-105, respectively, and the VL comprises LCDR1-3 set forth in SEQ ID NOs: 106-108, respectively; or
the VH comprises HCDR1-3 set forth in SEQ ID NOs: 115-117, respectively, and the VL comprises LCDR1-3 set forth in SEQ ID NOs: 118-120, respectively;
preferably, as defined according to the AbM numbering system,
the VH comprises HCDR1-3 set forth in SEQ ID NOs: 145-147, respectively, and the VL comprises LCDR1-3 set forth in SEQ ID NOs: 148-150, respectively;
the VH comprises HCDR1-3 set forth in SEQ ID NOs: 121-123, respectively, and the VL comprises LCDR1-3 set forth in SEQ ID NOs: 124-126, respectively;
the VH comprises HCDR1-3 set forth in SEQ ID NOs: 133-135, respectively, and the VL comprises LCDR1-3 set forth in SEQ ID NOs: 136-138, respectively;
the VH comprises HCDR1-3 set forth in SEQ ID NOs: 127-129, respectively, and the VL comprises LCDR1-3 set forth in SEQ ID NOs: 130-132, respectively;
the VH comprises HCDR1-3 set forth in SEQ ID NOs: 139-141, respectively, and the VL comprises LCDR1-3 set forth in SEQ ID NOs: 142-144, respectively; or
the VH comprises HCDR1-3 set forth in SEQ ID NOs: 151-153, respectively, and the VL comprises LCDR1-3 set forth in SEQ ID NOs: 154-156, respectively.

2. The anti-IL-2 antibody or the antigen-binding fragment thereof according to claim 1, comprising:
a VH set forth in one of SEQ ID NOs: 9, 1, 5, 3, 7 and 11, or a VH having at least 90% or at least 95% identity thereto; and
a VL set forth in one of SEQ ID NOs: 10, 2, 6, 4, 8 and 12, or a VL having at least 95% identity thereto; wherein
preferably, the anti-IL-2 antibody or the antigen-binding fragment thereof comprises:
a VH set forth in SEQ ID NO: 9 or a VH having at least 95% identity thereto, and
a VL set forth in SEQ ID NO: 10 or a VL having at least 95% identity thereto; or
a VH set forth in SEQ ID NO: 1 or a VH having at least 95% identity thereto, and
a VL set forth in SEQ ID NO: 2 or a VL having at least 95% identity thereto; or
a VH set forth in SEQ ID NO: 5 or a VH having at least 95% identity thereto, and
a VL set forth in SEQ ID NO: 6 or a VL having at least 95% identity thereto; or
a VH set forth in SEQ ID NO: 3 or a VH having at least 95% identity thereto, and
a VL set forth in SEQ ID NO: 4 or a VL having at least 95% identity thereto; or
a VH set forth in SEQ ID NO: 7 or a VH having at least 95% identity thereto, and
a VL set forth in SEQ ID NO: 8 or a VL having at least 95% identity thereto; or
a VH set forth in SEQ ID NO: 11 or a VH having at least 95% identity thereto, and
a VL set forth in SEQ ID NO: 12 or a VL having at least 95% identity thereto.

3. The anti-IL-2 antibody or the antigen-binding fragment thereof according to claim 1 or 2, comprising:
an HC set forth in one of SEQ ID NOs: 165, 157, 161, 159, 163 and 167, or an HC having at least 80%, 90% or 95% identity thereto; and
an LC set forth in one of SEQ ID NOs: 166, 158, 162, 160, 164 and 168, or an LC having at least 80%, 90% or 95% identity thereto; wherein
preferably, the anti-IL-2 antibody comprises:
an HC set forth in SEQ ID NO: 165 or an HC having at least 90% identity thereto, and
an LC set forth in SEQ ID NO: 166 or an LC having at least 90% identity thereto; or
an HC set forth in SEQ ID NO: 157 or an HC having at least 90% identity thereto, and
an LC set forth in SEQ ID NO: 158 or an LC having at least 90% identity thereto; or
an HC set forth in SEQ ID NO: 161 or an HC having at least 90% identity thereto, and
an LC set forth in SEQ ID NO: 162 or an LC having at least 90% identity thereto; or
an HC set forth in SEQ ID NO: 159 or an HC having at least 90% identity thereto, and
an LC set forth in SEQ ID NO: 160 or an LC having at least 90% identity thereto; or
an HC set forth in SEQ ID NO: 163 or an HC having at least 90% identity thereto, and
an LC set forth in SEQ ID NO: 164 or an LC having at least 90% identity thereto; or
an HC set forth in SEQ ID NO: 167 or an HC having at least 90% identity thereto, and
an LC set forth in SEQ ID NO: 168 or an LC having at least 90% identity thereto.

4. The anti-IL-2 antibody or the antigen-binding fragment thereof according to any one of the preceding claims, wherein the anti-IL-2 antibody or the antigen-binding fragment thereof is a fully human antibody or an antigen-binding fragment thereof.

5. The anti-IL-2 antibody or the antigen-binding fragment thereof according to any one of the preceding claims, wherein the antigen-binding fragment is an scFv, Fv, Fab or Fab' fragment.

6. The anti-IL-2 antibody or the antigen-binding fragment thereof according to any one of the preceding claims, wherein the anti-IL-2 antibody or the antigen-binding fragment thereof is an IgG antibody or an antigen-binding fragment thereof, preferably an IgG1 or IgG4 antibody or an antigen-binding fragment thereof.

7. A complex, comprising:
the anti-IL-2 antibody or the antigen fragment thereof according to any one of claims 1 to 6 and IL-2, and
IL-2.

8. The complex according to claim 7, wherein the complex selectively enhances the activity of or promotes the proliferation of cells highly expressing IL-2Ra, preferably, the cells highly expressing IL-2Ra are regulatory T cells (Tregs).

9. The complex according to claim 7 or 8, wherein the anti-IL-2 antibody or the antigen fragment thereof and the IL-2 are bound by covalent or non-covalent force.

10. The complex according to any one of claims 7 to 9, wherein the complex is a fusion protein formed by the anti-IL-2 antibody or the antigen fragment thereof and the IL-2.

11. The complex according to claim 10, wherein in the fusion protein, the IL-2 is linked to a light chain (LC) or a heavy chain (HC) of the anti-IL-2 antibody or the antigen fragment thereof;
preferably, the IL-2 is linked to the N-terminus of the LC or HC of the anti-IL-2 antibody or the antigen fragment thereof; and
more preferably, the IL-2 is linked to the N-terminus of the LC of the anti-IL-2 antibody or the antigen fragment thereof.

12. The complex according to claim 10 or 11, wherein in the fusion protein, the IL-2 is linked to the anti-IL-2 antibody or the antigen fragment thereof via a linker,
preferably, the linker comprises an amino acid sequence represented by (G₄S)ₓ, wherein x is independently selected from the group consisting of integers from 1 to 20; and
more preferably, the linker is an amino acid sequence represented by (G₄S)₅.

13. The complex according to any one of claims 7 to 12, wherein the IL-2 has an amino acid substitution at position 3 or an N-terminal deletion,
preferably, the amino acid substitution at position 3 is selected from the group consisting of any one of: 3A, 3Q and 3E; and
the N-terminal deletion is deletion of first 3 or 7 amino acids at the N-terminus.

14. The complex according to any one of claims 10 to 13, wherein the fusion protein comprises an HC and an LC selected from the group consisting of any one of the following groups:
an HC set forth in SEQ ID NO: 169 or an HC having at least 90% sequence identity thereto, and
an LC set forth in SEQ ID NO: 158 or an LC having at least 90% sequence identity thereto;
an HC set forth in SEQ ID NO: 171 or an HC having at least 90% sequence identity thereto, and
an LC set forth in SEQ ID NO: 160 or an LC having at least 90% sequence identity thereto;
an HC set forth in SEQ ID NO: 159 or an HC having at least 90% sequence identity thereto, and
an LC set forth in SEQ ID NO: 172 or an LC having at least 90% sequence identity thereto;
an HC set forth in SEQ ID NO: 173 or an HC having at least 90% sequence identity thereto, and
an LC set forth in SEQ ID NO: 162 or an LC having at least 90% sequence identity thereto;
an HC set forth in SEQ ID NO: 175 or an HC having at least 90% sequence identity thereto, and
an LC set forth in SEQ ID NO: 164 or an LC having at least 90% sequence identity thereto;
an HC set forth in SEQ ID NO: 163 or an HC having at least 90% sequence identity thereto, and
an LC set forth in SEQ ID NO: 176 or an LC having at least 90% sequence identity thereto;
an HC set forth in SEQ ID NO: 177 or an HC having at least 90% sequence identity thereto, and
an LC set forth in SEQ ID NO: 166 or an LC having at least 90% sequence identity thereto;
an HC set forth in SEQ ID NO: 179 or an HC having at least 90% sequence identity thereto, and
an LC set forth in SEQ ID NO: 168 or an LC having at least 90% sequence identity thereto;
an HC set forth in SEQ ID NO: 167 or an HC having at least 90% sequence identity thereto, and
an LC set forth in SEQ ID NO: 180 or an LC having at least 90% sequence identity thereto;
an HC set forth in SEQ ID NO: 161 or an HC having at least 90% sequence identity thereto, and
an LC set forth in any one of SEQ ID NOs: 174 and 181-186 or an LC having at least 90% sequence identity thereto;
an HC set forth in SEQ ID NO: 157 or an HC having at least 90% sequence identity thereto, and
an LC set forth in any one of SEQ ID NOs: 170 and 187 or an LC having at least 90% sequence identity thereto; and
an HC set forth in SEQ ID NO: 165 or an HC having at least 90% sequence identity thereto, and
an LC set forth in any one of SEQ ID NOs: 178, 188 and 189 or an LC having at least 90% sequence identity thereto.

15. The complex according to any one of claims 7 to 14, wherein the complex selectively enhances the effect of the IL-2 on the activity of Tregs.

16. An anti-IL-2 antibody or an antigen-binding fragment thereof, wherein the anti-IL-2 antibody or the antigen-binding fragment thereof reduces the binding of IL-2 to IL-2Rα and blocks the binding of IL-2 to IL-2Rβ, having an avidity for human IL-2 with a K_{D} value of > 5 nM, preferably > 5 nM and ≤ 100 nM, and more preferably ≥ 10 nM and ≤ 60 nM.

17. The anti-IL-2 antibody or the antigen-binding fragment thereof according to claim 16, comprising a VH and a VL, wherein
the VH comprises HCDR1, HCDR2 and HCDR3 in SEQ ID NO: 9, and the VL comprises LCDR1, LCDR2 and LCDR3 in SEQ ID NO: 10;
the VH comprises HCDR1, HCDR2 and HCDR3 in SEQ ID NO: 1, and the VL comprises LCDR1, LCDR2 and LCDR3 in SEQ ID NO: 2; or
the VH comprises HCDR1, HCDR2 and HCDR3 in SEQ ID NO: 5, and the VL comprises LCDR1, LCDR2 and LCDR3 in SEQ ID NO: 6;
the above CDRs are defined according to the Kabat, IMGT, Chothia, AbM or Contact numbering system.

18. An anti-IL-2 antibody or an antigen-binding fragment thereof, wherein the anti-IL-2 antibody or the antigen-binding fragment thereof blocks the binding of IL-2 to IL-2Rα and blocks the binding of IL-2 to IL-2Rβ, having an avidity for human IL-2 with a K_{D} value of ≤ 5 nM.

19. The anti-IL-2 antibody or the antigen-binding fragment thereof according to claim 18, comprising a VH and a VL, wherein
the VH comprises HCDR1, HCDR2 and HCDR3 in SEQ ID NO: 3, and the VL comprises LCDR1, LCDR2 and LCDR3 in SEQ ID NO: 4;
the VH comprises HCDR1, HCDR2 and HCDR3 in SEQ ID NO: 7, and the VL comprises LCDR1, LCDR2 and LCDR3 in SEQ ID NO: 8; or
the VH comprises HCDR1, HCDR2 and HCDR3 in SEQ ID NO: 11, and the VL comprises LCDR1, LCDR2 and LCDR3 in SEQ ID NO: 12;
the above CDRs are defined according to the Kabat, IMGT, Chothia, AbM or Contact numbering system.

20. An anti-IL-2 antibody or an antigen-binding fragment thereof, wherein the anti-IL-2 antibody or the antigen-binding fragment thereof competes for binding to human IL-2, preferably, the same epitope of human IL-2, with the anti-IL-2 antibody or the antigen-binding fragment thereof according to claims 1-6 or 16-19.

21. A pharmaceutical composition, comprising:
the anti-IL-2 antibody or the antigen-binding fragment thereof according to any one of claims 1-6 or 16-19, or the complex according to any one of claims 7-15, and
one or more pharmaceutically acceptable excipients, diluents or carriers.

22. An isolated polynucleotide, encoding the anti-IL-2 antibody or the antigen-binding fragment thereof according to any one of claims 1-6 or 16-19, or the complex according to any one of claims 7-15.

23. A host cell, transformed with or comprising the polynucleotide according to claim 22.

24. A method for preparing an anti-IL-2 antibody or an antigen-binding fragment thereof, or a complex thereof, comprising:
expressing the anti-IL-2 antibody, or the antigen-binding fragment or complex thereof, in the host cell according to claim 23, and
isolating the anti-IL-2 antibody or the antigen-binding fragment thereof, or the complex thereof, from the host cell.

25. A method for treating or delaying progression of an autoimmune disease, comprising:
administering to a subject the anti-IL-2 antibody or the antigen-binding fragment thereof according to any one of claims 1-6 or 16-19, the complex according to any one of claims 7-15, the pharmaceutical composition according to claim 21, or the polynucleotide according to claim 22 in an effective amount for treating or delaying the disease,
preferably, the autoimmune disease is selected from the group consisting of any one or a combination of the following: lupus, graft versus host disease, hepatitis C virus-induced vasculitis, type I diabetes, multiple sclerosis, inflammatory bowel disease, chronic GvHD, asthma, dermatitis, alopecia areata, acute lung injury, sepsis, reactive arthritis, and rheumatoid arthritis.

26. A method for increasing the ratio of Tregs to non-Tregs in a population of T cells, or increasing the number of Tregs, or increasing the activity of Tregs, comprising: contacting the population of T cells with an effective amount of the anti-IL-2 antibody or the antigen-binding fragment thereof according to any one of claims 1-6 or 16-19, the complex according to any one of claims 7-15, or the pharmaceutical composition according to claim 21.

27. A method for increasing the ratio of Tregs to non-Tregs in peripheral blood of a subject, or increasing the number of Tregs, or increasing the activity of Tregs, comprising:
administering to the subject an effective amount of the anti-IL-2 antibody or the antigen-binding fragment thereof according to any one of claims 1-6 or 16-19, the complex according to any one of claims 7-15, or the pharmaceutical composition according to claim 21.
